(19) Europäisches Patentamt
European Patent Office
Office européen des brevets

(11) Numéro de publication: **0 506 532 B1**

(12) # FASCICULE DE BREVET EUROPEEN

(45) Date de publication de fascicule du brevet: **07.09.94**

(51) Int. Cl.[5]: **C07D 209/14**, C07D 209/08, C07D 209/16, C07D 401/12, C07D 401/06, A61K 31/40

(21) Numéro de dépôt: **92400764.4**

(22) Date de dépôt: **20.03.92**

(54) **Nouveaux dérivés de l'indole, procédé de préparation et médicaments les contenant.**

(30) Priorité: **26.03.91 FR 9103618**

(43) Date de publication de la demande:
**30.09.92 Bulletin 92/40**

(45) Mention de la délivrance du brevet:
**07.09.94 Bulletin 94/36**

(84) Etats contractants désignés:
**AT BE CH DE DK ES FR GB GR IT LI LU NL PT SE**

(56) Documents cités:
**EP-A- 0 416 609**
**EP-A- 0 418 071**

**Chemical Abstracts 64: 2099a**

**Chemical Abstracts 111(1): 7128s**

**Chemical Abstracts 58: 3398f**

(73) Titulaire: **LIPHA, LYONNAISE INDUSTRIELLE PHARMACEUTIOUE**
**34, rue Saint Romain**
**Boîte Postale 8481**
**F-69008 Lyon (FR)**

(72) Inventeur: **Festal, Didier**
**Pins 5- Charrière Blanche**
**F-69130 Ecully (FR)**
Inventeur: **Descours, Denis**
**65, rue Anatole France**
**F-69100 Villeurbanne (FR)**
Inventeur: **Bellemin, Robert**
**49, rue Saint Maximin**
**F-69003 Lyon (FR)**
Inventeur: **Decerprit, Jacques**
**83, Chemin de Sermenaz**
**F-01700 Neyron (FR)**

(74) Mandataire: **Le Guen, Gérard et al**
**CABINET LAVOIX**
**2, place d'Estienne d'Orves**
**F-75441 Paris Cédex 09 (FR)**

EP 0 506 532 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne de nouveaux dérivés de l'indole, les procédés de préparation de ces composés, les compositions pharmaceutiques les contenant et leur utilisation comme médicaments notamment dans le traitement des hyperlipidémies et de l'athérosclérose.

On sait que les dépôts lipidiques, notamment les dépôts de cholestérol dans les vaisseaux sont à l'origine de la formation de plaques d'athérome qui sont la cause de maladies cardiovasculaires variées; plus précisément l'athérome est une forme d'athérosclérose caractérisée par une accumulation excessive de lipides en particulier d'esters du cholestérol dans la paroi des vaisseaux; on a récemment trouvé qu'une, enzyme, l'Acyl Coenzyme A: Cholestéryl Acyl Transférase (A.C.A.T.) était responsable de l'estérification du cholestérol, et on a mis en évidence une corrélation entre l'augmentation de l'activité de cette enzyme et l'accumulation d'esters du cholestérol dans la paroi vasculaire; on sait aussi que le cholestérol alimentaire est absorbé sous forme libre puis est estérifié par l'A.C.A.T. intestinale pour être libéré dans la circulation sanguine sous forme de VLDL et/ou de chylomicrons.

On a cherché à mettre au point des produits inhibiteurs de l'A.C.A.T., capables d'empêcher l'absorption intestinale du cholestérol alimentaire et biliaire et de s'opposer aux dépôts d'esters du cholestérol dans la paroi des vaisseaux.

EP-0 418071 décrit notamment des dérivés amides et urées inhibiteurs de l'acyl-coenzyme A, ne comprenant pas de système indolique.

Cette recherche d'inhibiteurs de l'A.C.A.T. a conduit les inventeurs à élaborer une nouvelle famille d'inhibiteurs dérivés de l'indole et à trouver que ces produits, de manière inattendue, manifestent une puissante activité inhibitrice de l'A.C.A.T. vasculaire associée à un effet anti-hyperlipidémiant intense sur différentes espèces animales.

Ces propriétés des composés de l'invention les rendent particulièrement utiles notamment pour le traitement des hyperlipidémies et de l'athérosclérose.

L'invention concerne plus particulièrement les composes de formule 1,

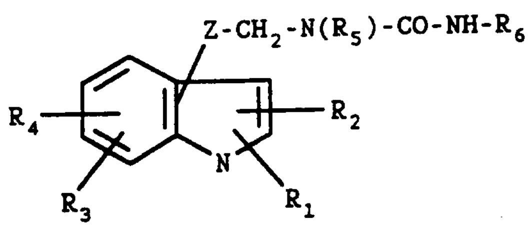

1

dans laquelle,

$R_1$ et $R_2$ qui peuvent être situés en position -1, -2 ou -3 du cycle de l'indole, représentent indépendamment un atome d'hydrogène, un radical alkyle linéaire de 1 à 12 atomes de carbone ou ramifié de 3 à 5 atomes de carbone, des radicaux alcényle, cycloalkyle, N-alkylaminoalkyl - ou N,N-dialkylaminoalkyle, ou l'un des substituants $R_1$ ou $R_2$ représente un radical pyridyl-2 (-3 ou -4)-méthyle et l'autre un atome d'hydrogène, étant entendu que lorsque l'atome d'azote du cycle indolique n'est substitué par aucun des groupes $R_1$, $R_2$ ou $-Z-CH_2-N(R_5)\ CONHR_6$, il est substitué par un atome d'hydrogène.

$R_3$ et $R_4$ qui peuvent être situés en position -4, -5, -6 ou -7 du cycle de l'indole, représentent indépendamment des atomes d'hydrogène ou d'halogène ou des radicaux alkyle, alkoxy ou alkylthio,

ou l'un des substituants $R_3$ ou $R_4$ désigne un atome d'hydrogène et l'autre des radicaux trifluorométhyle, nitro, N-alkylamino ou N,N-dialkylamino,

ou trois des substituants $R_1$, $R_2$, $R_3$ ou $R_4$ ont les significations qui viennent d'être définies et le quatrième représente un radical de formule **2**,

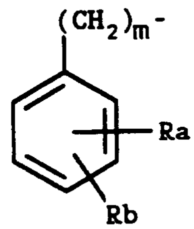

2

dans laquelle,

m peut prendre les valeurs 0,1 ou 2 et les substituants Ra et Rb désignent indépendamment des atomes d'hydrogène ou d'halogène ou des radicaux alkyle, alkoxy ou alkylthio,

$R_5$ désigne un atome d'hydrogène, un radical alkyle linéaire de 1 à 12 atomes de carbone ou ramifié de 3 à 5 atomes de carbone, un radical cycloalkyle ou un radical de formule **2**,dans laquelle m a la valeur 1 et Ra et Rb ont les significations définies précédemment,

$R_6$ désigne un radical alkyle ou un radical de formule **3**,

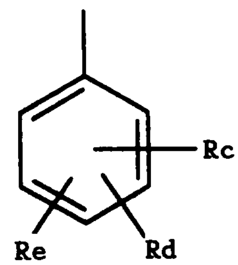

3

dans laquelle,

Rc, Rd et Re désignent indépendamment des atomes d'hydrogène ou d'halogène ou des radicaux alkyle, alkoxy ou alkylthio, ou deux des substituants Rc, Rd et Re peuvent avoir les significations qui viennent d'être définies et le troisième représente un radical trifluorométhyle,

$R_6$ peut aussi désigner les radicaux naphtyl-1 ou -2 ou un radical hétérocyclyle à 5 ou 6 chaînons et à un ou deux hétéroatomes, éventuellement fusionné avec un cycle benzénique et le cas échéant substitués par un à trois atomes d'halogène ou radicaux alkyle ou alkoxy,

Z, qui peut être relié aux sommets -1, -2, -3, -4, -5, -6 ou -7 du cycle de l'indole, désigne les biradicaux de formules: $-(CH_2)_n-C(R_7R_8)-(CH_2)_p-$, ou $-CH=CH-C(R_7R_8)-$, dans lesquelles, n et p désignent deux nombres entiers pouvant prendre les valeurs 0, 1 et 2 à condition que leur somme (n + p) soit inférieure ou égale à 2,

$R_7$ et $R_8$ représentent indépendamment un atome d'hydrogène, un radical alkyle linéaire de 1 à 12 atomes de carbone ou ramifié de 3 à 5 atomes de carbone, des radicaux alcényle, cycloalkyle, N-alkylamino, N,N-dialkylamino, N-alkylaminoalkyle ou N,N-dialkylaminoalkyle ou un groupe de formule **2**, dans laquelle m peut prendre les valeurs 0 ou 1 et Ra et Rb ont les significations déjà définies,

$R_7$ et $R_8$ peuvent aussi former ensemble une chaîne polyméthylène: $-(CH_2)_q-$ dans laquelle q peut prendre les valeurs 3 à 8 et susceptible le cas échéant, lorsque q est supérieur ou égal à 5, de contenir une double liaison,

$R_7$ et $R_8$ peuvent aussi former entre eux les chaînes: $-CH_2-O-(CH_2)_2-$, $-(CH_2)_2-O-(CH_2)_2-$, $-CH_2-S-(CH_2)_2-$, $-(CH_2)_2-S-(CH_2)_2-$, $-CH_2-N(R_9)-(CH_2)_2-$ ou $-(CH_2)_2-N(R_9)-(CH_2)_2-$,

$R_5$, $R_7$ et $R_8$ peuvent aussi, lorsqu'ils représentent des radicaux cycloalkyle, contenir une double liaison,

$R_9$ représente un radical alkyle.

Suivant une forme préférée de l'invention, celle-ci a pour objet les composés de formule **1**, dans laquelle, $R_1$ et $R_2$ représentent des atomes d'hydrogène, des radicaux alkyle, linéaires en $C_1$-$C_{12}$ ou ramifiés en $C_3$-$C_5$ , N-alkylaminoalkyle, N,N-dialkylaminoalkyle, $R_3$ et $R_4$ représentent des atomes d'hydrogène ou d'halogène ou des radicaux alkyle ou alkoxy, $R_5$ représente un atome d'hydrogène, un radical

alkyle linéaire en $C_1$-$C_{12}$ ou un groupe de formule **2** dans laquelle m = 1 et $R_6$ désigne un radical de formule **3**.

Par le terme "alkyle" on entend un enchaînement hydrocarboné, sauf précisions contraires, saturé, linéaire ou ramifié, dérivé de l'alcane correspondant par suppression d'un atome d'hydrogène et comprenant plus particulièrement 1 à 5 atomes de carbone comme par exemple: méthyle, éthyle, n.propyle, n.butyle, isopropyle, tertiobutyle ou diméthyl-2,2-propyle.

Le terme "alcényle" caractérise un radical hydrocarboné, linéaire ou ramifié, comprenant plus particulièrement de 3 à 6 atomes de carbone et contenant une double liaison, comme par exemple: allyle, butén-3-yle, méthyl-2prop-2-ényle.

Par le terme "halogène" on entend plus précisément le brome, le chlore ou le fluor.

Par "alkoxy" ou "alkylthio" on entend un enchaînement alkyle en $C_1$-$C_5$ tel que défini ci-desus lié à la molécule parent par un atome d'oxygène ou de soufre comme par exemple: méthoxy, éthoxy, n.propoxy, isopropoxy, n.butoxy, tertbutyloxy, méthylthio, éthylthio, n.propylthio ou isopropylthio.

Les termes "N-alkylamino" respectivement "N,N-dialkylamino" désignent un atome d'azote substitué par un atome d'hydrogène et un radical alkyle en $C_1$-$C_5$ respectivement par deux radicaux alkyle en $C_1$-$C_5$ et dont la valence libre réalise la liaison avec la molécule parent.

Par le terme "N-alkylaminoalkyle", respectivement N,N-dialkylaminoalkyle", on entend un radical alkyle en $C_1$-$C_5$ tel que défini ci-dessus et substitué en position oméga par un radical N-alkylamino respectivement N,N-dialkylamino.

Le terme "cycloalkyle" caractérise un enchaînement hydrocarboné saturé en $C_3$-$C_8$ cyclique, dérivé d'un cyclane tel que le cyclopropane, le cyclopentane, le cyclohexane, le cycloheptane ou le cyclooctane, par suppression d'un atome d'hydrogène.

Par "radical hétérocyclyle à 5 ou 6 chaînons et à 1 ou 2 hétéroatomes", on entend un radical dérivé, par suppression d'un atome d'hydrogène, d'un cycle à 5 ou 6 chaînons et contenant un ou deux hétéroéléments choisis parmi l'oxygène, le soufre ou l'azote comme par exemple les cycles thiophène, furane, pyrrole, pyridine, thiazole, isothiazole, oxazole, isoxazole, imidazole, pyrimidine ou pyrazine, notamment les radicaux thiényl- ou furyl-2 ou -3, pyrrolyl-1, oxazolyl-, thiazolyl- ou imidazolyl-2, -4 ou -5, isoxazolyl- ou isothiazolyl-3, -4 ou -5, pyridyl-2, -3 ou -4, pyrimidinyl-2 ou pyrazinyl-2 ou -3; on entend par "éventuellement fusionné avec un cycle benzénique" les radicaux dérivés, par suppression d'un atome d'hydrogène, des bicycles résultant de la fusion des hétérocycles sus-nommés avec le benzène tels que les cycles benzothiophène, benzofurane, indole, benzimidazole, quinoléine, isoquinoléine, quinoxaline ou quinazoline, en particulier les radicaux benzo(b)thiényl- ou benzo(b)furyl-5 ou -6, indolyl-2, -3, -5 ou -6, benzimidazolyl-2, -5 ou -6, quinolyl-2, -3, -4, -5, -6 ou -7, isoquinolyl-1, -3, -4, -6 ou -7, quinoxalyl-2, -3, -6 ou -7 ou quinazolyl-2, -4, -6 ou -7.

Les composés de formule **1** peuvent renfermer un ou plusieurs centres d'asymétrie, notamment lorsque les substituants $R_7$ et $R_8$ sont différents, ce qui génère des diastéréoisomères, énantiomères et racémiques qui font également partie de l'invention.

Il entre dans la compétence normale du spécialiste d'isoler ou de synthétiser une forme optiquement active d'un composé de formule **1**, par exemple par dédoublement d'un racémique ou par synthèse au départ d'un composé optiquement actif et de déterminer les propriétés biologiques des isomères ainsi isolés suivant les expérimentations décrites ci-après.

Les mélanges de stéréoisomères peuvent être séparés par les méthodes usuelles à celui des stades qui est le plus approprié; par méthodes usuelles, on entend l'ensemble des procédés familiers au spécialiste comme par exemple la cristallisation, la chromatographie ou la formation de combinaisons avec des composés optiquement actifs.

Les composés de formule **1** peuvent exister sous une ou plusieurs formes tautomères qui font aussi partie de l'invention.

Comme composés spécifiques de l'invention on peut citer, à titre d'exemple uniquement, les composés suivants dont les formules développées figurent à la fin de la description.

- **Composé n°1:** N1-(Diisopropyl-2,6-phényl)-N2-[(méthyl-1-indolyl-3)-1-cyclopentylméthyl]urée.
- **Composé n°2:** N1-[(Indolyl-1)-1-cyclopentylméthyl]-N2-(diisopropyl-2,6-phényl)urée.
- **Composé n°3:**N1-(Diisopropyl-2,6-phényl)-N2-[[(méthyl-1-indolyl-3)-1-cyclopentyl]-3-propyl]urée.
- **Composé n°4:**N1-(Diisopropyl-2,6-phényl)-N2-[(méthyl-1-indolyl-3)-1-cyclopentyl]-2-éthyl]urée.
- **Composé n°5:**N1-Benzyl-N2-(diisopropyl-2,6-phényl)-N1-[(méthyl-1-indolyl-3)-1-cyclopentylméthyl]-urée
- **Composé n°6:**N1-[(Méthyl-1-indolyl-3)-1-cyclopentylméthyl]-N2-phényl urée.
- **Composé n°7:**N1-[(Indolyl-3)-2-éthyl]-N2-(diisopropyl-2,6-phényl)urée.
- **Composé n°8:** N1-(Difluoro-2,4-phényl)-N2-[(méthyl-1-indolyl-3)-1-cyclopentylméthyl]urée.

. **Composé n°9:** N1-(Diisopropyl-2,6-phényl)-N2-[méthyl-3-(méthyl-1-indolyl-3)-2-butyl]urée.
. **Composén°10 :** N1-*tert*.Butyl-N2-[(méthyl-1-indolyl-3)-1-cyclopentylméthyl]urée.
. **Composé n°11:**N1-(Diisopropyl-2,6-phényl)-N2-((méthyl-1-indolyl-3)-2-propyl]urée.
. **Composé n°12 :** N1-[(Benzyl-1-indolyl-3)-1-cyclopentylméthyl]-N2-(diisopropyl-2,6-phényl)urée.
. **Composé n°13:**N1-[(Ethyl-1-indolyl-3)-1-cyclopentylméthyl]-N2-(diisopropyl-2,6-phényl)urée.
. **Composé n°14:**N1-(Diisopropyl-2,6-phényl)-N2-[(méthyl-1-indolyl-3)-2-butyl]urée.
. **Composé n°15:**N1-(Diisopropyl-2,6-phényl)-N2-[(diméthyl-1,-5-indolyl-3)-1-cyclopentylméthyl]urée.
. **Composé n°16:**N1-(Diisopropyl-2,6-phényl)-N2-[(méthyl-1-indolyl-3)-1-cyclohexylméthyl]urée.
. **Composé n°17 :** N1-[Ethyl-2-(méthyl-1-indolyl-3)-2-butyl]-N2-(diisopropyl-2,6-phényl)urée.
. **Composé n°18:**N1-(Diisopropyl-2,6-phényl)-N2-[méthyl-2-(méthyl-1-indolyl-3)-2-propyl]urée.
. **Composé n°19:** N1-[(Isopropyl-1-indolyl-3)-1-cyclopentylméthyl]-N2-(diisopropyl-2,6-phényl)urée.
. **Composé n°20:**N1-[(Allyl-1-indolyl-3)-1-cyclopentylméthyl]-N2-(diisopropyl-2,6-phényl)urée.
. **Composé n°21:**N2-(Diisopropyl-2,6-phényl)-N2-[(méthyl-1-indolyl-3)-2-phényl-2-éthyl]urée.
. **Composén°22 :** N1-[Allyl-2-(méthyl-1-indolyl-3)-2-pentén-4-yl]-N2-(diisopropyl-2,6-phényl)urée.
. **Composé n°23:**N1-(Diisopropyl-2,6-phényl)-N2-[(phényl-1-indolyl-3)-1-cyclopentylméthyl]urée.
. **Composé n°24:**N1-(Diisopropyl-2,6-phényl)-N2-[(méthyl-1-indolyl-3)-1-cyclobutylméthyl]urée.
. **Composé n°25:**N1-[Butyl-2-(méthyl-1-indolyl-3)-2-hexyl]-N2-(diisopropyl-2,6-phényl)urée.
. **Composé n°26:**N1-(Diisopropyl-2,6-phényl)-N2-[(méthyl-1-indolyl-3)-2-pentén-4-yl]urée.
. **Composé n°27 :** N1-[(Heptyl-1-indolyl-3)-1-cyclopentylméthyl]-N2-(diisopropyl-2,6-phényl)urée.
. **Composé n°28:**N1-[(Butyl-1-indolyl-3)-1-cyclopentylméthyl)]-N2-(diisopropyl-2,6-phényl)urée.
. **Composé n°29:**N1-(Diisopropyl-2,6-phényl)-N2-[(méthyl-1-indolyl-3)-2-hexyl]urée.
. **Composé n°30:**N1-(Diisopropyl-2,6-phényl)-N2-[(méthyl-1-indolyl-3)-2-nonyl]urée.
. **Composé n°31 :** N1-(Diisopropyl-2,6-phényl)-N2-[(diméthyl-4,4-(méthyl-1-indolyl-3)-2-pentyl]urée.
. **Composé n°32:**N1-(Diisopropyl-2,6-phényl)-N2-[(méthyl-1-indolyl-3)-2-phényl-3-propyl]urée.
. **Composé n°33:**N1-(Dichloro-2,6-phényl)-N2-[(méthyl-1-indolyl-3)-1-cyclopentylméthyl]urée.
. **Composé n°34:**N1-(Diisopropyl-2,6-phényl)-N2-[(méthyl-1-indolyl-3)-1-cycloheptylméthyl]urée.
. **Composé n°35:**N1-(Diisopropyl-2,6-phényl)-N2-[(méthyl-1-indolyl-3)-4-tétrahydropyranyl-4-méthyl]-urée.
. **Composé n°36:**N1-(Diéthyl-2,6-phényl)-N2-[(méthyl-1-indolyl-3)-1-cyclopentylméthyl]urée.
. **Composé n°37:** N1-(Diisopropyl-2,6-phényl)-N2-[[(diméthylamino-2-éthyl)-1-indolyl-3]-1-cyclopentylméthyl]urée.
. **Composé n°38:** N1-[[(Fluoro-4-benzyl)-1-indolyl-3]-1-cyclopentylméthyl]-N2-(diisopropyl-2,6-phényl)-urée.
. **Composé n°39:** N1-(Triméthoxy-2,4,6-phényl)-N2-[(méthyl-1-indolyl-3)-1-cyclopentylméthyl]urée.
. **Composé n°40:**N1-(Diméthoxy-4,6-pyrimidinyl-5)-N2-[(méthyl-1-indolyl-3)-1-cyclopentylméthyl]urée.
. **Composé n°41:** N1-(Diisopropyl-2,6-phényl)-N2-[(méthoxy-5-méthyl-1-indolyl-3)-1-cyclopentylméthyl]urée.
. **Composé n°42:** N1-(Diisopropyl-2,6-phényl)-N2-[diméthylamino-4-(méthyl-1-indolyl-3)-2-butyl]urée.
. **Composén°43:** N1-(Diisopropyl-2,6-phényl)-N2-[méthyl-5-(méthyl-1-indolyl-3)-2-hexén-4-yl]urée.
. **Composén°44:**N1-(Diisopropropoxy-2,6-phényl)-N2-[(méthyl-1-indolyl-3)-1-cyclopentylméthyl]urée.
. **Composén°45 :** N1-(Diisopropyl-2,6-phényl)-N2-[[(pyridyl-3-méthyl)-1-indolyl-3]-1-cyclopentylméthyl]-urée.
. **Composé n°46 :** N1-Benzyl-N1-[(méthyl-1-indolyl-3)-1-cyclopentylméthyl)-N2-(diméthyl-2,6-phényl)-urée.
. **Composé n°47 :** N1-Benzyl-N2-(dichloro-2,6-phényl)-N1-[(méthyl-1-indolyl-3)-1-cyclopentylméthyl]-urée.
. **Composé n°48 :** N1-Benzyl-N2-(difluoro-2,4-phényl)-N1-[(méthyl-1-indolyl-3)-1-cyclopentylméthyl]-urée.
. **Composé n°49 :** N2-(Diisopropyl-2,6-phényl)-N1-méthyl-N1-[(méthyl-1-indolyl-3)-1-cyclopentylméthyl]urée.
. **Composé n°50 :** N1-(Diisopropyl-2,6-phényl)-N2-[(diméthyl-1,2-indolyl-3)-1-cyclopentylméthyl]urée.
. **Composé n°51 :** N1-(Diisopropyl-2,6-phényl)-N2-[(méthyl-1-phényl-2-indolyl-3)-1-cyclopentylméthyl]-urée.
. **Composé n°52 :** N1-(Diisopropyl-2,6-phényl)-N2-[(diméthyl-1,3-indolyl-2)-1-cyclopentylméthyl]urée.
. **Composé n°53:** (-)-N1-(Disopropyl-2,6-phényl)-N2-[(méthyl-1-indolyl-3)-2-hexyl]urée.
. **Composé n°54:** (+)-N1-(Diisopropyl-2,6-phényl)-N2-[(méthyl-1-indolyl-3)-2-hexyl]urée.

L'invention concerne aussi les procédés de préparation des composés de formule **1**, caractérisés en ce qu'ils comportent au moins comme l'illustre le schéma 1 ci-après,

a) la réduction d'un nitrile de formule générale **6**, dans laquelle Z, $R_1$, $R_2$, $R_3$ et $R_4$ ont les significations générales ou particulières déjà définies, Z peut également représenter un radical $-C(R_7R_8)-CH=CH-$,

b) la réaction d'une amine de formule générale **5** dans laquelle Z, $R_1$, $R_2$, $R_3$ et $R_4$ ont les significations générales ou particulières déjà définies avec un composé halogéné de formule $R_5$-X dans laquelle X désigne un atome de brome, de chlore ou d'iode et $R_5$ a les significations générales ou particulières déjà indiquées,ou bien avec un aldéhyde de formule $R_{10}$-CHO ou un chlorure ou un anhydride d'acide respectivement de formules $R_{10}$-COCl ou $(R_{10}-CO)_2O$ dans lesquelles $R_{10}$ représente un radical alkyle contenant éventuellement une double liaison ou un radical de formule **2** précédemment définie dans laquelle m est égal à 0 et Ra et Rb ont les significations générales ou particulières déjà définies, suivie de la réduction de l'imine ou de l'amide correspondant ainsi obtenu,

c) la réaction du phosgène et d'une amine appropriée de formule $R_6NH_2$ ou d'un isocyanate de formule $R_6NCO$ ou bien encore d'un trichloracétamide de formule générale $Cl_3C-CO-NHR_6$, dans lesquelles $R_6$ a les significations générales ou particulières définies plus haut, avec une amine de formule générale **4** dans laquelle Z, $R_1$, $R_2$, $R_3$, $R_4$ et $R_5$ ont les significations générales et particulières déjà définies.

Schéma 1

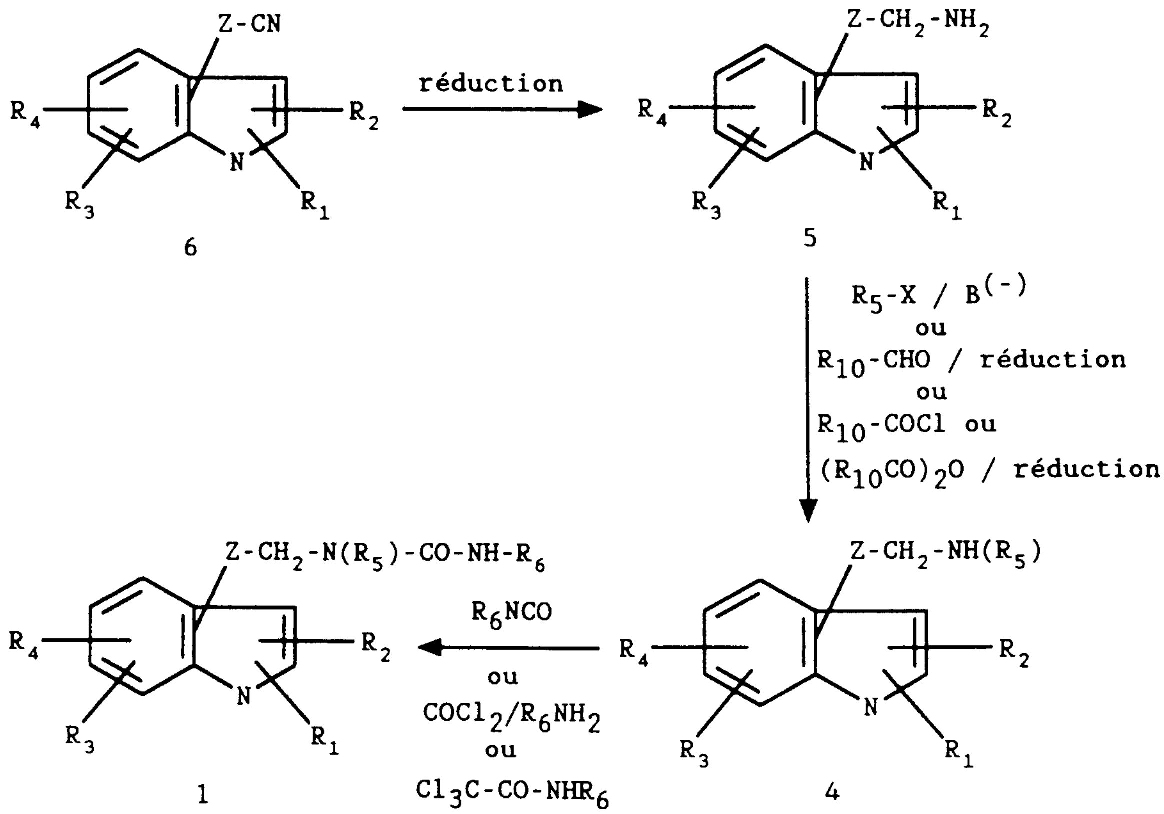


La réduction selon a) consiste en une hydrogénation catalytique ou bien en une réduction chimique notamment dans le cas où le composé intermédiaire **6** à réduire,renferme un ou plusieurs groupes sensibles à l'hydrogénation et qu'on ne désire pas réduire; l'hydrogénation catalytique est effectuée à la pression atmosphérique ou sous pression pouvant aller jusqu'à 180 bars, en présence d'un catalyseur métallique comme par exemple le palladium dispersé sur charbon ou le nickel de Raney et d'une base qui est généralement la soude en pastilles, l'ammoniac ou encore une amine tertiaire aliphatique comme la triéthylamine, dans un solvant compatible avec l'hydrogène, de préférence polaire, comme par exemple le tétrahydrofurane ou un alcool comme le méthanol, l'éthanol ou l'isopropanol; la réduction chimique est réalisée en utilisant de préférence comme réducteur, l'hydrure de lithium et d'aluminium, bien que d'autres réducteurs de la fonction nitrile puissent également convenir, au sein d'un solvant usuellement utilisé avec ce réducteur comme le tétrahydrofurane ou le diéthyléther, à une température comprise entre la température ambiante et la température de reflux du solvant utilisé mais de préférence à la température de reflux du solvant utilisé.

L'alkylation selon b) est réalisée en présence d'un agent basique, de préférence une amine tertiaire comme par exemple la triéthylamine, dans un solvant adapté, de préférence un solvant polaire comme par exemple le tétrahydrofurane et généralement à la température de reflux du solvant utilisé; la condensation selon b) d'un aldéhyde sur une amine **5**, comme alternative à l'alkylation, est effectuée dans un solvant ou un mélange de solvants, inerte vis à vis des réactifs et non miscible à l'eau; on peut utiliser comme solvants notamment des hydrocarbures aromatiques en particulier des alkylbenzènes comme le toluène ou le xylène; on peut le cas échéant, afin de faciliter la formation de l'imine, ajouter un agent de déshydratation comme par exemple l'acide paratoluènesulfonique; on opère de préférence à la température de reflux du solvant utilisé; l'imine ainsi obtenue est directement réduite en l'amine **4** par le borohydrure de sodium; cette réduction est effectuée dans un solvant adapté comme le tétrahydrofurane ou un alcool qui peut être par exemple le méthanol ou bien l'éthanol et à une température comprise entre la température ambiante et la température de reflux du solvant utilisé; l'acylation selon b), comme autre procédé de synthèse des composés **4**, est réalisée en présence d'un agent basique, de préférence une amine tertiaire aliphatique comme par exemple la triéthylamine, au sein d'un solvant qui est de préférence le tétrahydrofurane ou bien un hydrocarbure aromatique comme par exemple le benzène ou un alkylbenzène comme le toluène ou le xylène ou encore un solvant halogéné comme le chloroforme ou le chlorure de méthylène et de préférence à la température d'ébullition du solvant utilisé; on réduit ensuite l'amide ainsi obtenu par l'hydrure de lithium et d'aluminium, dans l'éther diéthylique ou le tétrahydrofurane, à la température d'ébullition du solvant utilisé.

La condensation d'un isocyanate selon c) est effectuée de préférence au sein d'un alcane comme le pentane ou l'hexane, ou encore d'un éther comme le diisopropyléther ou l'éther diéthylique ou bien, dans le cas d'une amine moins soluble, dans l'acétate d'éthyle et à la température la mieux adaptée pour obtenir la réaction qui est généralement la température ambiante; la réaction avec le phosgène selon c), comme méthode alternative de préparation des composés de l'invention, est réalisée au sein d'un hydrocarbure aromatique comme par exemple le toluène en présence d'un agent basique comme la triéthylamine et à une température proche de la température ambiante, lorsque la formation du chlorure de carbamoyle intermédiaire est complète on le met à réagir avec l'amine souhaitée à une température comprise entre la température ambiante et la température d'ébullition du solvant utilisé; l'aminocarbonylation selon c) dans laquelle on utilise un trichloracétamide de formule générale $Cl_3C\text{-}CO\text{-}NH\text{-}R_6$, est effectuée par chauffage au sein d'un solvant polaire aprotique comme par exemple le N,N-diméthylformamide, la tétraméthylurée, la N-méthylpyrrolidone ou encore l'hexaméthylphosphorotriamide, en présence d'un agent basique qui est de préférence un carbonate alcalin ou alcalino-terreux comme le carbonate de sodium ou de potassium et à une température comprise entre la température ambiante et 110 ° C et de préférence à une température voisine de 110 ° C.

L'invention vise également ceux des composés intermédiaires répondant aux formules générales **4**, **5** et **6** qui sont nouveaux ainsi que leurs procédés de synthèse.

Les nitriles intermédiaires de formule générale **6**, dans laquelle Z représente un biradical de formule générale:$-(CH_2)_n-C(R_7R_8)-(CH_2)_p-$ dans laquelle $n=p=0$ et $R_7$ et $R_8$ ont les significations générales ou particulières déja précisées, peuvent être préparés selon le schéma 2 ci-après, selon lequel on alkyle un N,N-diméthylaminométhylindole approprié de formule générale **7**, dans laquelle $R_1$, $R_2$, $R_3$, et $R_4$ ont les significations générales ou particulières déja indiquées, avec l'iodure de méthyle ou le sulfate de diméthyle, dans un solvant de préférence polaire tel que l'acétonitrile, l'acétone ou encore l'acétate d'éthyle, et de préférence à la température de reflux du solvant utilisé, puis on traite l'iodure d'indolylméthyltriméthylammonium ainsi obtenu par le cyanure de sodium ou de potassium, dans un solvant ou un mélange de solvants polaires comme par exemple l'éthanol ou le N,N-diméthylformamide ou les mélanges éthanol-eau ou N,N-diméthylformamide-eau, ou bien on réalise une N-alkylation d'un composé indolique de formule générale **8**, dans laquelle $R_1$, $R_2$, $R_3$ et $R_4$ ont les significations générales ou particulières déja définies, par le chloroacétonitrile en présence d'un agent basique comme l'hydrure de sodium ou un alcoolate alcalin comme par exemple le tertiobutylate de potassium dans un solvant polaire comme le N,N-diméthylformamide ou le diméthylsulfoxyde ou encore en présence de soude et d'un catalyseur de transfert de phase; le nitrile ainsi obtenu peut le cas

Schéma 2

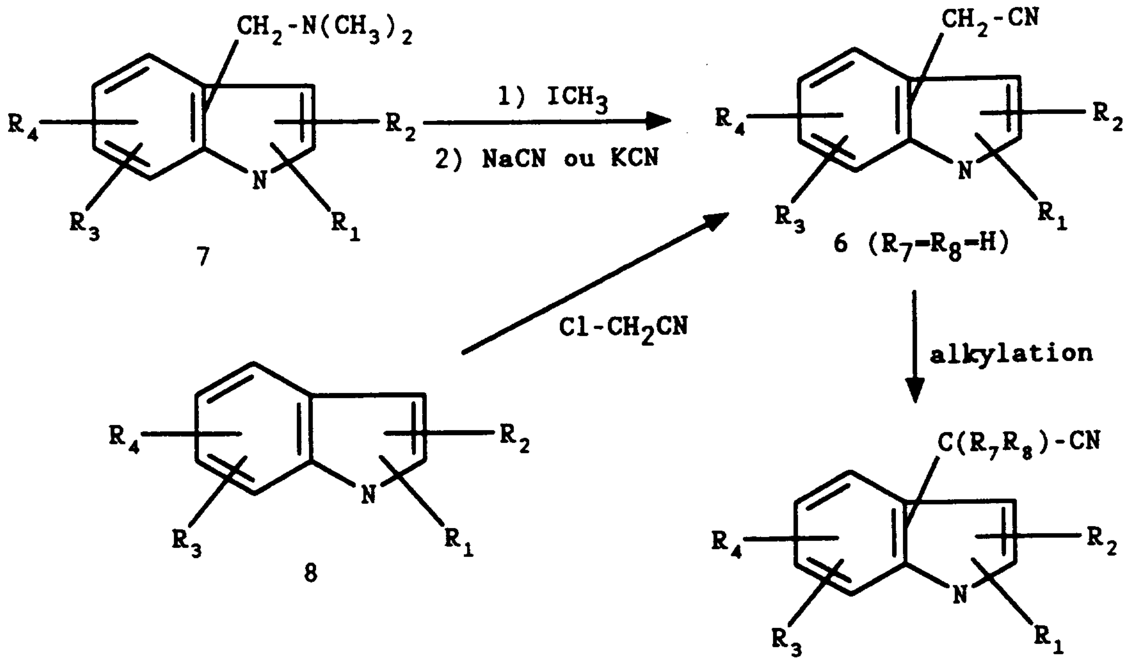


7

6 ($R_7=R_8=H$)

8

6

échéant être mono ou dialkylé par des halogénures de formule $R_7$-X ou $R_8$-X ou des dihalogénures de formule X-$(CH_2)_q$-X dans lesquelles, q, $R_7$ et $R_8$ ont les significations générales ou particulières spécifiées précédemment et le cas échéant $R_7$ et $R_8$ renferment une double liaison, en présence d'un agent basique comme l'hydrure de sodium ou encore un amidure alcalin ou alcalino-terreux comme l'amidure de sodium ou le diisopropylamidure de lithium, dans un solvant ou un mélange de solvants adaptés comme le benzène ou le toluène ou encore le diéthyléther et de préférence à une température généralement comprise entre 20 et 80 °C ou entre -50 °C et la température ambiante dans le cas particulier où c'est le diisopropylamidure de lithium qui est employé.

Les N,N-diméthylaminométhylindoles de formule générale **7** précédemment définie, sont préparés selon le schéma 3 suivant,

Schéma 3

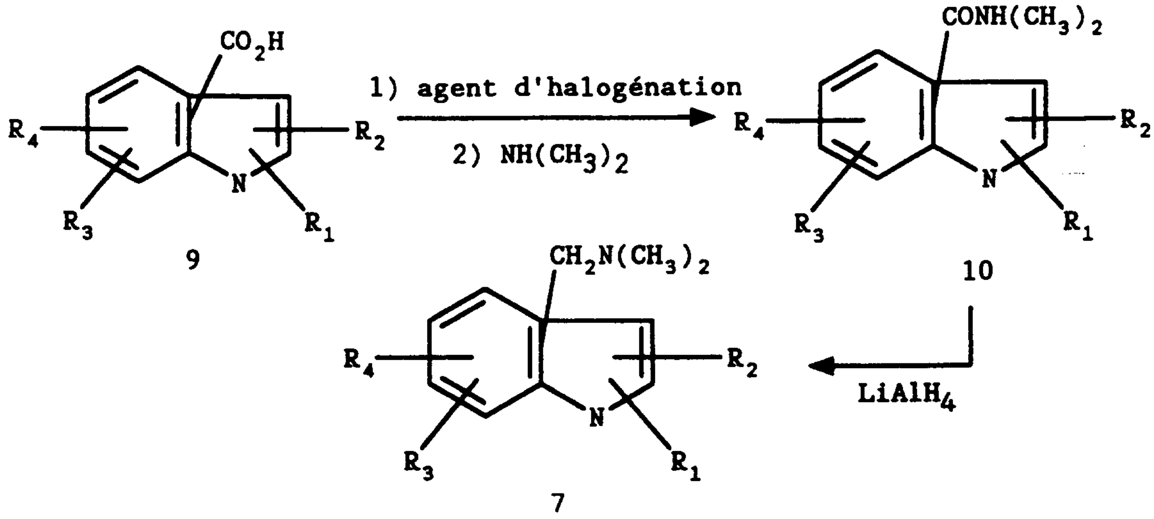


d'après lequel, on convertit un acide de formule générale **9**, dans laquelle $R_1$, $R_2$, $R_3$ et $R_4$ ont les significations générales ou particulières déja définies, en les N,N-diméthylcarboxamides de formule générale **10**, en le traitant successivement par un agent d'halogénation comme par exemple le trichlorure ou le pentachlorure de phosphore ou encore le chlorure de thionyle dans un solvant inerte comme par exemple un hydrocarbure aromatique tel que le benzène ou le toluène ou bien un solvant halogéné comme le chlorure de méthylène ou le chloroforme et à la température la mieux adaptée pour que se développe la réaction qui est généralement la température de reflux du solvant utilisé, puis par la N,N-diméthylamine; les N,N-diméthylindolecarboxamides de formule générale **10** ainsi formés sont ensuite réduits par un réducteur de la fonction carboxamide, de préférence l'hydrure de lithium et d'aluminium, au sein d'un solvant adapté comme par exemple le diéthyléther ou le tétrahydrofurane et à la température de reflux du solvant utilisé.

Les N,N-diméthylaminométhylindoles de formule générale **7** dans laquelle le radical N,N-diméthylaminométhyle se trouve en position -3 du cycle de l'indole peuvent aussi être préparés en traitant les norindoles correspondants dans les conditions de Mannich décrites par H.KUHN et O.STEIN, Chem.Ber., 1937, 70, 567 et par E.WALTON et collaborateurs, J.Med.Chem., 1965, 8, 204.

Les nitriles intermédiaires de formule générale **6** dans laquelle, Z représente un biradical de formule: $-CH=CH-C(R_7R_8)-$ dans laquelle $R_7$ et $R_8$ ont les significations générales ou particulières précédemment définies, peuvent être préparés selon le schéma 4 ci-après, d'après lequel on fait réagir un formylpropénylindole de formule générale **11**, dans laquelle $R_1$, $R_2$, $R_3$ et $R_4$ ont les significations générales ou particulières déja spécifiées, avec un diéthylphosphorocyanidate en présence de cyanure de lithium ou de diisopropylamidure de lithium dans le tétrahydrofurane pour donner le cyanophosphate **12** qui est ensuite traité par l'iodure de samarium pour donner le nitrile **13**; on opère à une température qui peut être comprise entre la température ambiante et la température de reflux du solvant utilisé. Le nitrile **13** est ensuite alkylé

Schéma 4

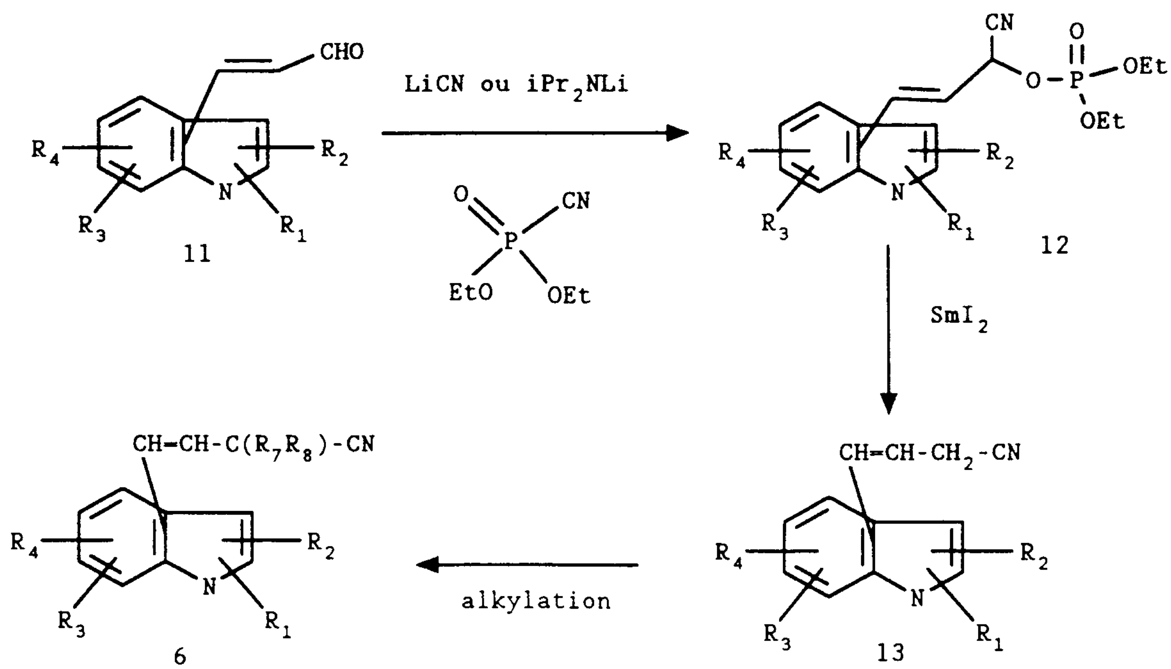


dans les conditions décrites précédemment pour donner le nitrile **6**.

Les aldéhydes de formule générale **11** peuvent être préparés par une réaction de Vilsmeier sur le norindole correspondant en utilisant la dimethylaminoacroléïne dans les conditions décrites par G.F.SMITH, J.Chem.Soc., 1954, 3842.

Les nitriles intermédiaires de formule générale **6**, dans laquelle Z désigne un biradical de formule: $-(CH_2)_n-C(R_7R_8)-(CH_2)p-$ dans laquelle $R_7$ et $R_8$ ont les significations générales ou particulières déja explicitées et n = 0 et p = 1, sont préparés selon le schéma 5 suivant,

**Schéma 5**

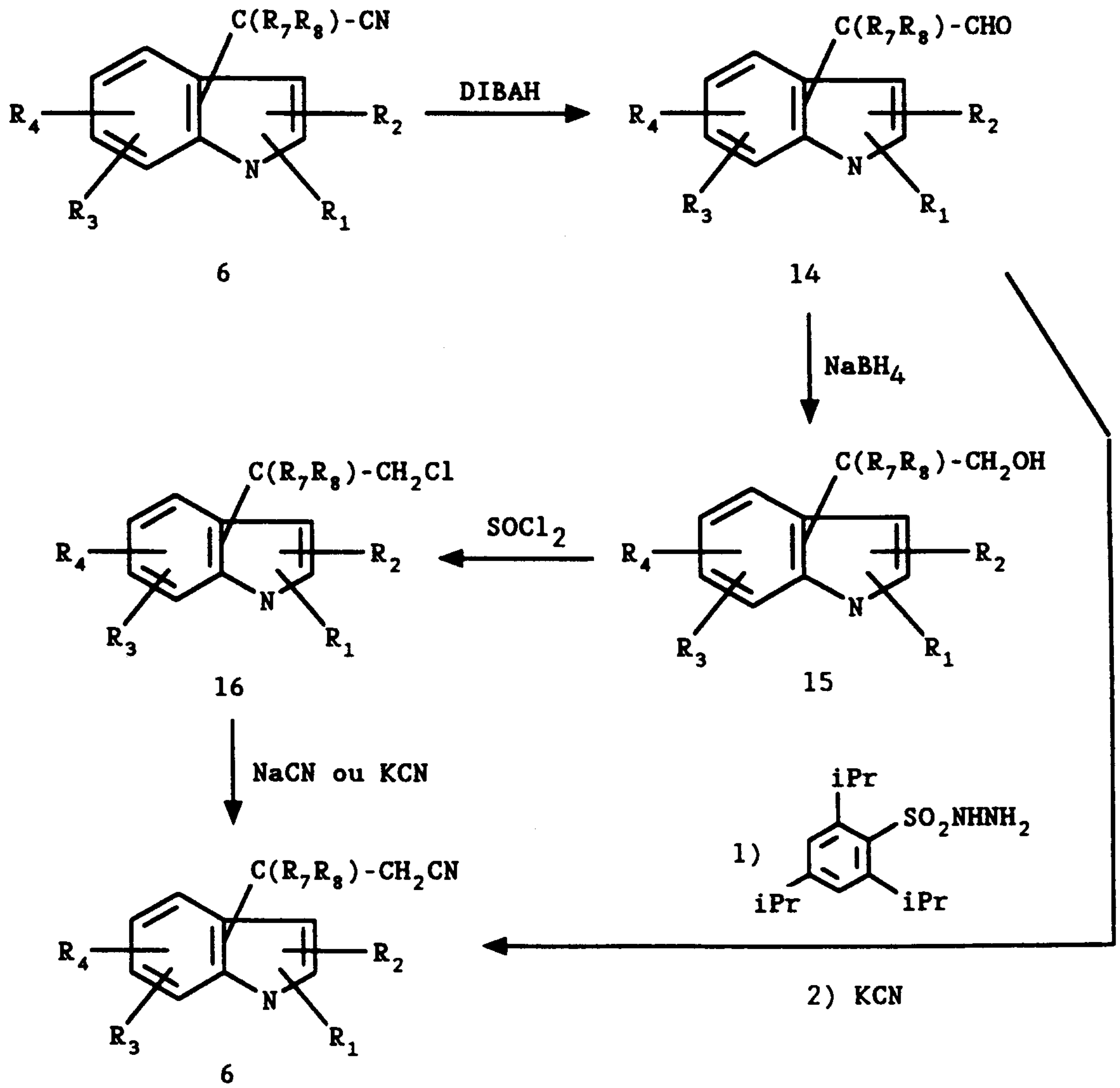


6

14

16

15

6

selon lequel on réduit un nitrile de formule générale **6**, dans laquelle $Z=-(CH_2)_n-C(R_7R_8)-(CH_2)_p-$, $n=p=0$, $R_7$ et $R_8$ ont les significations générales ou particulières déja définies, par l'hydrure de diisobutylaluminium, en l'aldéhyde correspondant de formule générale **14**, en utilisant comme solvant l'éther diéthylique ou le tétrahydrofurane ou encore le chlorure de méthylène ou bien le toluène, à une température qui se situe entre la température ambiante et la température d'ébullition du solvant utilisé, lequel aldéhyde est ensuite réduit en l'indole éthanol de formule générale **15**, par le borohydrure de sodium dans un solvant polaire comme le tétrahydrofurane ou un alcool comme le méthanol, l'éthanol ou l'isopropanol et à une température qui est de préférence la température d'ébullition du solvant employé; l'indole éthanol de formule générale **15** ainsi préparé est ensuite halogéné par le chlorure de thionyle ou tout autre agent d'halogénation similaire, dans le benzène ou dans un solvant chloré comme le chloroforme ou le chlorure de méthylène et à la température de reflux du solvant utilisé; le dérivé chloré de formule générale **16** ainsi obtenu est ensuite traité par le cyanure de sodium ou de potassium au sein d'un solvant ou d'un mélange de solvants polaires comme l'éthanol, le tétrahydrofurane ou le N,N-diméthylformamide ou d'un mélange éthanol-eau ou N,N-diméthylformamide-eau et à une température comprise entre la température ambiante et 80 °C, généralement plus proche de 80 °C que de la température ambiante.

Une alternative, selon le schéma 5, pour préparer les nitriles de formule générale **6** dans laquelle $Z=-(CH_2)_n-C(R_7R_8)-(CH_2)_p-$, $n=0$, $p=1$, $R_7$ et $R_8$ ont les significations générales ou particulières déja explicitées, consiste à faire réagir, à la température ambiante, un aldéhyde de formule générale **14** avec la triisopropyl-2,4,6-benzènesulfonohydrazide dans un solvant polaire comme par exemple le tétrahydrofurane

puis à traiter la sulfonylhydrazone correspondante ainsi obtenue par le cyanure de potassium de préférence dans un alcool comme le méthanol par exemple et à la température de reflux du solvant utilisé.

Les nitriles intermédiaires de formule générale **6**, dans laquelle Z représente un biradical de formule: $-(CH_2)_n-C(R_7R_8)-(CH_2)p-$ dans laquelle $R_7$ et $R_8$ ont les significations générales et particulières déjà définies et n = 1 et p = 0, sont préparés selon le schéma 6 suivant,selon

**Schéma 6**

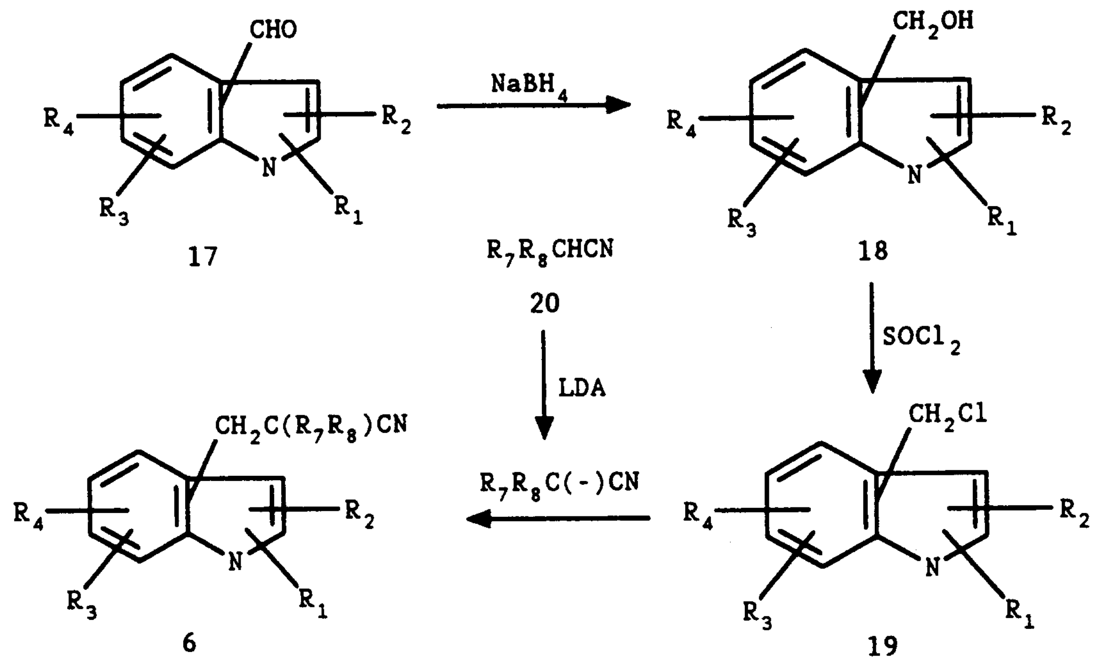


17 18 19 6

lequel, on convertit un aldéhyde de formule générale **17** en un halogénure de formule générale **19**, dans laquelle $R_1$, $R_2$, $R_3$ et $R_4$ ont les significations générales ou particulières précédemment définies, en utilisant la même séquence réactionnelle que celle du schéma 5 ci-dessus et selon les mêmes conditions opératoires; le chlorure de formule générale **19** est ensuite traité par un acétonitrile approprié de formule générale **20** préalablement lithié par une base forte qui est de préférence le diisopropylamidure de lithium; ces deux réactions sont réalisées dans un solvant adapté tel que l'éther diéthylique ou le tétrahydrofurane, à basse température, usuellement aux environs de -70 ° C jusqu'à la température ambiante pour ce qui est de la métallation du nitrile **20**, et généralement à la température de reflux du solvant utilisé pour ce qui est de la condensation du lithien avec le dérivé chloré **19**.

Les aldéhydes de formule générale **17** sont préparés selon le schéma 7 suivant, d'après lequel on réduit un indole

Schéma 7

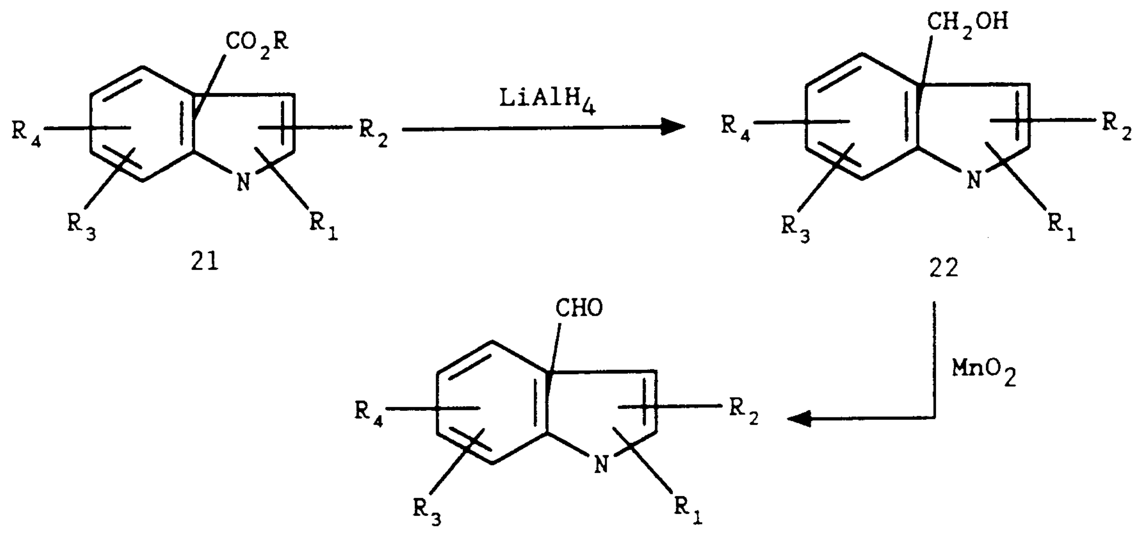


21

22

17

carboxylate d'alkyle de formule générale **21**, dans laquelle $R_1$, $R_2$, $R_3$ et $R_4$ ont les significations générales ou particulières précédemment spécifiées et R désigne un radical alkyle, de préférence le radical méthyle ou éthyle; cette réduction est réalisée par un réducteur de la fonction ester comme par exemple l'hydrure double de lithium et d'aluminium, dans le solvant adapté qui peut être par exemple l'éther ou le tétrahydrofurane et à la température du solvant utilisé; l'indole méthanol ainsi obtenu est ensuite oxydé par le bioxyde de manganèse dans les conditions décrites par J.HARLEY-MASON et E.H.PAVRI, J.Chem.Soc., 1963, 2565 et par H.PLIENINGER, M.HOBEL et V.LIEDE, Chem. Ber., 1963, 96, 1618.

Les aldéhydes de formule générale **17** dans laquelle le radical formyle se trouve en position -3 du cycle indolique peuvent également être préparés par une réaction de Vilsmeier sur le nor-indole correspondant dans les conditions décrites par G.F.SMITH J.Chem.Soc., 1954, 3842.

Les nitriles intermédiaires de formule générale **6**, dans laquelle Z représente un biradical de formule: $-C(R_7R_8)-CH=CH-$ dans laquelle $R_7$ et $R_8$ peuvent avoir les significations générales ou particulières déja énoncées, sont préparés selon le schéma 8 suivant, selon lequel on

Schéma 8

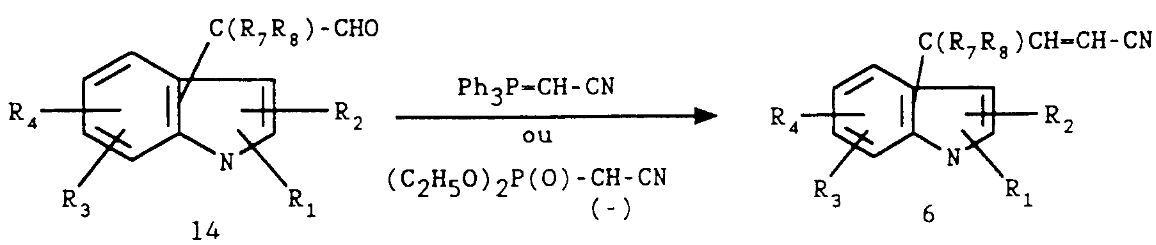


14

6

fait réagir le cyanométhylènetriphénylphosphorane ou le phosphonate correspondant sur un indole acétaldéhyde, de formule générale **14** précédemment définie, dans un solvant comme par exemple l'acétonitrile ou un hydrocarbure aromatique tel que le benzène ou le toluène et à une température qui peut être comprise entre la température ambiante et la température de reflux du solvant utilisé.

Les composés de formule **1** ont la propriété d'inhiber l'Acyl coenzyme A:Cholestérol Acyl Transférase (A.C.A.T.) et par voie de conséquence exercent une action hypolipidémiante et anti-athéromateuse; certains des composés de formule **1** manifestent également des propriétés hypoglycémiantes.

Ces propriétés des composés de l'invention rendent particulièrement intéressante leur utilisation comme médicament pour le traitement ou la prévention des hyperlipidémies, de l'athérosclérose et du

diabète d'autant plus que ces pathologies se trouvent souvent associées.

Les propriétés pharmacologiques des composés de l'invention ont été démontrées par les tests suivants,

**Test A:** mesure de l'inhibition de l'A.C.A.T. aortique *in vitro* chez le lapin: des lapins néo-zélandais mâles pesant entre 2,2 et 2,5 kg, préalablement soumis pendant 15 jours à un régime enrichi de 1,25 % en cholestérol, sont sacrifiés par dislocation cervicale; l'aorte est prélevée, disséquée et homogénéisée afin d'en préparer la fraction microsomiale par ultracentrifugation; ces microsomes sont incubés en présence de $^{14}$C-oléyl coenzyme A conformément à la méthode décrite par P.J.GILLIES et coll., Exp. and Mol. Pathol., 1986, 44, 329-339; on extrait les lipides de l'incubat par un mélange méthanol-chloroforme et le $^{14}$C-oléyl cholestérol est séparé par CCM: ce dernier représente la mesure de l'activité A.C.A.T. et les résultats sont exprimés sous forme de concentration inhibitrice 50 ($CI_{50}$) représentant la concentration de composé qui inhibe l'activité A.C.A.T. de 50 %.

**Test B:** mesure de l'effet hypocholestérolémiant chez le rongeur: des rats Wistar mâles de 200-220 g, sont soumis à un régime enrichi de 2,5 % en cholestérol pendant 8 jours; les deux derniers jours, ils sont traités oralement par le produit à tester, 24 heures et 4 heures avant leur sacrifice par exsanguination: la cholestérolémie est évaluée sur une fraction aliquote de sérum par une méthode enzymatique automatisée. Les résultats sont exprimés sous forme de dose efficace 25 ($DE_{25}$) en mg par kg d'animal et qui représente la quantité de composé qui abaisse la cholestérolémie de 25 %.

**Test C:** mesure de l'inhibition de l'absorption intestinale chez le rat; des rats Wistar mâles de 230-250 g, à jeun depuis 24 heures, sont traités simultanément par le produit à tester administré *per os* et par du triton WR1339 administré par voie I.V.; une heure plus tard, on les traite à nouveau oralement par du $^{3}$H-cholestérol; trois heures plus tard, sous anesthésie à l'éther, on leur prélève 1 ml de sang au sinus rétro-orbitaire: la radioactivité sanguine, évaluée sur 0,1 ml de sérum, représente la mesure de l'absorption du $^{3}$H-cholestérol administré. Les résultats sont exprimés sous forme de dose efficace 50 ($DE_{50}$) en mg par kg d'animal et qui représente la quantité de composé qui inhibe l'absorption intestinale du cholestérol de 50 %.

**Test D:** mesure de l'effet hypoglycémiant: des souris Swiss mâles de 26-28 g sont traitées *per os* par le produit à tester. Une heure plus tard, les animaux sont sacrifiés par exsanguination, et on mesure la glycémie sur une fraction aliquote de plasma, par une méthode enzymatique utilisant la glucose-oxydase.

On a par exemple obtenu pour le composé n°1 sur le test A, une $CI_{50}$ de 46 x $10^{-9}$ Mole.$l^{-1}$, sur le test B, une $DE_{25}$ de 0,098 mg.$kg^{-1}$, sur le test C, une $DE_{50}$ de 0,176 mg.$kg^{-1}$ et sur le test D, une baisse significative de la glycémie de 21 % à la dose de 60 mg. $kg^{-1}$.

Les toxicités animales des composés de l'invention ont été également évaluées notamment chez le rat et la souris, il s'est avéré que les composés de l'invention sont particulièrement bien tolérés puisque à des doses supérieures à 3200 mg.$kg^{-1}$ *per os* il n'a été observé aucune mortalité.

Ces propriétés des composés de l'invention rendent particulièrement intéressante leur utilisation comme médicaments notamment pour le traitement des hyperlipidémies,de l'athérosclérose et du diabète.

Les médicaments de l'invention sont caractérisés en ce qu'ils renferment une quantité efficace d'au moins un composé de formule générale **1** en association avec un véhicule pharmacologiquement acceptable et le cas échéant avec tout autre produit acceptable du point de vue pharmaceutique qui peut être inerte ou physiologiquement actif.

Ces médicaments peuvent être administrés selon une grande variété de formes d'administration différentes, par exemple sous des formes solides, telles que comprimés, capsules, granules, gélules, poudres, suppositoires etc..., ou sous formes liquides telles que sirops, élixirs ou solutions injectables; dans ces compositions le principe actif peut être par exemple mélangé à un ou plusieurs diluants inertes tels que lactose ou amidon, en outre ces compositions peuvent comprendre des substances autres que des diluants par exemple des lubrifiants tels que talc ou stéarate de magnésium; lorsqu'on désire des suspensions aqueuses, élixirs ou sirops pour administration orale, l'ingrédient actif essentiel peut y être associé à divers édulcorants et/ou aromatisants, le cas échéant des émulsionnants et/ou des agents de mise en suspension en même temps que des diluants tels que l'eau, l'éthanol, le propylèneglycol, et diverses associations similaires.

Ces compositions pharmaceutiques selon l'invention se présentent généralement sous forme de dose unitaire pouvant contenir une quantité de principe actif comprise entre 10 et 100 % de leur poids total et de préférence située dans l'intervalle 10 à 60 %.

Lorsque le composé de l'invention est utilisé comme agent hypolipidémiant, ou anti-athéroscléreux, ou dans le traitement du diabète, le dosage adopté et le nombre des administrations sont fonction du sexe, du poids et de l'acuité des symptômes du patient ainsi que de la nature et du degré de l'effet thérapeutique

escompté. Généralement, par voie orale il est administré de préférence aux doses de 10 à 500 mg par jour en une ou plusieurs fractions ce qui correspond pour un adulte d'un poids moyen de 70 kg à un intervalle de doses d'environ 0,15 à 7 mg par kilo et par jour.

L'exemple suivant, donné à titre non limitatif, illustre une composition de ce type,

**Example:**

| | |
|---|---|
| Principe actif: composé n° 1 | 50 mg |
| Lactose | 69 mg |
| Phosphate dicalcique | 69 mg |
| Carboxyméthyl cellulose sodique | 10 mg |
| Stéarate de magnésium | 2 mg |

L'invention est illustrée par les exemples non limitatifs décrits ci-après dans lesquels:

. toutes les évaporations sont exécutées, sauf indication contraire, dans un évaporateur rotatif sous pression réduite,
. les températures sont exprimées en degrés centigrade (° C),
. lorsqu'il est indiqué "température ambiante", il s'agit d'une température qui peut être comprise entre 18 et 25 ° C,
. sauf indication contraire, le degré d'avancement de la réaction est contrôlé par chromatographie en couche mince (CCM),
. les nouveaux composés sont le cas échéant caractérisés par leurs constantes physiques: point de fusion noté PF ou point d'ébullition noté Eb suivi éventuellement de l'indication de la pression exprimée en millibars,
. les spectres de résonnance magnétique nucléaire sont sauf indication contraire ceux du proton et sont relevés à 60 MHz en présence de tétraméthylsilane comme étalon interne, les déplacements chimiques sont indiqués en ppm; les signaux sont décrits par les abréviations suivantes: s = singulet, d = doublet, qs = quasisingulet, dd = doublet de doublet, t = triplet, q = quartet, hept. = heptuplet, M = multiplet,
. les spectres infra-rouge des composés sont enregistrés à partir d'échantillons dispersés dans le bromure de potassium dans le cas des composés solides ou bien en film dans le cas des liquides.

**Example 1:**

**N1-(Diisopropyl-2,6-phényl)-N2-[(méthyl-1-indolyl-3)-1-cycl opentylméthyl]urée** (composé n° 1,formule 1:$R_1$ = 1-$CH_3$,-$R_2$ = $R_3$ = $R_4$ = $R_5$ = H,$R_6$ = 2,6-diisopropylphényle,Z = $-(CH2)_n-C(R_7R_8)-(CH_2)_p-$ en position -3,n = p = 0,$R_7$-$R_8$ = $-(CH_2)_4-$).

Stade 1:

**Méthyl-1-indolyl-3-acétonitrile** ($C_{11}H_{10}N_2$-PM = 170,21).

A un mélange de 7,8g (0,05 mole) d'indolyl-3-acétonitrile, de 14,2g (0,1 mole) d'iodure de méthyle et de 0,83g d'une solution de triton B dans le méthanol, on ajoute à un débit tel que la température du mélange réactionnel n'excède pas 35 ° C (1h 30 environ), 20 $cm^3$ d'une solution aqueuse de soude à 50% puis on agite le mélange réactionnel pendant 3 heures à la température ambiante; on y ajoute ensuite 100 $cm^3$ d'eau en refroidissant et on extrait à l'éther diéthylique, on lave ensuite l'extrait éthéré à l'eau jusqu'à neutralité, on le sèche sur sulfate de sodium puis on le filtre, on évapore l'éther et on cristallise le résidu dans la quantité suffisante de pentane; le solide ainsi obtenu est essoré puis séché.

PF = 59-60 ° C. Rendement = 4,1g = 48%.

CCM ($SiO_2$/hexane-acétate d'éthyle:3-1):$R_f$ = 0,43.

IR: $\nu$CN = 2240$cm^{-1}$

RMN ($CDCl_3$): 3,65(s,3H); 3,70(s,2H); 6,9-7,6(M,5H).

Stade 2:

**(Méthyl-1-indolyl-3)-1-cyclopentanecarbonitrile** ($C_{15}H_{16}N_2$-PM = 224,30).

On opère sous atmosphère d'azote sec. A une suspension de 2,1g d'hydrure de sodium à 60% dans l'huile (0,048 mole + 10%), préalablement lavé de son huile, dans 30 $cm^3$ de diméthylsulfoxyde, on ajoute un mélange comprenant 4,1g (0,024 mole) de composé du stade 1, 5,7g (0,024 mole) de dibromo-1,4-butane et 60 $cm^3$ d'éther diéthylique; le débit de cette addition est réglé de façon à maintenir un doux reflux du mélange que l'on maintient ensuite au reflux, par chauffage, pendant 4 heures puis on y ajoute 150 $cm^3$ d'eau et on extrait à l'éther diéthylique, on lave ensuite l'extrait éthéré à l'eau jusqu'à neutralité, on le sèche sur sulfate de sodium puis on le filtre, on évapore l'éther et on chromatographie le résidu sur une colonne d'alumine (40g) en utilisant l'éther diéthylique comme éluant; la première fraction éluée, fournit après évaporation de l'éther un solide qu'on cristalise par dispersion dans le pentane; on filtre ce solide et on le sèche.

PF = 118-120 ° C. Rendement = 2,9g = 54%.

CCM ($SiO_2$/hexane-acétate d'éthyle:3-1):$R_f$ = 0,5.

IR: $\nu$CN = 2223 $cm^{-1}$.

RMN ($CDCl_3$): 1,8-2,2(M,4H); 2,2-2,7(M,4H); 3,7(s,3H); 6,9-7,8 (M,5H).

Stade 3 :

**(Méthyl-1-indolyl-3)-1-cyclopentylméthylmine** ($C_{15}H_{20}N_2$-PM = 228,33).

On opère sous atmosphère d'azote sec. A 0,73g (0,0193 mole) d'hydrure de lithium et d'aluminium dans 18 $cm^3$ d'éther diéthylique, on ajoute goutte à goutte, de façon à maintenir un doux reflux de l'éther, une solution de 2,9g (0,0129 mode) de composé du stade 2 dans 100 $cm^3$ d'éther diéthylique, puis on chauffe le mélange pendant 3h30 au reflux; on y ajoute ensuite de la soude aqueuse diluée jusqu'à destruction du complexe de lithium et d'aluminium, on décante la phase éthérée, on la sèche sur sulfate de sodium puis on la filtre et on évapore l'éther du filtrat; on obtient ainsi une huile qu'on dissout dans la quantité suffisante d'hexane, on filtre cette solution puis on évapore le filtrat, on isole ainsi une huile qui est utilisée telle quelle dans la suite de la synthèse.

Rendement = 2,6g = 88%.

CCM ($SiO_2$/acétate d'éthyle-dichlorométhane-méthanol:4-5-1):1 spot étalé.

IR: $\nu NH_2$ = 3370 et 3390 $cm^{-1}$.

Stade 4:

**N1- (Diisopropl-2,6-phényl)-N2-[(méthyl-1-indolyl-3)-1-cyclopentylméthyl)urée** (composé n ° 1).

A une solution de 2,6g (0,0144 mode) de composé du stade 3 dans 50 $cm^3$ d'hexane, on ajoute goutte à goutte une solution de 2,03g (0,01 mode) de diisopropyl-2,6-phénylisocyanate dans 20 $cm^3$ d'hexane et on agite le mélange pendant 2 heures à la température ambiante; on essore ensuite le précipité obtenu, on le lave au diisopropyléther puis au pentane et on le sèche.

PF = 193-195 ° C (diisopropyléther). Rendement = 2g = 46%.

IR: $\nu$CO = 1638 $cm^{-1}$.

RMN ($CDCl_3$): 0,9(d,12H); 1,5-2(M,8H); 3(hept.,2H); 3,45(s,3H); 3,2-4(M,3H); 5,85(s,1H); 6,2(s,1H); 6,8-7,6-(M,7H).

| Analyse centésimale ($C_{28}H_{37}N_3O$-PM = 431,60): | | | |
|---|---|---|---|
| | C | H | M |
| %calculé<br>%trouvé | 77,92<br>77,63 | 8,64<br>8,80 | 9,74<br>9,66 |

**Exemple 2:**

**N1-[(Indolyl-1)-1-cyclopentylméthyl]-N2-(diisopropyl-2,6-phényl)urée** (composé n ° 2, formule 1:$R_1 = R_2 = R_3 = R_4 = R_5$ = H,$R_6$ = 2,6-diisopropylphényle,Z = $-(CH_2)_n-C(R_7R_8)-(CH_2)_p-$en position-1, n = p = 0,$R_7-R_8 = -(CH_2)_4-$).

Stade 1:

**Indolyl-1-acétonitrile** ($C_{10}H_8N_2$-PM = 156,18).

On opère sous atmosphère d'azote sec. A une suspension de 8g d'hydrure de sodium à 60% dans l'huile (0,2 mole), préalablement lavé de son huile, dans 150 $cm^3$ de diméthylformamide, on ajoute 23,4g (0,2 mole) d'indole en solution dans 50 $cm^3$ de diméthylformamide; on règle le débit de cette addition de façon à ce que la température du mélange réactionnel n'excède pas 40 ° C; on agite ensuite le mélange pendant 1h à la température ambiante puis on y ajoute goutte à goutte, de façon à ce que la température n'excède pas 40 ° C, 15,1g (0,2 mole) de chloracétonitrile en solution dans 40 $cm^3$ de diméthylformamide; on agite ensuite le mélange pendant 3 heures à la température ambiante puis on y ajoute 300 $cm^3$ d'eau, on extrait à l'éther diéthylique, on lave l'extrait éthéré à l'eau jusqu'à neutralité, on le sèche sur sulfate de sodium, on le filtre et on évapore le filtrat à sec; on isole ainsi un solide qu'on disperse dans le diisopropyléther, puis on le filtre et on le sèche.

PF = 75-77 ° C. Rendement = 8,1g = 26%.

CCM ($SiO_2$/hexane-acétate d'éthyle:2-1):$R_f$ = 0,63.

IR: $\nu$CN = 2245 $cm^{-1}$.

RMN ($CDCl_3$): 4,8(s,2H); 6,52(d,1H); 6,98(d,1H); 7,2-7,7(M,4H).

Stade 2:

**(Indolyl-1)-1-cyclopentanecarbonitrile** ($C_{14}H_{14}N_2$-PM = 210,27).

Préparé à partir du composé du stade 1, par le procédé décrit au stade 2 de l'exemple 1.

Rendement = 77%.

CCM ($SiO_2$/hexane-acétate d'éthyle:2-1).

IR: $\nu$CN = 2237 $cm^{-1}$.

RMN ($CDCl_3$): 1,7-2,1(M,4H); 2,4-2,9(M,4H); 6,45(d,1H); 7-7,7(M,5H).

Stade 3:

**(Indolyl-1)-1-cyclopentylméthylamine** ($C_{14}H_{18}N_2$-PM = 214,30).

Préparé à partir du composé du stade 2, par le procédé décrit au stade 3 de l'exemple 1. le composé obtenu est utilisé brut.

Rendement = 81%

IR $\nu NH_2$ = 3381 et 3310 $cm^{-1}$.

RMN ($CDCl_3$): 0,9(s,2H); 1,5-2(M,4H); 2,6-3,1(M,4H); 3,05(s,2H); 6,4(d,1H); 7-7,8(M,5H).

Stade 4:

**N1-[(Indolyl-1)-1-cyclopentylméthyl]-N2-(diisopropyl-2,6-phényl)urée** (composé n ° 2).

Préparé à partir du composé du stade 3, par le procédé décrit au stade 4 de l'exemple 1.

PF = 188-190 ° C. Rendement = 63%.

IR: $\nu$CO = 1637 $cm^{-1}$.

RMN ($CDCl_3$): 1(d,12H); 1,5-2(M,4H); 2-2,5(M,4H); 3(hept.,2H); 3,55(s,2H); 3,2-4(large,1H); 6,05(d,1H); 6,2-(s,1H); 6,5-7,6(M,8H).

| Analyse centésimale ($C_{27}H_{35}N_3O$-PM = 417,57): | | | |
|---|---|---|---|
| | C | H | N |
| %calculé | 77,66 | 8,45 | 10,06 |
| %trouvé | 77,50 | 8,66 | 10,01 |

Exemple 3:

**N1-(Diisopropyl-2,6-phényl)-N2-[[(méthyl-1-indolyl-3)-1-cyclopentyl]-3-propyl]urée** (composé n ° 3,formule 1:$R_1$ = 1-$CH_3$,$R_2$ = $R_3$ = $R_4$ = $R_5$ = H,$R_6$ = 2,6-diisopropylphényle,Z = $-(CH_2)_n-C(R_7R_8)-(CH_2)_p-$ en position-3,n = 0,p = 2,$R_7$-$R_8$ = $-(CH_2)_4-$).

Stade 1:

**(Méthyl-1-indolyl-3)-1-cyclopentanecarboxaldéhyde** ($C_{15}H_{17}NO$-PM = 227,30).
On opère sous atmosphère d'azote sec. A une solution de 13,3g (0,059 mole) de (méthyl-1-indolyl-3)-1-cyclopentanecarbonitrile dans 230 $cm^3$ de toluène, on ajoute à -60 ° C, 94,4 $cm^3$ (0,0944 mole) d'une solution toluènique 1N d'hydrure de diisobutylaluminium. On laisse ensuite remonter la température du mélange à la température ambiante puis on stoppe la réaction par addition au mélange réactionnel de 50 $cm^3$ de méthanol puis de 230 $cm^3$ d'acide chlorhydrique 3N. On extrait le mélange au dichlorométhane, on lave l'extrait organique à l'eau, on le sèche sur sulfate de sodium, on le filtre et on évapore le filtrat. Successivement, on reprend le résidu obtenu par l'éther diisopropylique, on élimine un insoluble par filtration, on évapore le solvant et on cristallise le résidu dans l'hexane.
PF = 69-70 ° C.Rendement = 7,1g = 53%.
IR: $\nu CO = 1714 cm^{-1}$
RMN($CDCl_3$):1,4-3(M,8H);3,7(s,3H);6,9(s,1H);6,9-7,6(M,4H) ;9,4(s,1H).

Stade 2:

**(E/Z)-[(Méthyl-1-indolyl-3)-1-cyclopentyl]-3-propène-2-nitrile** ($C_{17}H_{18}N_2$-PM = 250,33).
On opère sous atmosphère d'azote sec. A une suspension de 1,4g (0,033 mole + 5%) d'hydrure de sodium en suspension à 60% dans l'huile dans 40 $cm^3$ de tétrahydrofurane, on ajoute goutte à goutte à une température comprise entre 20 et 25 ° C (refroidissement par bain de glace), 5,3 $cm^3$ (0,033 mole) de cyanométhylphosphonate de diéthyle. On agite ensuite le mélange pendant 1 heure à la température ambiante puis on y ajoute 7,5g (0,033 mole) de composé du stade 1 en solution dans 40 $cm^3$ de tétrahydrofurane, on agite pendant 1 heure supplémentaire puis on chauffe au reflux pendant 2,5 heures. Après refroidissement du mélange réactionnel, successivement on y ajoute de l'eau glacée, on extrait à l'éther éthylique, on lave l'extrait éthéré à l'eau, on le sèche sur sulfate de sodium, on le filtre, on évapore le filtrat et on cristallise le résidu dans l'hexane.
PF = 57-59 ° C.Rendement = 6g = 73%.
RMN($CDCl_3$):1,5-2,5(M,8H);3,7(s,3H);4,95-5,1(d,J = 16,5,J = 1 2,1H);6,6-7,7(M,6H).

Stade 3:

**[(Méthyl-1-indolyl-3)-1-cyclopentyl]-3-propylamine** ($C_{17}H_{24}N_2$-PM = 250,33)
On hydrogène sous pression, 2,503g (0,01 mole) de composé du stade 2 en solution dans 70 $cm^3$ d'éthanol préalablement saturé d'ammoniac et en présence de 2g de nickel de Raney. Après 5 heures de chauffage à 70 ° C, on filtre le mélange, on évapore à sec le filtrat.
Huile.Rendement = 2,1g = 84%.
CCM($SiO_2$/dichlorométhane-méthanol:4-1).
IR: $\nu NH_2 = 2360 cm^{-1}$.
RMN($CDCl_3$): 0,8-3(M,16H);3,6(s,3H);6,7(s,1H);6,8-7,6(M,4H).

Stade 4:

**N1-(Diisopropyl-2,6-phényl)-N2-[(méthyl-1-indolyl-3)-1-cyclopentyl]-3-propyl]urée** (composé n ° 3).
On chauffe à 110 ° C pendant 30 minutes une solution de 5,25g (0,02 mole + 5%) de composé de stade 3 et de 6,45g (0,02 mode) de trichloro-2,2,2-N-(diisopropyl-2,6-phényl)acétamide dans 30 $cm^3$ de N,N-diméthylformamide en présence de 8,3g (0,06 mode) de carbonate de potassium. Après refroidissement, on ajoute au mélange réactionnel 200 $cm^3$ d'eau froide, on essore le précipité formé, on le lave à l'eau puis on le sèche. On recristallise dans l'éthanol.
PF = 194-196 ° C.Rendement = 6,89g = 75%.
IR: $\nu CO = 1634 cm^{-1}$
RMN($DMSOd_6$):1,1(d,12H);1,5-2,2(M,12H);2,7-3,4(M,4H);3,25(s, 1H);3,7(s,3H);5,75(large,1H);6,8-7,8(M,8H).

| Analyse centésimale($C_{30}H_{41}N_3O$-PM = 459,68): | | | |
|---|---|---|---|
| | C | H | N |
| %calculé:<br>%trouvé: | 78,39<br>78,30 | 8,99<br>8,94 | 9,14<br>9,11 |

**Example 4:**

**N1-(Diisopropyl-2,6-phényl)-N2-[[(méthyl-1-indolyl-3)-1-cyclopentyl)-2-éthyl]urée** (composé n° 4, formule 1: $R_1$ = 1-$CH_3$, $R_2$ = $R_3$ = $R_4$ = $R_5$ = H, $R_6$ = 2,6-diisopropylphényle, Z = $-(CH_2)_n-C(R_7R_8)-(CH_2)_p-$ en position-3, n = 0, p = 1, $R_7$-$R_8$ = $-(CH_2)_4-$).

Stade 1:

**(Méthyl-1-indolyl-3)-1-cyclopentaneacétonitrile** ($C_{16}H_{18}N_2$-PM = 238,32).
On agite à la température ambiante pendant 3 heures 2,9g (0,0134 mole) de (méthyl-1-indolyl-3)-1-cyclopentanecarboxaldéhyde et 5g (0,0166 mole) de triisopropyl-2,4,6-benzènesulfonohydrazide en solution dans 30 $cm^3$ de tétrahydrofurane. On évapore ensuite complètement le tétrahydrofurane et on ajoute au résidu, 2,6g (0,0402 mole) de cyanure de potassium et 30 $cm^3$ de méthanol. On chauffe le mélange au reflux pendant 4,5 heures puis on le refroidit, on ajoute de l'eau et on extrait au dichlorométhane. Successivement, on lave l'extrait organique obtenu avec une solution aqueuse d'hydrogénocarbonate de sodium, on le sèche sur sulfate de sodium, on le filtre et on évapore le solvant. On purifie le produit brut ainsi isolé par chromatographie sur colonne de gel de silice dans le dichlorométhane puis le cas échéant par "flash chromatography" en utilisant le mélange hexane-acétate d'éthyle:3-1 comme éluant.
Huile. Rendement = 0,8g = 25%.
IR: $\nu$CN = 2250 $cm^{-1}$.
RMN($CDCl_3$): 1,5-2,7(M,8H); 2,8(s,2H); 3,7(s,3H); 6,8-8(M,5H).

Stade 2:

**[(Méthyl-1-indolyl-3)-1-cyclopentyl]-2-éthylamine** ($C_{16}H_{22}N_2$-PM = 242,36).
Préparé par réduction par l'hydrure de lithium et d'aluminium du composé du stade 1 exactement selon le procédé du stade 3 de l'exemple 1.
Huile. Rendement = 74%

Stade 3:

**N1-(Diisopropyl-2,6-phényl)-N2-[[(méthyl-1-indolyl-3)-1-cyclopentyl]-2-éthyl]urée** (composé n° 4).
Préparé par condensation du diisopropyl-2,6-phénylisocyanate sur le composé du stade 2 dans les conditions de l'exemple 1.
PF = 239-241 °C (acétone). Rendement = 25%.
IR: $\nu$NH = 3145,3252$cm^{-1}$; $\nu$CO = 1647$cm^{-1}$.

| Analyse centésimale($C_{29}H_{39}N_3O$-PM = 445,65): | | | |
|---|---|---|---|
| | C | H | N |
| %calculé:<br>%trouvé: | 78,16<br>77,85 | 8,82<br>8,92 | 9,43<br>9,20 |

**Exemple 5:**

**N1-Benzyl-N2-(diisopropyl-2,6-phényl)-N1-[(méthyl-1-indolyl -3)-1-cyclopentylméthyl]urée** (composé n° 5, formule 1: $R_1$ = 1-$CH_3$, $R_2$ = $R_3$ = $R_4$ = H, $R_5$ = $C_6H_5CH_2$, $R_6$ = diisopropyl-2,6-phényle , Z = $-(CH_2)_nC(R_7R_8)-(CH_2)_p-$ en position-3, n = p = 0, $R_7$-$R_8$ = --$(CH_2)_4$-.

Stade 1 :

**N-[(Méthyl-1-indolyl-3)-1-cyclopentylméthyl]benzamide** ($C_{22}H_{24}N_2O$-PM = 332,45).

On opère sous atmosphère d'azote sec. A une solution de 16g (0,07 mole) de (méthyl-1-indolyl-3)-1-cyclopentylméthylamine et de 7,08g (0,07 mole) de triéthylamine dans 70 $cm^3$ d'éther diéthylique, on ajoute goutte à goutte de façon à maintenir un doux reflux de l'éther, 9,8g (0,07 mole) de chlorure de benzoyle en solution dans 70 $cm^3$ d'éther diéthylique puis on agite le mélange pendant 2 heures à la température ambiante. On essore ensuite le précipité formé, on lave la solution éthérée à l'eau, on la sèche sur sulfate de sodium, on la filtre et on évapore l'éther du filtrat.On cristallise le résidu ainsi obtenu par lavage avec du diisopropyléther froid.

PF = 109-111 ° C.Rendement = 87%.

CCM($SiO_2$/hexane-acétate d'éthyle:2-1):$R_f$ = 0,45.

IR: $\nu$NH = 3340-3324 $cm^{-1}$; $\nu$CO = 1640$cm^{-1}$.

Stade 2:

**N-Benzyl-N-[(méthyl-1-indolyl-3-)-1-cyclopentylméthyl]amine** ($C_{22}H_{26}N_2$-PM = 318,46).

On opère sous atmosphère d'azote sec. A 4,6g (0,0607 mole) d'hydrure de lithium et d'aluminium en suspension dans 125 $cm^3$ d'éther diéthylique, on ajoute goutte à goutte, de façon à maintenir un doux reflux de l'éther, 20,2g (0,0607 mole) du composé du stade 1 en solution dans 250 $cm^3$ d'un mélange 1-1 de diéthyléther et tétrahydrofurane. On chauffe à reflux pendant 42 heures. Après 8 heures de chauffage à reflux, on remplace l'éther par du tétrahydrofurane. En fin de chauffage, on refroidit le mélange à 20 ° C et on y ajoute la quantité suffisante de soude aqueuse diluée pour détruire le complexe de lithium et d'aluminium. On essore le précipité formé, on le lave à l'éther qu'on joint au filtrat, et on évapore complètement les solvants du filtrat.

Huile.Rendement = 75%.

CCM($SiO_2$/hexane-acétate d'éthyle:1-1): $R_f$ = 0,40.

RMN($CDCl_3$):1,2(large,1H); 1,5-2,2(M,8H); 2,8(s,2H); 3,6(s,2H); 3,65(s,3H); 6,8-7,7(M,10H).

Stade 3:

**N1-Benzyl-N2-(diisopropyl-2,6-phényl)-N1-[(méthyl-1-indolyl 3)-1-cyclopentylméthyl]urée** (composé n ° 5)

Préparé à partir du composé du stade 2 et du diisopropyl-2,6-phénylisocyanate exactement selon le procédé décrit au stade 4 de l'exemple 1.

PF = 101-103 ° C (diisopropyléther).Rendement = 66%.

CCM($SiO_2$/hexane-acétate d'éthyle:2-1): $R_f$ = 0,70.

RMN($CDCl_3$):0,9-1,2(M,12H); 1,5-2,3 (M,8H); 2,8(hept.,2H); 3,7(s,3H); 3,9(s,2H); 3,95(s,2H); 5,4(large,1H); 6,8(s,1H); 7-7,9(M,12H).

| Analyse centésimale($C_{35}H_{43}N_3O$-PM = 521,75). | | | |
|---|---|---|---|
| | C | H | N |
| %calculé: | 80,57 | 8,31 | 8,05 |
| %trouvé: | 80,71 | 8,43 | 7,99 |

**Exemple 6:**

En utilisant les procédés appropriés des exemples 1 à 5, on a préparé les composés (voir tableau 1 ci-dessous) de formule générale 1 dans laquelle, $R_2 = R_4 = R_5 = H$,Z = $-(CH_2)_n-C(R_7R_8)-(CH_2)_p-$ en position -3,n = p = 0.

Les significations des abréviations utilisées dans les tableaux 1 à 3 ci-après, sont les suivantes, Me = $CH_3$-, iPr = $(CH_3)_2CH$-, Et = $CH_3CH_2$-, allyl = $CH_2$ = CH-$CH_2$-, n.Bu = n.$C_4H_9$-,DMAE = $(CH_3)_2N$-$CH_2CH_2$-, 4F-Bn = 4F-$C_6H_4CH_2$-, pyrim = pyrimidinyle, MeO = $CH_3O$-, iPrO = $(CH_3)_2CHO$, pyr = pyridyle.

Tableau 1

| composé n° | $R_1$ | $R_3$ | $R_6$ | $R_7$ | $R_8$ | PF°C |
|---|---|---|---|---|---|---|
| 6 | 1-Me | H | $C_6H_5$ | $-(CH_2)_4-$ | | 214-216 |
| 7 | H | H | 2,6-$iPr_2C_6H_3$ | H | H | 160-162 |
| 8 | 1-Me | H | 2,4-$F_2C_6H_3$ | $-(CH_2)_4-$ | | 226-228 |

Tableau 1 (suite)

| composé n° | $R_1$ | $R_3$ | $R_6$ | $R_7$ | $R_8$ | PF°C |
|---|---|---|---|---|---|---|
| 9 | 1-Me | H | $2,6\text{-}iPr_2C_6H_3$ | iPr | H | 155-157 |
| 10 | 1-Me | H | $C(Me)_3$ | $-(CH_2)_4-$ | | 214-216 |
| 11 | 1-Me | H | $2,6\text{-}iPr_2C_6H_3$ | Me | H | 207-209 |
| 12 | $1\text{-}CH_2C_6H_5$ | H | $2,6\text{-}iPr_2C_6H_3$ | $-(CH_2)_4-$ | | 83-86 |
| 13 | 1-Et | H | $2,6\text{-}iPr_2C_6H_3$ | $-(CH_2)_4-$ | | 166-168 |
| 14 | 1-Me | H | $2,6\text{-}iPr_2C_6H_3$ | Et | H | 159-161 |
| 15 | 1-Me | 5-Me | $2,6\text{-}iPr_2C_6H_3$ | $-(CH_2)_4-$ | | 188-190 |
| 16 | 1-Me | H | $2,6\text{-}iPr_2C_6H_3$ | $-(CH_2)_5-$ | | 187-189 |
| 17 | 1-Me | H | $2,6\text{-}iPr_2C_6H_3$ | Et | Et | 183-184 |
| 18 | 1-Me | H | $2,6\text{-}iPr_2C_6H_3$ | Me | Me | 172-174 |
| 19 | 1-iPr | H | $2,6\text{-}iPr_2C_6H_3$ | $-(CH_2)_4-$ | | 168-170 |
| 20 | 1-allyl | H | $2,6\text{-}iPr_2C_6H_3$ | $-(CH_2)_4-$ | | 131-133 |
| 21 | 1-Me | H | $2,6\text{-}iPr_2C_6H_3$ | $C_6H_5$ | H | 168-170 |
| 22 | 1-Me | H | $2,6\text{-}iPr_2C_6H_3$ | allyl | allyl | 121-123 |
| 23 | $1\text{-}C_6H_5$ | H | $2,6\text{-}iPr_2C_6H_3$ | $-(CH_2)_4-$ | | 121-123 |
| 24 | 1-Me | H | $2,6\text{-}iPr_2C_6H_3$ | $-(CH_2)_3-$ | | 201-203 |
| 25 | 1-Me | H | $2,6\text{-}iPr_2C_6H_3$ | n.Bu | n.Bu | 93-96 |
| 26 | 1-Me | H | $2,6\text{-}iPr_2C_6H_3$ | allyl | H | 148-150 |

Tableau 1 (suite et fin)

| composé n° | $R_1$ | $R_3$ | $R_6$ | $R_7$ | $R_8$ | PF°C |
|---|---|---|---|---|---|---|
| 27 | $1\text{-n.}C_7H_{15}$ | H | $2,6\text{-iPr}_2C_6H_3$ | $-(CH_2)_4-$ | | 74-80 |
| 28 | $1\text{-n.}C_4H_9$ | H | $2,6\text{-iPr}_2C_6H_3$ | $-(CH_2)_4-$ | | 128-130 |
| 29 | 1-Me | H | $2,6\text{-iPr}_2C_6H_3$ | $n.C_4H_9$ | H | 143-145 |
| 30 | 1-Me | H | $2,6\text{-iPr}_2C_6H_3$ | $n.C_7H_{15}$ | H | 120-122 |
| 31 | 1-Me | H | $2,6\text{-iPr}_2C_6H_3$ | $(CH_3)_3CCH_2$ | H | 145-160 |
| 32 | 1-Me | H | $2,6\text{-iPr}_2C_6H_3$ | $C_6H_5CH_2$ | H | 158-160 |
| 33 | 1-Me | H | $2,6\text{-Cl}_2C_6H_3$ | $-(CH_2)_4-$ | | 199-201 |
| 34 | 1-Me | H | $2,6\text{-iPr}_2C_6H_3$ | $-(CH_2)_6-$ | | 182-184 |
| 35 | 1-Me | H | $2,6\text{-iPr}_2C_6H_3$ | $-(CH_2)_2O(CH_2)_2-$ | | 197-201 |
| 36 | 1-Me | H | $2,6\text{-Et}_2C_6H_3$ | $-(CH_2)_4-$ | | 171-173 |
| 37 | 1-DMAE | H | $2,6\text{-iPr}_2C_6H_3$ | $-(CH_2)_4-$ | | 132-136 |
| 38 | 1-(4F-Bn) | H | $2,6\text{-iPr}_2C_6H_3$ | $-(CH_2)_4-$ | | 68-74 |
| 39 | 1-Me | H | $2,4,6\text{-MeO}_3C_6H_2$ | $-(CH_2)_4-$ | | 176-178 |
| 40 | 1-Me | H | $4,6\text{-MeO}_2\text{pyrim-5}$ | $-(CH_2)_4-$ | | 220-222 |
| 41 | 1-Me | 5-MeO | $2,6\text{-iPr}_2C_6H_3$ | $-(CH_2)_4-$ | | 157-160 |
| 42 | 1-Me | H | $2,6\text{-iPr}_2C_6H_3$ | DMAE | H | 172-173 |
| 43 | 1-Me | H | $2,6\text{-iPr}_2C_6H_3$ | 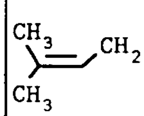 | H | 148-150 |
| 44 | 1-Me | H | $2,6\text{-iPrO}_2C_6H_3$ | $-(CH_2)_4-$ | | 145-147 |
| 45 | $1\text{-(pyr-3-CH}_2)$ | H | $2,6\text{-iPr}_2C_6H_3$ | $-(CH_2)_4-$ | | 123-124 |

**Exemple 7:**

En utilisant les procédés appropriés des exemples 1 à 5, on a préparé les composés consignés dans le tableau 2 ci-dessous de formule générale 1 dans laquelle, $R_1$ = 1-Me, $R_2 = R_3 = R_4$ = H, Z = $-(CH_2)_n-C(R_7R_8)-(CH_2)_p-$ en position-3, n = p = 0, $R_7$-$R_8$ = $-(CH_2)_4-$

Tableau n° 2

| composé n° | $R_5$ | $R_6$ | PF °C |
|---|---|---|---|
| 46 | $C_6H_5CH_2$ | 2.6-$Me_2C_6H_3$ | 185-187 |
| 47 | $C_6H_5CH_2$ | 2,6-$Cl_2C_6H_3$ | 200-202 |
| 48 | $C_6H_5CH_2$ | 2,4-$F_2C_6H_3$ | 134-136 |
| 49 | $CH_3$ | 2,6-$iPr_2C_6H_3$ | 186-188 |

**Exemple 8:**

En utilisant les procédés appropriés des exemples 1 à 5, on a préparé les composés (voir tableau 3 ci-dessous) de formule 1 dans laquelle, $R_1$ = 1-Me, $R_3 = R_4 = R_5$ = H, $R_6$ = diisopropyl-2,6-phényle, Z = $-(CH_2)_n-C(R_7R_8)-(CH_2)_p-$, n = p = 0, $R_7$-$R_8$ = $-(CH_2)_4-$.

Tableau n° 3

| composé n° | $R_2$ | Position de Z | PF °C |
|---|---|---|---|
| 50 | 2-Me | 3 | 211-213 |
| 51 | 2-$C_6H_5$ | 3 | 210-214 |
| 52 | 3-Me | 2 | 206-208 |

**Exemple 9:**

**(-)-N1-(Diisopropyl-2,6-phényl)-N2-[(méthyl-1-indolyl-3)-2- hexyl]urée** (composé n°53, formule 1:$R_1$ = 1-$CH_3$,$R_2 = R_3 = R_4$ = H,$R_6$ = 2,6-diisopropylphényle,Z = $-(CH_2)_n-C(R_7R_8)(CH_2)_p-$ en position-3,n = p = 0,$R_7$ = n.$C_4H_9$-,$R_8$ = H - énantiomère lévogyre).

Stade 1:

**(-)-Mandélate de (-)-(méthyl-1-indolyl-3)-2-hexylamine** ($C_{23}H_{30}N_2O_3$-PM = 382,50).
On dissout dans 600 $cm^3$ d'éther, 24g (0,104 mole) de (méthyl-1-indolyl-3)-2-hexylamine préparée dans les conditions de l'exemple 1 et 15,8g (0,104 mole) d'acide (R)-(-)-mandélique; après 3 heures d'agitation à 20 °C, on essore le solide formé et on le sèche (PF = 92-100 °C, quantité = 23,1g).
Les eaux mères sont conservées pour préparer l'énantiomère dextrogyre correspondant comme décrit à l'exemple 10.
On recristallise le solide obtenu ci-dessus successivement 2 fois dans la quantité suffisante d'acétate d'éthyle; on isole ainsi un solide blanc.
PF = 115-116 °C. Rendement = 15,1%
IR: $\nu CO_2^-$ = 1635 $cm^{-1}$.

Stade 2:

**(-)-(Méthyl-1-indolyl-3)-2-hexylamine** ($C_{15}H_{22}N_2$-PM = 230,35).
On alcalinise 6,7g (O,O175 mole) de composé du stade 1 par une solution aqueuse de soude 2N; on extrait le mélange par l'éther (2 x 25$cm^3$), on sèche l'extrait éthéré sur sulfate de sodium, on filtre puis on évapore le solvant; on isole ainsi une huile incolore.
Rendement = 96,8%.
L'excès énantiomérique (e.e.) est mesuré par analyse H.P.L.C. du produit de condensation avec le (S)-(-)-α-méthylbenzylisocyanate selon le procédé décrit au stade 4 de l'exemple 1: e.e. = 98%.
$[\alpha]_D^{20}$ = -9,7 ° (c = 3,5; $CH_2Cl_2$).
IR: $\nu NH_2$ = 3369 et 3297 $cm^{-1}$.
RMN ($CDCl_3$): 0,5-2,2(M,3H); 1-2(M,6H); 1,1(s,2H); 2,85(qs,3H); 3,6(s,3H); 6,7(s,1H); 6,8-7,6(M,4H).

Stade 3:

**(-)-N1-(Diisopropyl-2,6-phényl)-N2-[(méthyl-1-indolyl-3)-2-hexyl]urée** (composé n° 53).
Préparée à partir du composé du stade 2, par le procédé décrit au stade 4 de l'exemple 1 en remplaçant l'hexane par le diisopropyléther.
PF = 138-142 ° C (diisopropyléther-acétate d'éthyle:3-1). Rendement = 86,7%
e.e. = 98%.
$[\alpha]_D^{20}$ = -23 ° (c = 3; $CHCl_3$).
IR: $\nu$NH = 3383 et 3279 $cm^{-1}$.
RMN ($CDCl_3$): 0,5-2,2(M,21H); 2,4-3,5(M,5H); 3,55(s,3H); 4(large,1H); 5,7(s,1H); 6,4(s,1H); 6,6-7,6(M,7H).

| Analyse centésimale ($C_{28}H_{39}N_3O$-PM = 433,64): | | | |
|---|---|---|---|
| | C | H | N |
| %calculé<br>%trouvé | 77,55<br>77,86 | 9,06<br>9,22 | 9,69<br>9,67 |

**Exemple 10:**

**(+)-N1-(Diisopropyl-2,6-phényl)-N2-[(méthyl-1-indolyl-3)-2-hexyl]urée** (composé n° 54, formule 1:$R_1$ = 1-$CH_3$,$R_2$ = $R_3$ = $R_4$ = H,$R_6$ = 2,6-diisopropylphényle,Z = -$(CH_2)_n$-C($R_7R_8$)-$(CH_2)_p$- en position-3,n = p = 0,$R_7$ = n.$C_4H_9$-,$R_8$ = H - énantiomère dextrogyre).

Stade 1:

**(+)-Mandélate de (+)-(méthyl-1-indolyl-3)-2-hexylamine** ($C_{23}H_{30}N_2O_3$-PM = 382,50).
On alcalinise les eaux mères obtenues au stade 1 de l'exemple 9 selon le procédé décrit au stade 2 du même exemple; l'amine isolée selon ce procédé est ensuite salifiée par l'acide (S)-(+)-mandélique selon le procédé décrit au stade 1 de l'exemple 9; on isole ainsi un solide qu'on recristallise successivement dans le mélange diisopropyléther-acétate d'éthyle:7-3 puis 2 fois dans l'acétate d'éthyle additionné de la quantité suffisante d'éther pour obtenir la séparation attendue.
On isole ainsi un solide blanc.
PF = 112-114 ° C. Rendement = 29,5%.
IR: $\nu CO_2^-$1635$cm^{-1}$.

Stade 2:

**(+)-(méthyl-1-indolyl-3)-2-hexylamine** ($C_{15}H_{22}N_2$-PM = 230,35).
Préparé à partir du composé du stade 1 selon le procédé décrit au stade 2 de l'exemple 9.
On isole ainsi une huile incolore.
Rendement = 99,3%.
L'excès énantiomérique (e.e.) est mesuré par analyse H.P.L.C. du produit de condensation avec le (S)-(-)-α-méthylbenzylisocyanate obtenu par le procédé décrit au stade 3 de l'exemple 9: e.e = 99,6%.
$[\alpha]_D^{20}$ = +10,3 ° (c = 3,5; $CH_2Cl_2$).
IR: $\nu NH_2$ = 3373 et 3297 $cm^{-1}$.
RMN ($CDCl_3$): 0,6-1(M,3H); 1-2(M,6H); 1,4(s,2H); 2,9(qs,3H); 3,6(s,3H); 6,75(s,1H); 6,8-7,7(m,4H).

Stade 3:

**(+)-N1-(Diisopropyl-2,6-phényl)-N2-[(méthyl-1-indolyl-3)-2-hexyl]urée** (composé n° 54).
Préparé à partir du composé du stade 2 par le procédé décrit au stade 4 de l'exemple 1 en utilisant le diisopropyléther à la place de l'hexane.
On isole ainsi un solide blanc.
PF = 132-140 ° C (diisopropyléther-acétate d'éthyle:3-1).
e.e. = 99,6%.
$[\alpha]_D^{20}$ = +23,6 ° (c = 3; $CHCl_3$).

IR: $\nu$NH = 3383 et 3278cm$^{-1}$; $\nu$CO = 1644cm$^{-1}$.
RMN ($CDCl_3$): 0,5-2,2(M,21H); 2,4-3,5(M,5H); 3,55(s,3H); 4(large,1H); 5,7(s,1H); 6,4(s,1H); 6,6-7,6(M,7H).

| Analyse centésimale ($C_{28}H_{39}N_3O$-PM = 433,64): | | | |
|---|---|---|---|
| | C | H | N |
| %calculé | 77,55 | 9,06 | 9,69 |
| %trouvé | 77,53 | 9,10 | 9,82 |

**Exemple 11:**

Composés intermédiaires de formule générale **6** dans laquelle, Z = $-(CH_2)_n-C(R_7R_8)-(CH_2)_p-$ en position -3, n = p = 0, et $R_2 = R_4 = R_7 = R_8$ = H (tableau n° 4):

Tableau n° 4

| composé n° | $R_1$ | $R_3$ | PF° C PE/mm |
|---|---|---|---|
| 55 | 1-$CH_2C_6H_5$ | H | 89-90 |
| 56 | 1-Et | H | huile |
| 57 | 1-Me | 5-Me | huile |
| 58 | 1-iPr | H | 59-61 |
| 59 | 1-allyl | H | 145-150/0,2 |
| 60 | 1-$C_6H_5$ | H | huile |
| 61 | 1-n.$C_7H_{15}$ | H | huile |
| 62 | 1-n.$C_4H_9$ | H | huile |
| 63 | 1-DMAE | H | huile |
| 64 | 1-(4F-Bn) | H | 94-96 |
| 65 | 1-Me | 5-MeO | 104-106 |
| 66 | 1-(pyr-3-$CH_2$) | H | 88-90 |

**Exemple 12:**

Composés intermédiaires de formule générale **6** dans laquelle, Z = $-(CH_2)_n-C(R_7R_8)-(CH_2)_p-$ en position -3, n = p = 0, et $R_2 = R_4$ = H (tableau n° 5):

**Tableau n°5**

| composé n° | $R_1$ | $R_3$ | $R_7$ | $R_8$ | PF°C |
|---|---|---|---|---|---|
| 67 | 1-Me | H | iPr | H | 80-81 |
| 68 | 1-Me | H | Me | H | 61-64 |
| 69 | 1-$CH_2C_6H_5$ | H | $-(CH_2)_4-$ | | 144-146 |

Tableau n°5 (suite)

| composé n° | $R_1$ | $R_3$ | $R_7$ | $R_8$ | PF°C |
|---|---|---|---|---|---|
| 70 | 1-Et | H | $-(CH_2)_4-$ | | 76-78 |
| 71 | 1-Me | H | Et | H | huile |
| 72 | 1-Me | 5-Me | $-(CH_2)_4-$ | | amorphe |
| 73 | 1-Me | H | $-(CH_2)_5-$ | | 128-130 |
| 74 | 1-Me | H | Et | Et | 58-60 |
| 75 | 1-Me | H | Me | Me | 44-46 |
| 76 | 1-iPr | H | $-(CH_2)_4-$ | | 69-71 |
| 77 | 1-allyl | H | $-(CH_2)_4-$ | | 58-60 |
| 78 | 1-Me | H | allyl | allyl | 76-79 |
| 79 | $1-C_6H_5$ | H | $-(CH_2)_4-$ | | huile |
| 80 | 1-Me | H | $-(CH_2)_3-$ | | 98-100 |
| 81 | 1-Me | H | $n.C_4H_9$ | $n.C_4H_9$ | 73-75 |
| 82 | 1-Me | H | allyl | H | 60-62 |
| 83 | $1-n.C_7H_{15}$ | H | $-(CH_2)_4-$ | | huile |
| 84 | $1-n.C_4H_9$ | H | $-(CH_2)_4-$ | | huile |
| 85 | 1-Me | H | $n.C_4H_9$ | H | 63-66 |
| 86 | 1-Me | H | $n.C_7H_{15}$ | H | 43-45 |
| 87 | 1-Me | H | $-(CH_2)_6-$ | | 99-101 |
| 88 | 1-Me | H | $-(CH_2)_2O(CH_2)_2-$ | | 178-180 |
| 89 | 1-DMAE | H | $-(CH_2)_4-$ | | 56-58 |
| 90 | 1-(4F-Bn) | H | $-(CH_2)_4-$ | | 79-81 |
| 91 | 1-Me | 5-MeO | $-(CH_2)_4-$ | | 100-101 |
| 92 | 1-Me | H | DMAE | H | huile |

**Tableau n°5 (suite et fin)**

| composé n° | $R_1$ | $R_3$ | $R_7$ | $R_8$ | PF°C |
|---|---|---|---|---|---|
| 93 | 1-Me | H | 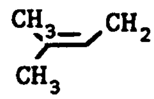 | H | huile |
| 94 | 1-(pyr-3-$CH_2$) | H | $-(CH_2)_4-$ | | 130-132 |

**Exemple 13:**

Composés intermédiaires de formule générale **6** dans laquelle, $Z = -(CH_2)_n-C(R_7R_8)-(CH_2)_p-$, $n = p = 0$, $R_1$ = 1-Me, $R_3 = R_4$ = H et $R_7$-$R_8$ = $-(CH_2)_4-$ (tableau n° 6):

Tableau n° 6

| composé n° | $R_2$ | position de Z | PF° C |
|---|---|---|---|
| 95 | 2-Me | 3 | amorphe |
| 96 | 2-$C_6H_5$ | 3 | 150-152 |
| 97 | 3-Me | 2 | 135-137 |

**Exemple 14:**

Composés intermédiaires de formule générale 5 dans laquelle, $Z = -(CH_2)_n-C(R_7R_8)-(CH_2)_p-$, $n = p = 0$, $R_1$ = 1-Me, $R_3 = R_4$ = H et $R_7$-$R_8$ = $-(CH_2)_4-$ (tableau n° 7):

Tableau n° 7

| composé n° | $R_2$ | position de Z | PF° C | $\nu NH_2 (cm^{-1})$ |
|---|---|---|---|---|
| 98 | 2-Me | 3 | amorphe | 3370 |
| 99 | 2-$C_6H_5$ | 3 | 150-152 | 3380 |
| 100 | 3-Me | 2 | 135-137 | 3377 |

**Exemple 15:**

Composés intermédiaires de formule générale **5** dans laquelle, $Z = -(CH_2)_n-C(R_7R_8)-(CH_2)_p-$ en position -3, $n = p = 0$ et $R_2 = R_4$ = H (tableau n° 8):

Tableau n° 8

| composé n° | $R_1$ | $R_3$ | $R_7$ | $R_8$ | PF °C | $\nu NH_2 (cm^{-1})$ |
|---|---|---|---|---|---|---|
| 101 | 1-Me | H | iPr | H | huile | 3380,3300 |
| 102 | 1-Me | H | Me | H | huile | 3380 |
| 103 | $1\text{-}CH_2C_6H_5$ | H | $-(CH_2)_4-$ | | huile | 3360 |
| 104 | 1-Et | H | $-(CH_2)_4-$ | | huile | 3374 |
| 105 | 1-Me | H | Et | H | huile | 3380 |
| 106 | 1-Me | 5-Me | $-(CH_2)_4-$ | | huile | 3370 |
| 107 | 1-Me | H | $-(CH_2)_5-$ | | huile | 3376 |
| 108 | 1-Me | H | Et | Et | huile | 3286 |
| 109 | 1-Me | H | Me | Me | huile | 3380,3300 |
| 110 | 1-iPr | H | $-(CH_2)_4-$ | | huile | 3377 |
| 111 | 1-allyl | H | $-(CH_2)_4-$ | | huile | 3360 |
| 112 | 1-Me | H | allyl | allyl | huile | 3390 |
| 113 | $1\text{-}C_6H_5$ | H | $-(CH_2)_4-$ | | huile | 3360 |
| 114 | 1-Me | H | $-(CH_2)_3-$ | | huile | 3380 |

| | |
|---|---|
| 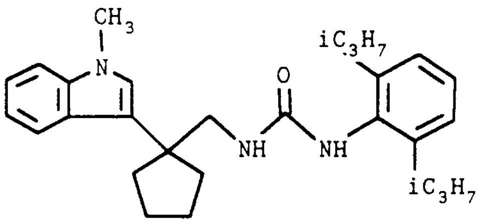<br>composé n°1 | 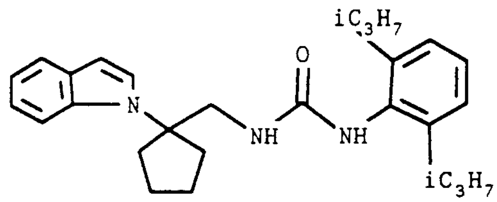<br>composé n°2 |
| 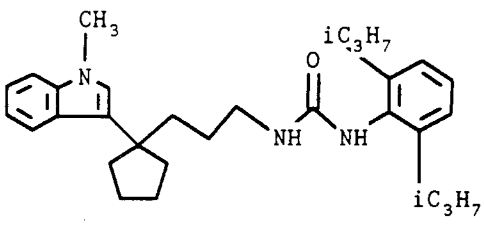<br>composé n°3 | 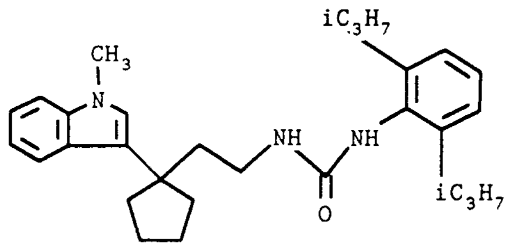<br>composé n°4 |
| 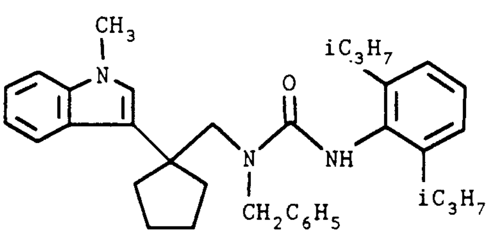<br>composé n°5 | 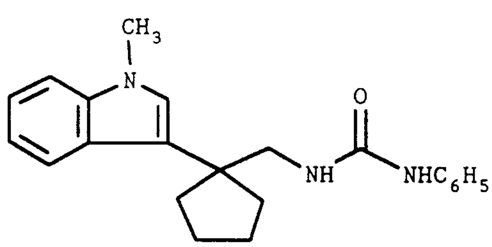<br>composé n°6 |
| 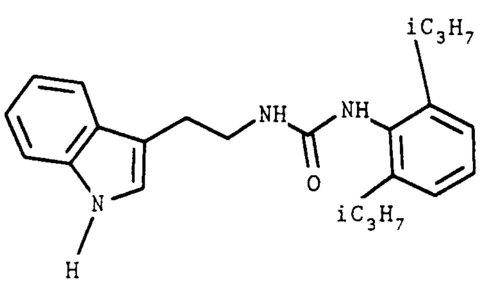<br>composé n°7 | 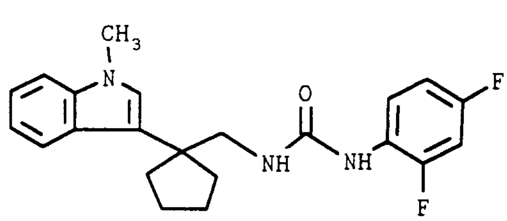<br>composé n°8 |

| | |
|---|---|
| 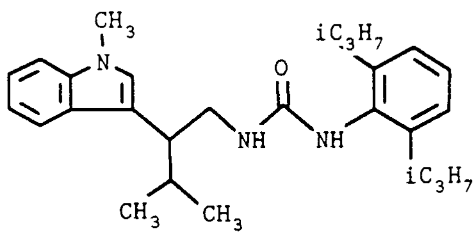 composé n°9 | 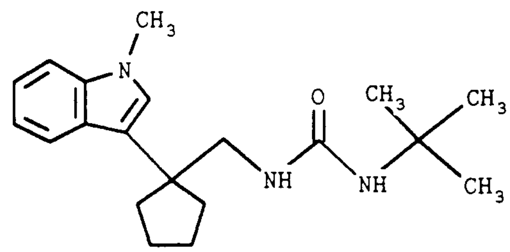 composé n°10 |
| 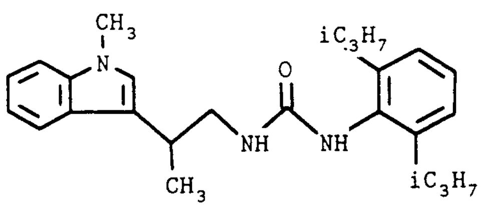 composé n°11 | 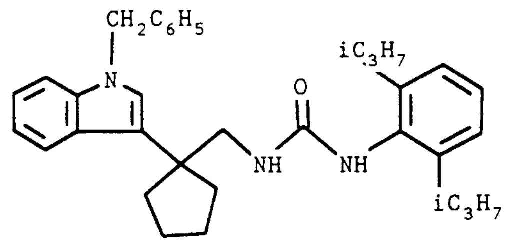 composé n°12 |
| 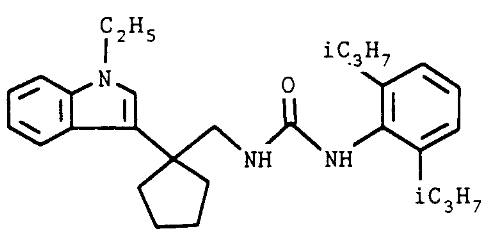 composé n°13 | 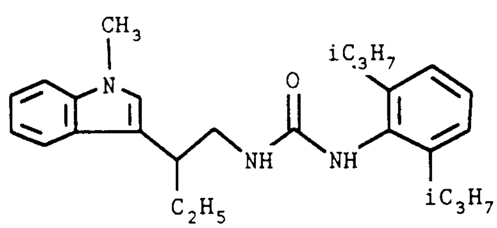 composé n°14 |
| 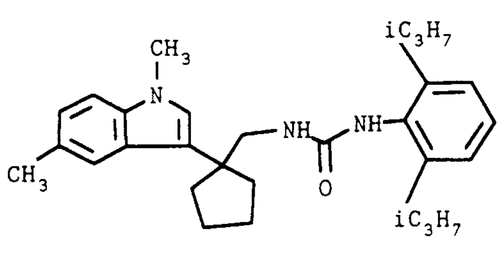 composé n°15 | 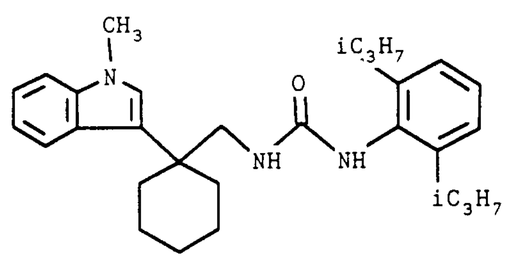 composé n°16 |

| | |
|---|---|
| 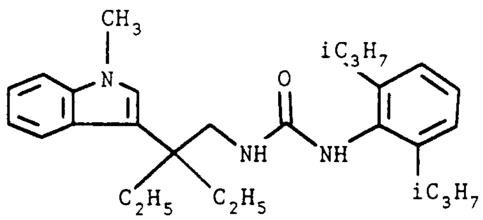<br>composé n°17 | 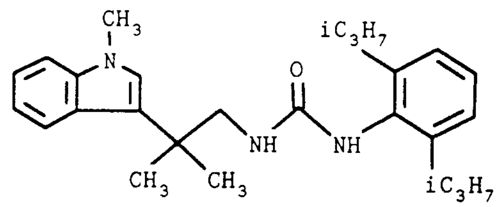<br>composé n°18 |
| 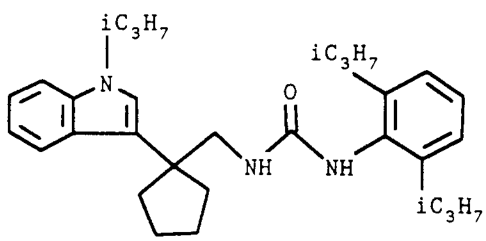<br>composé n°19 | 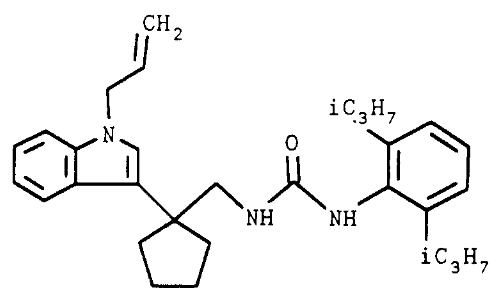<br>composé n°20 |
| 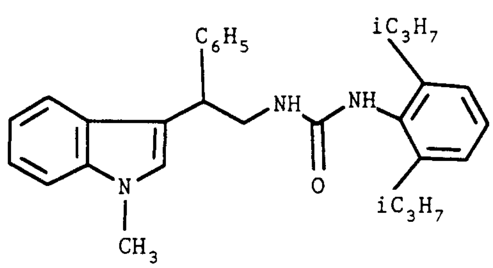<br>composé n°21 | 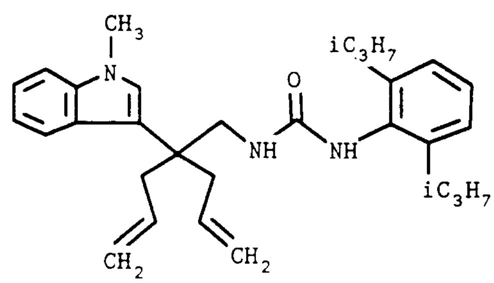<br>composé n°22 |
| 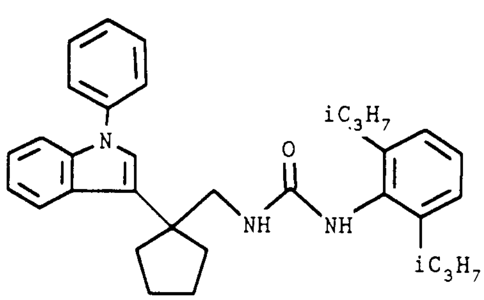<br>composé n°23 | 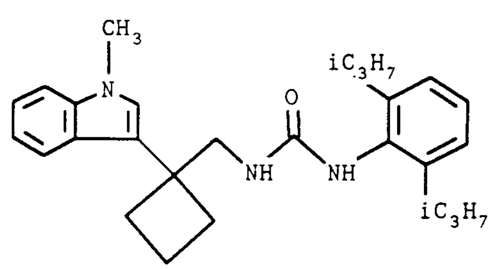<br>composé n°24 |

| | |
|---|---|
| 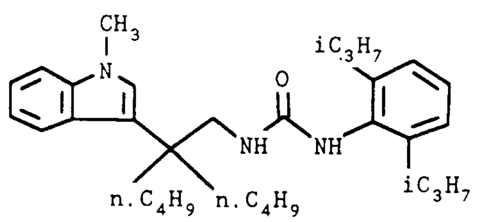<br>composé n°25 | 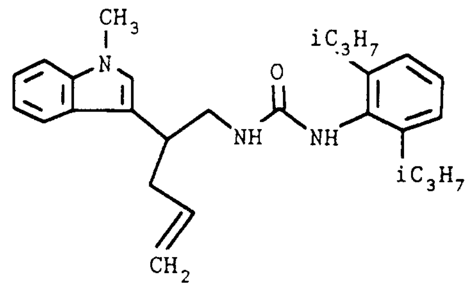<br>composé n°26 |
| 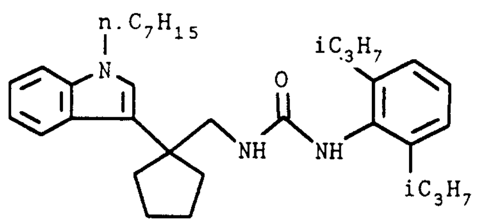<br>composé n°27 | 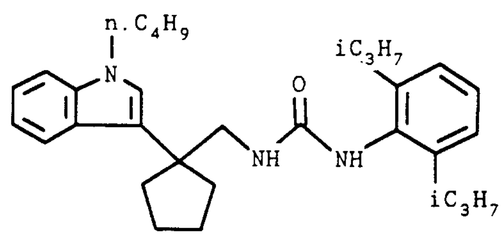<br>composé n°28 |
| 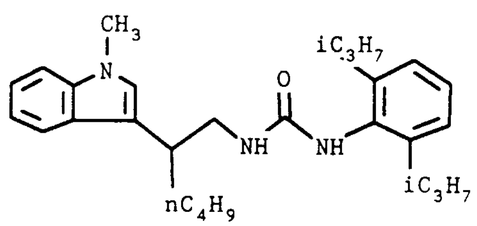<br>composé n°29 | 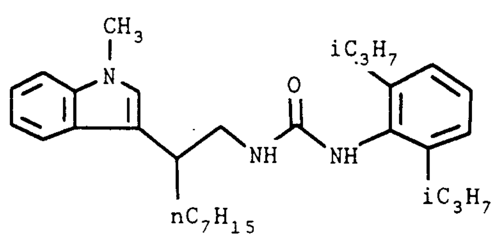<br>composé n°30 |
| 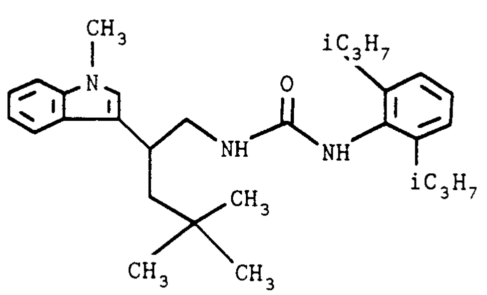<br>composé n°31 | 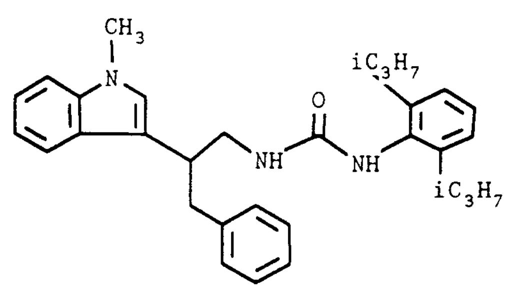<br>composé n°32 |

| | |
|---|---|
| 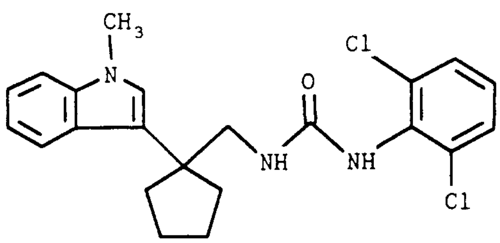<br>composé n°33 | 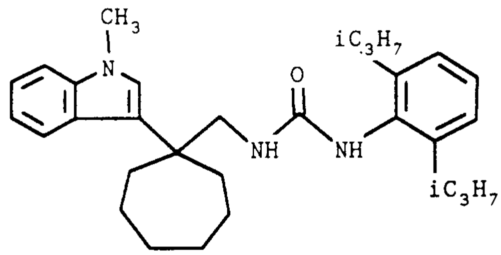<br>composé n°34 |
| 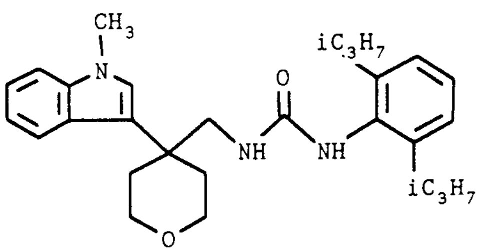<br>composé n°35 | 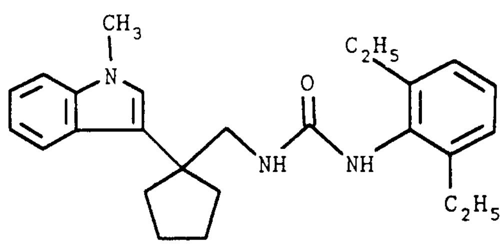<br>composé n°36 |
| 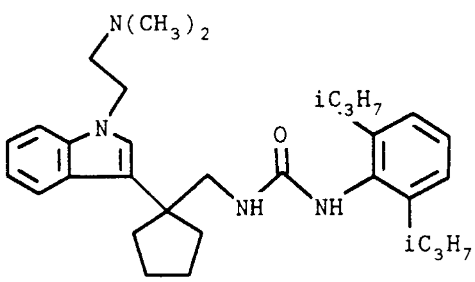<br>composé n°37 | 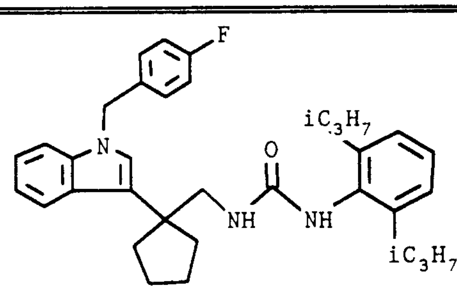<br>composé n°38 |
| 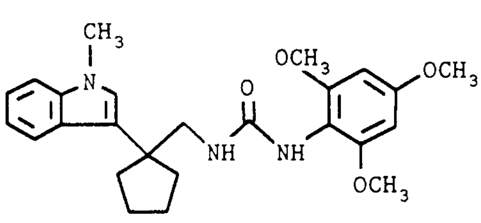<br>composé n°39 | 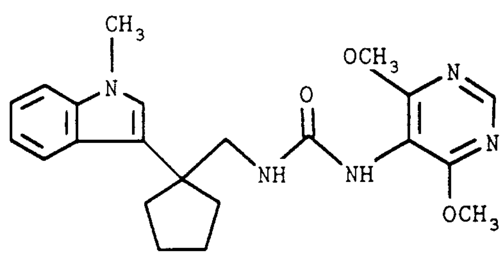<br>composé n°40 |

| | |
|---|---|
| 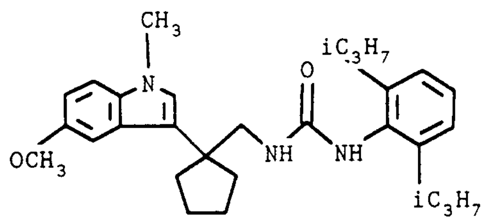 composé n°41 | 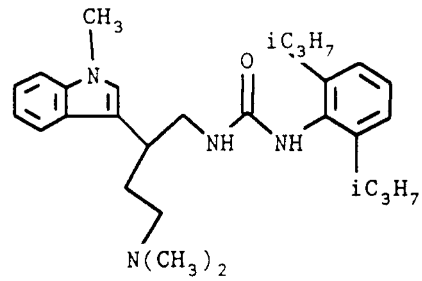 composé n°42 |
| 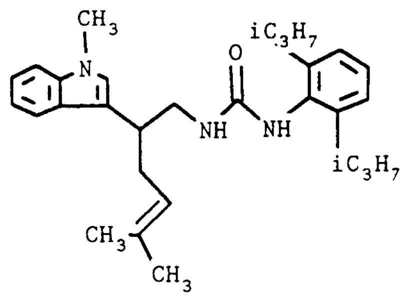 composé n°43 | 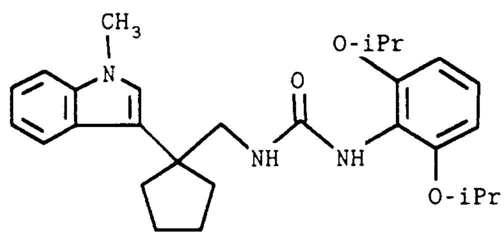 composé n°44 |
| 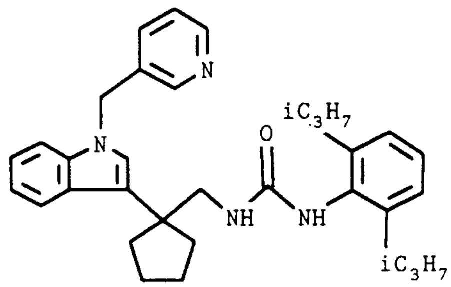 composé n°45 | 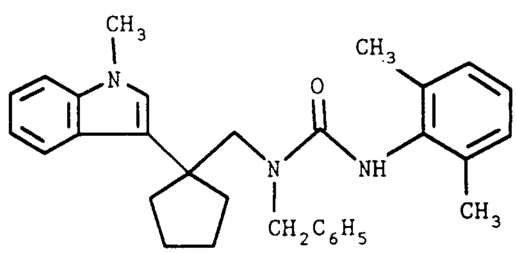 composé n°46 |
| 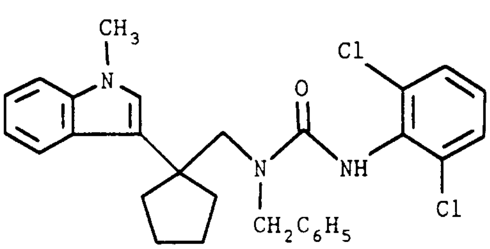 composé n°47 | 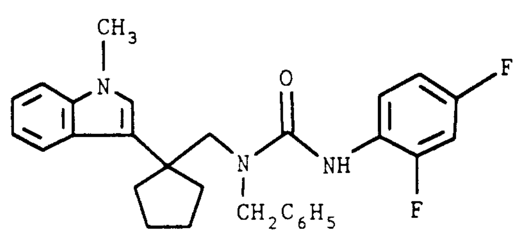 composé n°48 |

| | |
|---|---|
| 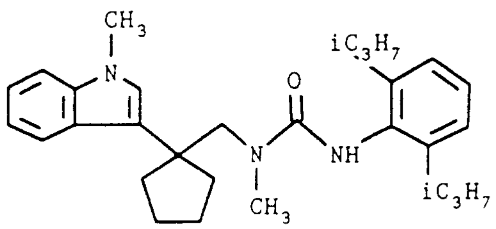 composé n°49 | 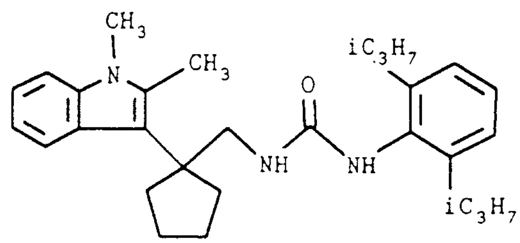 composé n°50 |
| 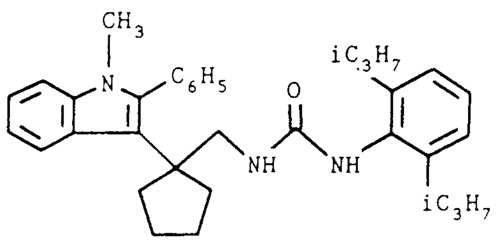 composé n°51 | 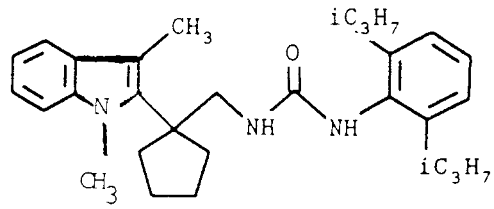 composé n°52 |
| 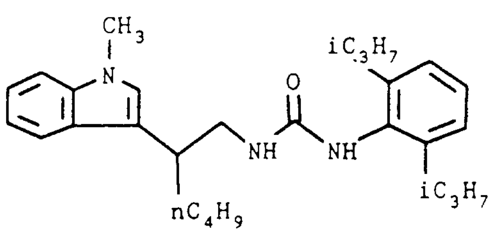 isomère (-) composé n°53 | 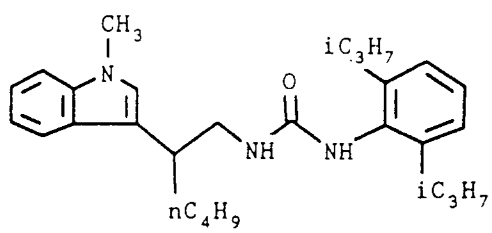 isomère (+) composé n°54 |

**Revendications**

**Revendications pour les Etats contractants suivants : AT, BE, CH, LI, DE, DK, FR, GB, GR, IT, LU, NL, PT, SE**

**1.** Nouveaux dérives de l'indole de formule générale 1 suivante

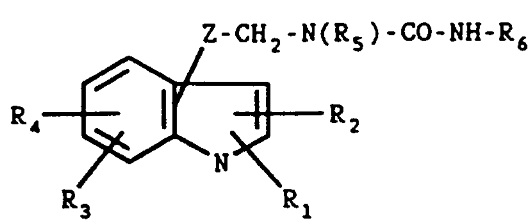

1

dans laquelle :

$R_1$ et $R_2$ qui peuvent être situés en position -1, -2 ou -3 du cycle de l'indole, représentent indépendamment un atome d'hydrogène, un radical alkyle linéaire de 1 à 12 atomes de carbone ou ramifié de 3 à 5 atomes de carbone, des radicaux alcényle en $C_3$-$C_6$, cycloalkyle en $C_3$-$C_8$, N-(alkyl en $C_1$-$C_5$) amino(alkyle en $C_1$-$C_5$) ou N,N-(dialkyl en $C_1$-$C_5$) amino(alkyle en $C_1$-$C_5$) ou l'un des substituants $R_1$ ou $R_2$ représente un radical pyridyl-2 (-3 ou -4) méthyle et l'autre un atome d'hydrogène, étant entendu que lorsque l'atome d'azote du cycle indolique n'est substitué par aucun des groupes $R_1$, $R_2$ ou -Z-$CH_2$-N($R_5$)CONH$R_6$, il est substitué par un atome d'hydrogène,

$R_3$ et $R_4$ qui peuvent être situés en position -4, -5, -6 ou -7 du cycle de l'indole, représentent indépendamment des atomes d'hydrogène ou d'halogène ou des radicaux alkyle en $C_1$-$C_5$, alkoxy en $C_1$-$C_5$ ou alkylthio en $C_1$-$C_5$,

ou l'un des substituants $R_3$ ou $R_4$ désigne un atome d'hydrogène et l'autre des radicaux trifluorométhyle, nitro, N-(alkyl en $C_1$-$C_5$) amino ou N,N-(dialkyl en $C_1$-$C_5$) amino,

ou trois des substituants $R_1$, $R_2$, $R_3$ ou $R_4$ ont les significations qui viennent d'être définies et le quatrième représente un radical de formule 2 suivante:

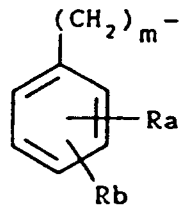

2

dans laquelle m peut prendre les valeurs 0, 1 ou 2 et les substituants Ra et Rb désignent indépendamment des atomes d'hydrogène ou d'halogène ou des radicaux alkyle en $C_1$-$C_5$,alkoxy en $C_1$-$C_5$ ou alkylthio en $C_1$-$C_5$,

$R_5$ désigne un atome d'hydrogène, un radical alkyle linéaire de 1 à 12 atomes de carbone ou ramifié de 3 à 5 atomes de carbone, un radical cycloalkyle en $C_3$-$C_8$ ou un radical de formule 2, dans laquelle m a la valeur 1 et Ra et Rb ont les significations définies précédemment, $R_6$ désigne un radical alkyle en $C_1$-$C_5$ ou un radical de formule 3 suivante,

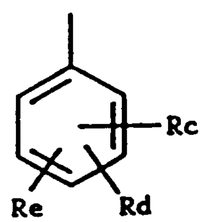

3

dans laquelle Rc, Rd et Re désignent indépendamment des atomes d'hydrogène ou d'halogène ou des radicaux alkyle en $C_1$-$C_5$, alkoxy en $C_1$-$C_5$ ou alkylthio en $C_1$-$C_5$, ou deux des substituants Rc, Rd et Re peuvent avoir les significations qui viennent d'être définies et le troisième représente un radical trifluorométhyle,

$R_6$ peut aussi désigner les radicaux naphtyl-1 ou -2 ou un radical hétérocyclyle à 5 ou 6 chaînons et à un ou deux hétéroatomes éventuellement fusionné avec un cycle benzénique et le cas échéant substitués par un à trois atomes d'halogène ou radicaux alkyle en $C_1$-$C_5$ ou alcoxy en $C_1$-$C_5$, Z, qui peut être relié aux sommets -1, -2, -3, -4, -5, - 6 ou -7 du cycle de l'indole, désigne les biradicaux de formules : -CH=CH-C($R_7R_8$)- ou -$(CH_2)_n$C($R_7R_8$)-$(CH_2)_p$- dans lesquelles, n et p désignent deux nombres entiers pouvant prendre les valeurs 0, 1 et 2 à condition que leur somme (n + p) soit inférieure ou égale à 2,

$R_7$ et $R_8$ représentent indépendamment un atome d'hydrogène, un radical alkyle linéaire de 1 à 12 atomes de carbone ou ramifié de 3 à 5 atomes de carbone, des radicaux alcényle en $C_3$-$C_6$, cycloalkyle en $C_3$-$C_8$, N-(alkyl en $C_1$-$C_5$) amino, N,N-(dialkyl en $C_1$-$C_5$) amino, N-(alkyl en $C_1$-$C_5$) amino

(alkyle en $C_1$-$C_5$) ou N,N-(dialkyl en $C_1$-$C_5$) amino (alkyle en $C_1$-$C_5$) ou un groupe de formule 2, dans laquelle m peut prendre les valeurs 0 ou 1 et Ra et Rb ont les significations définies précédemment,
$R_7$ et $R_8$ peuvent aussi former ensemble une chaîne polyméthylène : $-(CH_2)_q-$ dans laquelle q peut prendre les valeurs 3 à 8 et susceptible le cas échéant, lorsque q est supérieur ou égal à 5, de contenir une double liaison,
$R_7$ et $R_8$ peuvent aussi former ensemble les chaînes:-
$-CH_2-O-(CH_2)_2-$, $-(CH_2)_2-O-(CH_2)_2-$, $-CH_2-S-(CH_2)_2-$, $-(CH_2)_2-S-(CH_2)_2-$, $-CH_2-N(R_9)-(CH_2)_2-$ ou $-(CH_2)_2-N(R_9)-(CH_2)_2-$,
$R_5$, $R_7$ et $R_8$ peuvent aussi, lorsqu'ils représentent un radical cycloalkyle en $C_3$-$C_8$, contenir une double liaison,
et $R_9$ représente un radical alkyle en $C_1$-$C_5$.

2. Composés selon la revendication 1, caractérisés en ce que les substituants $R_3$ et $R_4$ représentent des atomes d'hydrogène ou d'halogène ou des radicaux alkyle en $C_1$-$C_5$, N-(alkyl en $C_1$-$C_5$) amino, N,N-(dialkyl en $C_1$-$C_5$) amino ou alkoxy en $C_1$-$C_5$, $R_5$ représente un atome d'hydrogène, un radical alkyle linéaire en $C_1$-$C_{12}$ ou un groupe de formule 2 suivante,

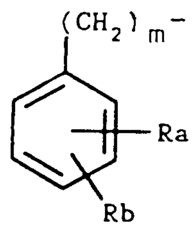

2

dans laquelle,
m = 1 et les substituants Ra et Rb designent indépendamment des atomes d'hydrogène ou d'halogène ou des radicaux alkyle en $C_1$-$C_5$, alkoxy en $C_1$-$C_5$ ou alkylthio en $C_1$-$C_5$ et $R_6$ désigne un groupe de formule 3,

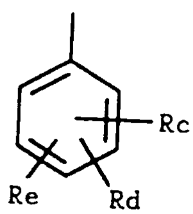

3

dans laquelle Rc, Rd et Re désignent indépendamment des atomes d'hydrogène ou d'halogène ou des radicaux alkyle en $C_1$-$C_5$, alkoxy en $C_1$-$C_5$ ou alkylthio en $C_1$-$C_5$ ou deux des substituants Rc, Rd et Re peuvent avoir les significations qui viennent d'être définies et le troisième représente un radical trifluorométhyle.

3. Composés selon la revendication 1, choisis parmi les composés suivants :
N1-(Diisopropyl-2,6-phényl)-N2-[(méthyl-1-indolyl-3)-1-cyclopentylméthyl]urée, N1-[(Indolyl-1)-1-cyclopentylméthyl]-N2-(diisopropyl-2,6-phényl)urée, N1-(Diisopropyl-2,6-phényl)-N2-[[(méthyl-1-indolyl-3)-1-cyclopentyl]-3-propyl]urée,N1-(Diisopropyl-2,6-phényl)-N2-[[(méthyl-1-indolyl-3)-1-cyclopentyl]-2-éthyl]-urée,N1-Benzyl-N2-(diisopropyl-2,6-phényl)-N1-[(méthyl-1-indolyl-3)-1-cyclopentylméthyl]urée,-N2 -[-(méthyl-1-indolyl-3)-1-cyclopentylméthyl]-N1-phényl-urée, N1-[(Indolyl-3)-2-éthyl]-N2-(diisopropyl-2,6-phényl)urée, N1-(Difluoro-2,4-phényl)-N2-[(méthyl-1-indolyl-3)-1-cyclopentylméthyl]urée, N1-(Diisopropyl-2,6-phényl)-N2-[méthyl-3-(méthyl-1-indolyl-3)-2-butyl]urée, N1-*tert*.Butyl-N2-[(méthyl-1-indolyl-3)-1-cyclopentylméthyl]urée, N1-(Diisopropyl-2,6-phényl)-N2-[(méthyl-1-indolyl-3)-2-propyl]urée,N1-[(Benzyl-1-indolyl-3)-1-cyclopentylméthyl]-N2-(diisopropyl-2,6-phényl)urée, N1-[(Ethyl-1-indolyl-3)-1-cyclopentylméthyl]-N2-(diisopropyl-2,6-phényl)urée, N1-(Diisopropyl-2,6-phényl)-N2-[(méthyl-1-indolyl-3)-2-butyl]-urée, N1-(Diisopropyl-2,6-phényl)-N2-[(diméthyl-1,5-indolyl-3)-1-cyclopentylméthyl]urée, N1-(Diisopropyl-2,6-phényl)-N2-[(méthyl-1-indolyl-3)-1-cyclohexylméthyl]urée,N1-[Ethyl-2-(méthyl-1-indolyl-3)-2-butyl]-N2-(diisopropyl-2,6-phényl)urée,N1-(Diisopropyl-2,6-phényl)-N2-[méthyl-2-(méthyl-1-indolyl-3)-2-propyl]urée, N1-[(Isopropyl-1-indolyl-3)-1-cyclopentylméthyl]-N2-(diisopropyl-2,6-phényl)urée,N1-[(Allyl-1-indolyl-3)-1-cyclopentylméthyl]-N2-(diisopropyl-2,6-phényl)urée,N1-(Diisopropyl-2,6-phényl)-N2-[-(méthyl-1-indolyl-3)-2-phényl-2-éthyl]urée, N1-[Allyl-2-(méthyl-1-indolyl-3)-2-pentén-4-yl]-N2 -(Diisopropyl-2,6-phényl)urée, N1-(Diisopropyl-2,6-phényl)-N2-[(phényl-1-indolyl-3)-1-cyclopentylméthyl]urée,N1-(Diisopropyl-2,6-phényl)-N2-[(méthyl-1-indolyl-3)-1-cyclobutylméthyl]urée,N1-[Butyl-2-(méthyl-1-indolyl-3)-2-hexyl]-N2-(diisopropyl-2,6-phényl)urée, N1-(Diisopropyl-2,6-phényl)-N2-[(méthyl-1-indolyl-3)-2-pentèn-4-yl]urée, N1-[(Heptyl-1-indolyl-3)-1-cyclopentylméthyl]-N2-(diisopropyl-2,6-phényl)urée, N1-[(Butyl-1-indolyl-3)-1-cyclopentylméthyl)]-N2-(diisopropyl-2,6-phényl)urée,N1-(Diisopropyl-2,6-phényl)-N2-[-(méthyl-1-indolyl-3)-2-hexyl]urée,N1-(Diisopropyl-2,6-phényl)-N2-[( méthyl-1-indolyl-3)-2-nonyl]urée, N1-(Diisopropyl-2,6-phényl)-N2-[(diméthyl-4,4-(méthyl-1-indolyl-3)-2-pentyl)urée, N1-(Diisopropyl-2,6-phényl)-N2-[(méthyl-1-indolyl-3)-2-phényl-3-propyl]urée, N1-(Dichloro-2,6-phényl)-N2-[(méthyl-1-indolyl-3)-1-cyclopentylméthyl]urée, N1-(Diisopropyl-2,6-phényl)-N2-[(méthyl-1-indolyl-3)-1-cycloheptylméthyl]-urée, N1-(Diisopropyl-2,6-phényl)-N2-((méthyl-1-indolyl-3)-4-tétrahydropyranyl-4-méthyl]urée,N1-(Diéthyl-2,6-phényl)-N2-[(méthyl-1-indolyl-3)-1-cyclopentylméthyl]urée, N1-(Diisopropyl-2,6-phényl)-N2-[[-(diméthylamino-2-éthyl)-1-indolyl-3]-1-cyclopentylméthyl]urée, N1-[[(Fluoro-4-benzyl)-1-indolyl-3]-1-cyclopentylméthyl]-N2-(diisopropyl-2,6-phényl)urée, N1-(Triméthoxy-2,4,6-phényl)-N2-[(méthyl-1-indolyl-3)-1-cyclopentylméthyl]urée,N1-(Diméthoxy-4,6-pyrimidinyl-5)-N2-[(méthyl-1-indolyl-3)-1-cyclopentylméthyl]urée,N1-(Diisopropyl-2,6-phényl)-N2-[(méthoxy-5-méthyl-1-indolyl-3)-1-cyclopentylméthyl]urée, N1-(Diisopropyl-2,6-phényl)-N2-[diméthylamino-4-(méthyl-1-indolyl-3)-2-butyl]urée, N1-(Diisopropyl-2,6-phényl)-N2-[méthyl-5-(méthyl-1-indolyl-3)-2-hexèn-4-yl]urée,N1-(Diisopropropoxy-2,6-phényl)-N2-[(méthyl-1-indolyl-3)-1-cyclopentylméthyl]urée,N1-(Diisopr opyl-2,6-phényl)-N2-[[(pyridyl-3-méthyl)-1-indolyl-3]-1-cyclopentylméthyl]urée, N1-Benzyl-N1-((méthyl-1-indolyl-3)-1-cyclopentylméthyl]-N2-(diméthyl-2,6-phényl)urée,N1-Benzyl-N2-(dichloro-2,6-phényl)-N1-[(méthyl-1-indolyl-3)-1-cyclopentylméthyl)urée,N1-Ben zyl-N2-(difluoro-2,4-phényl)-N1-[(méthyl-1-indolyl-3)-1-cyclopentylméthyl]urée,N2-(Diisopropyl-2,6-phényl)-N1-méthyl-N1-[(méthyl-1-indolyl-3)-1-cyclopentylméthyl]urée, N1-(Diisopropyl-2,6-phényl)-N2-[-(diméthyl-1,2-indolyl-3)-1-cyclopentylméthyl]urée-N1-(Diisopropyl-2,6-phényl)-N2-[(méthyl-1-phényl-2-indolyl-3)-1-cyclopentylméthyl]urée,N1-(Diisopropyl-2,6-phényl)-N2-[(diméthyl-1,3-indolyl-2)-1-cyclopentylméthyl]urée,(-)-N 1-(Diisopropyl-2,6-phényl)-N2-[(méthyl-1-indolyl-3)-2-hexyl]urée,( + )-N1-(Diisopropyl-2,6-phényl)-N2-[(méthyl-1-indolyl-3)-2-hexyl]urée.

4. Procédé de préparation des composés de formule 1 tels que définis à la revendication 1, comprenant la réaction d'un composé de formule générale 4

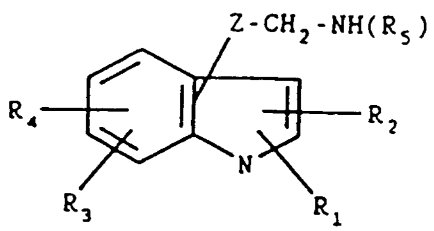

4

avec le phosgène et une amine $R_6NH_2$, ou avec un isocyanate $R_6NCO$ ou bien avec un trichloroacétamide $CCl_3$-CO-$NHR_6$.

5. Procédé selon la revendication 4, comprenant la réaction d'un composé de formule générale 5

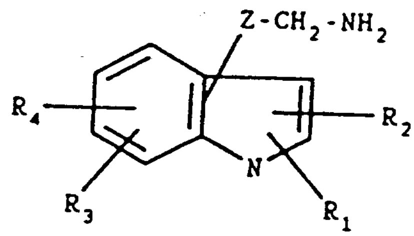

5

avec un composé halogéné $R_5$-X ou bien avec un aldéhyde $R_{10}$-CHO ou un chlorure d' acide $R_{10}$-COCl ou un anhydride d'acide $(R_{10}CO)_2O$ où $R_{10}$ représente un radical alkyle contenant éventuellement une double liaison ou un radical de formule 2 tel que défini dans la revendication 1 dans laquelle m est égal à O et $R_a$ et $R_b$ ont les significations définies à là revendication 1 suivie de la réduction de l'imine ou de l'amide formé, pour obtenir un composé de formule 4.

6. Procédé selon la revendication 5, comprenant la réduction d'un nitrile de formule 6,

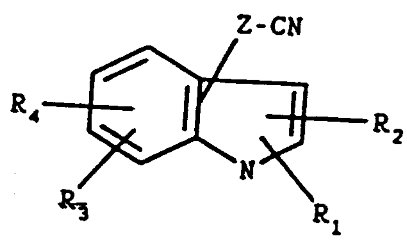

6

pour obtenir un composé de formule 5.

7. Composés intermédiaires pour la préparation des composés de formule générale I, choisi parmi:
   . (méthyl-1-indolyl-3)-1-cyclopentanecarbonitrile
   . (méthyl-1-indolyl-3)-1-cyclopentylméthylamine
   . (indolyl-1)-1-cyclopentanecarbonitrile
   . (indolyl-1)-1-cyclopentylméthylamine
   . [(méthyl-1-indolyl-3)-1-cyclopentyl]-3-propylamine
   . (méthyl-1-indolyl-3)-1-cyclopentaneacétonitrile
   . [(méthyl-1-indolyl-3)-1-cyclopentyl]-2-éthylamine
   . [(méthyl-1-indolyl-3)-1-cyclopentyl]-3-propène-2-nitrile
   . N-benzyl-N[(méthyl-1-indolyl-3)-1-cyclopentylméthyl] amine
   . (-)-(méthyl-1-indolyl-3)-2-hexylamine
   . ( + )-(méthyl-1-indolyl-3)-2-hexylamine.

8. Procédé de préparation des composés de formule générale 6 pour lesquels Z représente un biradical de formule $-(CH_2)_n-C(R_7R_8)-(CH_2)_p-$dans laquelle n = p = 0 et $R_7$ et $R_8$ sont tels que définis dans la revendication 1, ledit procédé étant caractérisé en ce que l'on effectue les étapes a) ou b) suivantes :

a) on alkyle par l'iodure de méthyle un N,N-diméthylaminométhyl-indole de formule générale 7 :

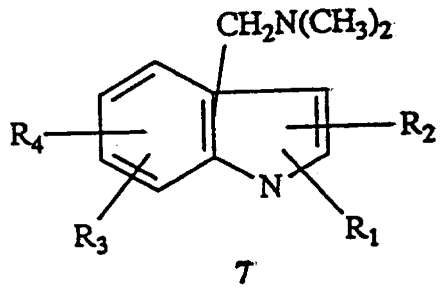

7

où $R_1$, $R_2$, $R_3$ et $R_4$ sont tels que définis dans la revendication 1, le produit obtenu est alors traité par KCN ou NaCN,

b) on N-alkyle par le chloroacétonitrile un composé de formule générale 8 :

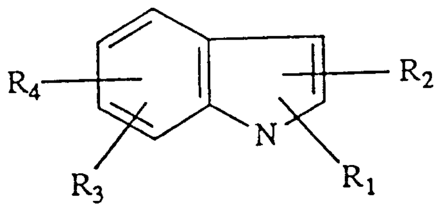

8

où $R_1$, $R_2$, $R_3$ et $R_4$ ont les significations indiquées ci-dessus, puis, le cas échéant, on effectue une mono ou di C-alkylation des composés obtenus dans les étapes a) ou b) avec un halogènure.

**9.** Procédé de préparation des composés de formule générale 6 pour lesquels Z représente un biradical de formule $-CH=CH-C(R_7R_8)$ ou $-C(R_7R_8)-CH=CH-$, dans lesquelles $R_7$ et $R_8$ sont tels que définis dans la revendication 1, ledit procédé étant caractérisé en ce que l'on fait réagir respectivement, un composé de formule 11 :

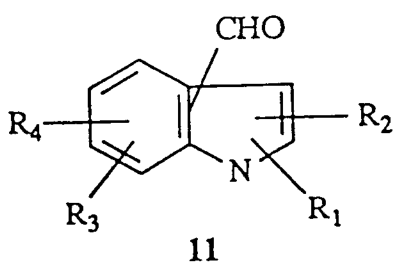

11

ou un composé de formule 14 :

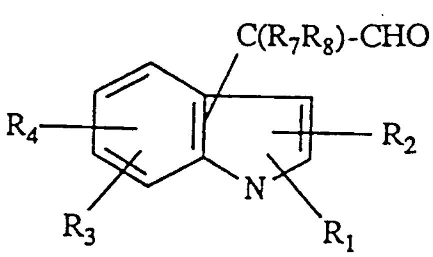

14

dans lesquelles $R_1$, $R_2$, $R_3$ et $R_4$ sont tels que définis dans la revendication 1, avec un phosphonate ou un cyanoalkylénetriphénylphosphorane.

**10.** Procédé de préparation des composés de formule générale 6 pour lesquels Z représente un biradical de formule $-(CH_2)_n-C(R_7R_8)-(CH_2)_p-$dans laquelle $R_7$ et $R_8$ sont tels que définis dans la revendication 1, et soit n = 0 et p = 1, soit p = 0 et n = 1, ledit procédé étant caractérisé en ce qu'on réduit par le borohydrure un composé de formule 11 ou de formule 14, tels que définis dans la revendication 9, on transforme le produit ainsi obtenu en son chlorure correspondant par action du chlorure de thionyle, puis on fait réagir ledit chlorure avec un nitrile en présence d'une base.

**11.** Compositions pharmaceutiques caractérisées en ce qu'elles comportent une quantité efficace d'au moins un composé de formule 1 tel que défini à la revendication 1 en mélange avec un excipient pharmaceutiquement acceptable.

**12.** Médicaments, notamment à activités hypolipidémiante, anti-athéroscléreuse et anti-diabétique, selon la revendication 11, sous forme d'unité de dosage dans laquelle chaque unité de dosage contient de 10 à 500 mg de principe actif en mélange avec un excipient pharmaceutiquement acceptable.

**13.** Utilisation d'un composé selon la revendication 1 pour la préparation d'un médicament destiné à traiter les hyperglycémies.

**14.** Utilisation d'un composé selon la revendication 1 pour la préparation d'un médicament destiné au traitement des hyperlipidémies.

**15.** Utilisation d'un composé selon la revendication 1 Pour la préparation d'un médicament destiné au traitement de l'athérosclérose.

**Revendications pour l'Etat contractant suivant : ES**

**1.** Procédé de préparation de nouveaux dérivés de l'indole de formule générale 1 suivante

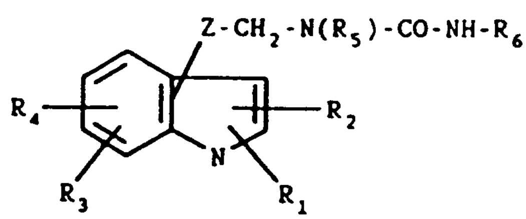

1

dans laquelle :

$R_1$ et $R_2$ qui peuvent être situés en position -1, -2 ou -3 du cycle de l'indole, représentent indépendamment un atome d'hydrogène, un radical alkyle linéaire de 1 à 12 atomes de carbone ou ramifié de 3 à 5 atomes de carbone, des radicaux alcényle en $C_3$-$C_6$, cycloalkyle en $C_3$-$C_8$, N-(alkyl en $C_1$-$C_5$) amino(alkyle en $C_1$-$C_5$) ou N,N-(dialkyl en $C_1$-$C_5$) amino(alkyle en $C_1$-$C_5$) ou l'un des substituants $R_1$ ou $R_2$ représente un radical pyridyl-2 (-3 ou -4) méthyle et l'autre un atome d'hydrogène, étant entendu que lorsque l'atome d'azote du cycle indolique n'est substitué par aucun des groupes $R_1$, $R_2$ ou $-Z-CH_2-N(R_5)CONHR_6$, il est substitué par un atome d'hydrogène,

$R_3$ et $R_4$ qui peuvent être situés en position -4, -5, -6 ou -7 du cycle de l'indole, représentent indépendamment des atomes d'hydrogène ou d'halogène ou des radicaux alkyle en $C_1$-$C_5$, alkoxy en $C_1$-$C_5$ ou alkylthio en $C_1$-$C_5$, ou l'un des substituants $R_3$ ou $R_4$ désigne un atome d'hydrogène et l'autre des radicaux trifluorométhyle, nitro, N-(alkyl en $C_1$-$C_5$) amino ou N,N-(dialkyl en $C_1$-$C_5$) amino,

ou trois des substituants $R_1$, $R_2$, $R_3$ ou $R_4$ ont les significations qui viennent d'être définies et le quatrième représente un radical de formule 2 suivante:

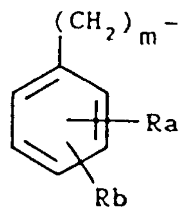

2

dans laquelle m peut prendre les valeurs 0, 1 ou 2 et les substituants Ra et Rb désignent indépendamment des atomes d'hydrogène ou d'halogène ou des radicaux alkyle en $C_1$-$C_5$,alkoxy en $C_1$-$C_5$ ou alkylthio en $C_1$-$C_5$,

$R_5$ désigne un atome d'hydrogène, un radical alkyle linéaire de 1 à 12 atomes de carbone ou ramifié de 3 à 5 atomes de carbone, un radical cycloalkyle en $C_3$-$C_8$ ou un radical de formule 2, dans laquelle m a la valeur 1 et Ra et Rb ont les significations définies précédemment, $R_6$ désigne un radical alkyle en $C_1$-$C_5$ ou un radical de formule 3 suivante,

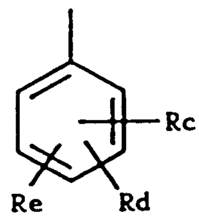

3

dans laquelle Rc, Rd et Re désignent indépendamment des atomes d'hydrogène ou d'halogène ou des radicaux alkyle en $C_1$-$C_5$, alkoxy en $C_1$-$C_5$ ou alkylthio en $C_1$-$C_5$, ou deux des substituants Rc, Rd et Re peuvent avoir les significations qui viennent d'être définies et le troisième représente un radical trifluorométhyle,

$R_6$ peut aussi désigner les radicaux naphtyl-1 ou -2 ou un radical hétérocyclyle à 5 ou 6 chaînons et à un ou deux hétéroatomes éventuellement fusionné avec un cycle benzénique et le cas échéant substitués par un à trois atomes d'halogène ou radicaux alkyle en $C_1$-$C_5$ ou alcoxy en $C_1$-$C_5$, Z, qui peut être relié aux sommets -1, -2, -3, -4, -5, - 6 ou -7 du cycle de l'indole désigne les biradicaux de formules : $-CH=CH-C(R_7R_8)-$ ou $-(CH_2)_nC(R_7R_8)-(CH_2)_p-$ dans lesquelles, n et p désignent deux nombres entiers pouvant prendre les valeurs 0, 1 et 2 à condition que leur somme (n + p) soit inférieure ou égale à 2,

$R_7$ et $R_8$ représentent indépendamment un atome d'hydrogène, un radical alkyle linéaire de 1 à 12 atomes de carbone ou ramifié de 3 à 5 atomes de carbone, des radicaux alcényle en $C_3$-$C_6$, cycloalkyle en $C_3$-$C_8$, N-(alkyl en $C_1$-$C_5$) amino, N,N-(dialkyl en $C_1$-$C_5$) amino, N-(alkyl en $C_1$-$C_5$) amino (alkyle en $C_1$-$C_5$) ou N,N-(dialkyl en $C_1$-$C_5$) amino (alkyle en $C_1$-$C_5$) ou un groupe de formule 2, dans laquelle m peut prendre les valeurs 0 ou 1 et Ra et Rb ont les significations définies précédemment,

$R_7$ et $R_8$ peuvent aussi former ensemble une chaîne polyméthylène : $-(CH_2)_q-$ dans laquelle q peut prendre les valeurs 3 à 8 et susceptible le cas échéant, lorsque q est supérieur ou égal à 5, de contenir une double liaison,

$R_7$ et $R_8$ peuvent aussi former ensemble les chaînes:-

$-CH_2-O-(CH_2)_2-$, $-(CH_2)_2-O-(CH_2)_2-$, $-CH_2-S-(CH_2)_2-$, $-(CH_2)_2-S-(CH_2)_2-$, $-CH_2-N(R_9)-(CH_2)_2-$ ou $-(CH_2)_2-N(R_9)-(CH_2)_2-$,

$R_5$, $R_7$ et $R_8$ peuvent aussi, lorsqu'ils représentent un radical cycloalkyle en $C_3$-$C_8$, contenir une double liaison,

et $R_9$ représente un radical alkyle en $C_1$-$C_5$,

comprenant la réaction d'un composé de formule générale 4

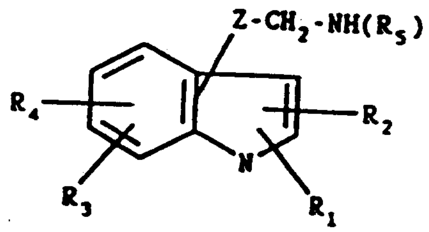

4

avec le phosgène et une amine $R_6NH_2$, ou avec un isocyanate $R_6NCO$ ou bien avec un trichloroacétamide $CCl_3$-CO-$NHR_6$.

**2.** Procédé selon la revendication 1, caractérisé en ce que les substituants $R_3$ et $R_4$ représentent des atomes d'hydrogène ou d'halogène ou des radicaux alkyle en $C_1$-$C_5$, N-(alkyl en $C_1$-$C_5$) amino, N,N-(dialkyl en $C_1$-$C_5$) amino ou alkoxy en $C_1$-$C_5$, $R_5$ représente un atome d'hydrogène, un radical alkyle linéaire en $C_1$-$C_{12}$ ou un groupe de formule 2 suivante,

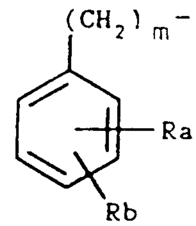

2

dans laquelle,
m = 1 et les substituants Ra et Rb designent indépendamment des atomes d'hydrogène ou d'halogène ou des radicaux alkyle en $C_1$-$C_5$, alkoxy en $C_1$-$C_5$ ou alkylthio en $C_1$-$C_5$ et $R_6$ désigne un groupe de formule 3,

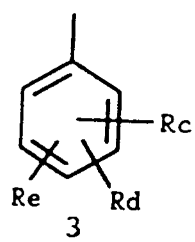

3

dans laquelle Rc, Rd et Re désignent indépendamment des atomes d'hydrogène ou d'halogène ou des radicaux alkyle en $C_1$-$C_5$, alkoxy en $C_1$-$C_5$ ou alkylthio en $C_1$-$C_5$ ou deux des substituants Rc, Rd et Re peuvent avoir les significations qui viennent d'être définies et le troisième représente un radical trifluorométhyle.

**3.** Procédé selon la revendication 1, caractérisé en ce que l'on prépare un composé choisi parmi les composés suivants :
N1-(Diisopropyl-2,6-phényl)-N2-[(méthyl-1-indolyl-3)-1-cyclopentylméthyl]urée, N1-[(Indolyl-1)-1-cyclopentylméthyl]-N2-(diisopropyl-2,6-phényl)urée, N1-(Diisopropyl-2,6-phényl)-N2-[[(méthyl-1-indolyl-3)-1-cyclopentyl]-3-propyl]urée,N1-(Diisopropyl-2,6-phényl)-N2-[[(méthyl-1-indolyl-3)-1-cyclopentyl]-2-éthyl]-urée,N1-Benzyl-N2-(diisopropyl-2,6-phényl)-N1-[(méthyl-1-indolyl-3)-1-cyclopentylméthyl]urée,N2-[-(méthyl-1-indolyl-3)-1-cyclopentylméthyl]-N1-phényl-urée, N1-[(Indolyl-3)-2-éthyl]-N2-(diisopropyl-2,6-phényl)urée, N1-(Difluoro-2,4-phényl)-N2-[(méthyl-1-indolyl-3)-1-cyclopentylméthyl]urée, N1-(Diisopropyl-2,6-phényl)-N2-(méthyl-3-(méthyl-1-indolyl-3)-2-butyl]urée, N1-*tert.*Butyl-N2-[(méthyl-1-indolyl-3)-1-cyclopentylméthyl]urée, N1-(Diisopropyl-2,6-phényl)-N2-[(méthyl-1-indolyl-3)-2-propyl]urée,N1-[(Benzyl-1-indolyl-3)-1-cyclopentylméthyl]-N2-(diisopropyl-2,6-phényl)urée, N1-[(Ethyl-1-indolyl-3)-1-cyclopentylméthyl]-N2-(diisopropyl-2,6-phényl)urée, N1-(Diisopropyl-2,6-phényl)-N2-[(méthyl-1-indolyl-3)-2-butyl]-

urée, N1-(Diisopropyl-2,6-phényl)-N2-[(diméthyl-1,5-indolyl-3)-1-cyclopentylméthyl]urée, N1-(Diisopropyl-2,6-phényl)-N2-((méthyl-1-indolyl-3)-1-cyclohexylméthyl]urée,N1-[Ethyl-2-(méthyl-1-indolyl-3)-2-butyl]-N2-(diisopropyl-2,6-phényl)urée,N1-(Diisopropyl-2,6-phényl)-N2-[méthyl-2-(méthyl-1-indolyl-3)-2-propyl]urée, N1-[(Isopropyl-1-indolyl-3)-1-cyclopentylméthyl]-N2-(diisopropyl-2,6-phényl)urée,N1-[(Allyl-1-indolyl-3)-1-cyclopentylméthyl]-N2-(diisopropyl-2,6-phényl)urée,N1-(Diisopropyl-2,6-phényl)-N2-[-(méthyl-1-indolyl-3)-2-phényl-2-éthyl]urée, N1-(Allyl-2-(méthyl-1-indolyl-3)-2-pentén-4-yl)-N2 -(Diisopropyl-2,6-phényl)urée, N1-(Diisopropyl-2,6-phényl)-N2-[(phényl-1-indolyl-3)-1-cyclopentylméthyl]urée,N1-(Diisopropyl-2,6-phényl)-N2-[(méthyl-1-indolyl-3)-1-cyclobutylméthyl]urée,N1-[Butyl-2-(méthyl-1-indolyl-3)-2-hexyl]-N2-(diisopropyl-2,6-phényl)urée, N1-(Diisopropyl-2,6-phényl)-N2-[(méthyl-1-indolyl-3)-2-pentèn-4-yl]urée, N1-[(Heptyl-1-indolyl-3)-1-cyclopentylméthyl]-N2-(diisopropyl2,6-phényl)urée. N1-[(Butyl-1-indolyl-3)-1-cyclopentylméthyl)]-N2-(diisopropyl-2,6-phényl)urée,N1-(Diisopropyl-2,6-phényl)-N2-[-(méthyl-1-indolyl-3)-2-hexyl]urée,N1-(Diisopropyl-2,6-phényl)-N2-[(méthyl-1-indolyl-3)-2-nonyl]urée, N1-(Diisopropyl-2,6-phényl)-N2-[(diméthyl-4,4-(méthyl-1-indolyl-3)-2-pentyl]urée, N1-(Diisopropyl-2,6-phényl)-N2-[(méthyl-1-indolyl-3)-2-phényl-3-propyl)urée, N1-(Dichloro-2,6-phényl)-N2-[(méthyl-1-indolyl-3)-1-cyclopentylméthyl]urée, N1-(Diisopropyl-2,6-phényl)-N2-[(méthyl-1-indolyl-3)-1-cycloheptylméthyl]-urée, N1-(Diisopropyl-2,6-phényl)-N2-[(méthyl-1-indolyl-3)-4-tétrahydropyranyl-4-méthyl]urée,N1-(Diéthyl-2,6-phényl)-N2-[(méthyl-1-indolyl-3)-1-cyclopentylméthyl]urée, N1-(Diisopropyl-2,6-phényl)-N2-[[-(diméthylamino-2-éthyl)-1-indolyl-3]-1-cyclopentylméthyl]urée, N1-[[(Fluoro-4-benzyl)-1-indolyl-3]-1-cyclopentylméthyl]-N2-(diisopropyl-2,6-phényl)urée, N1-(Triméthoxy-2,4,6-phényl)-N2-[(méthyl-1-indolyl-3)-1-cyclopentylméthyl]urée,N1-(Diméthoxy-4,6-pyrimidinyl-5)-N2-[(méthyl-1-indolyl-3)-1-cyclopentylméthyl]urée,N1-(Diisopropyl-2,6-phényl)-N2-[(méthoxy-5-méthyl-1-indolyl-3)-1-cyclopentylméthyl]urée, N1-(Diisopropyl-2,6-phényl)-N2-[diméthylamino-4-(méthyl-1-indolyl-3)-2-butyl]urée, N1-(Diisopropyl-2,6-phényl)-N2-[méthyl-5-(méthyl-1-indolyl-3)-2-hexèn-4-yl]urée,N1-(Diisopropropoxy-2,6-phényl)-N2-[(méthyl-1-indolyl-3)-1-cyclopentylméthyl]urée,N1-(Diisopr opyl-2,6-phényl)-N2-[[(pyridyl-3-méthyl)-1-indolyl-3]-1-cyclopentylméthyl]urée, N1-Benzyl-N1-[(méthyl-1-indolyl-3)-1-cyclopentylméthyl]-N2-(diméthyl-2,6-phényl)urée,N1-Benzyl-N2-(dichloro-2,6-phényl)-N1-[(méthyl-1-indolyl-3)-1-cyclopentylméthyl]urée,N1-Benzyl-N2-(difluoro-2,4-phényl)-N1-[(méthyl-1-indolyl-3)-1-cyclopentylméthyl]urée,N2-(Diisopropyl-2,6-phényl)-N1-méthyl-N1-[(méthyl-1-indolyl-3)-1-cyclopentylméthyl]urée, N1-(Diisopropyl-2,6-phényl)-N2-[-(diméthyl-1,2-indolyl-3)-1-cyclopentylméthyl]urée-N1-(Diisopropyl-2,6-phényl)-N2-[(méthyl-1-phényl-2-indolyl-3)-1-cyclopentylméthyl]urée,N1-(Diisopropyl-2,6-phényl)-N2-[(diméthyl-1,3-indolyl-2)-1-cyclopentylméthyl]urée,(-)-N 1-(Diisopropyl-2,6-phényl)-N2-[(méthyl-1-indolyl-3)-2-hexyl]urée,(+)-N1-(Diisopropyl-2,6-phényl)-N2-[(méthyl-1-indolyl3)-2-hexyl]urée.

4. Procédé selon la revendication 1, comprenant la réaction d'un composé de formule générale 5

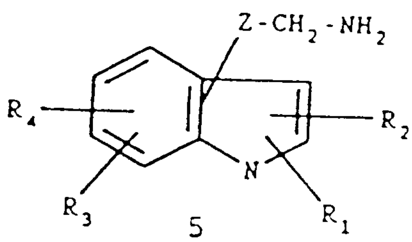

5

avec un composé halogéné $R_5$-X ou bien avec un aldéhyde $R_{10}$-CHO ou un chlorure d'acide $R_{10}$-COCl ou un anhydride d'acide $(R_{10}CO)_2O$ où $R_{10}$ représente un radical alkyle contenant éventuellement une double liaison ou un radical de formule 2 tel que défini dans la revendication 1 dans laquelle m est égal à O et $R_a$ et $R_b$ ont les significations définies à la revendication 1 suivie de la réduction de l'imine ou de l'amide formé, pour obtenir un composé de formule 4.

**5.** Procédé selon la revendication 4, comprenant la réduction d'un nitrile de formule 6,

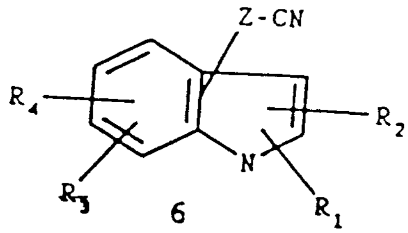

6

pour obtenir un composé de formule 5.

**6.** Procédé de préparation des composés de formule générale 6

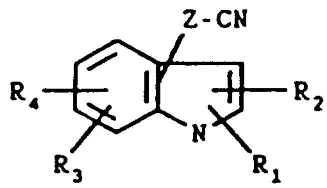

6

pour lesquels $R_1$, $R_2$, $R_3$ et $R_4$ ont la signification donnée à la revendication 1 et Z représente un biradical de formule $-(CH_2)_n-C(R_7R_8)-(CH_2)_p-$ dans laquelle n = p = O et $R_7$ et $R_8$ sont tels que définis dans la revendication 1, ledit procédé étant caractérisé en ce que l'on effectue les étapes a) ou b) suivantes :

a) on alkyle par l'iodure de méthyle un N,N-diméthylaminométhyl-indole de formule générale 7

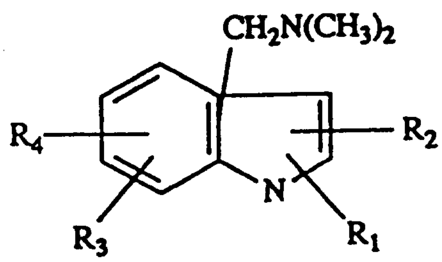

7

où $R_1$, $R_2$, $R_3$ et $R_4$ sont tels que définis dans la revendication 1, le produit obtenu est alors traité par KCN ou NaCN,

b) on N-alkyle par le chloroacétonitrile un composé de formule générale 8 :

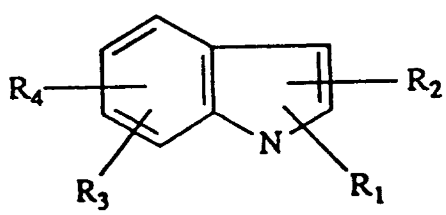

8

où $R_1$, $R_2$, $R_3$ et $R_4$ ont les significations indiquées ci-dessus, puis, le cas échéant, on effectue une mono ou di C-alkylation des composés obtenus dans les étapes a) ou b) avec un halogènure.

**7.** Procédé de préparation des composés de formule générale 6

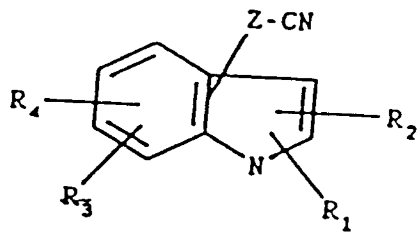

6

pour lesquels $R_1$, $R_2$, $R_3$ et $R_4$ ont la signification donnée à la revendication 1 et Z représente un biradical de formule -CH=CH-C($R_7R_8$) ou -C($R_7R_8$)-CH=CH-, dans lesquelles $R_7$ et $R_8$ sont tels que définis dans la revendication 1, ledit procédé étant caractérisé en ce que l'on fait réagir respectivement, un composé de formule 11 :

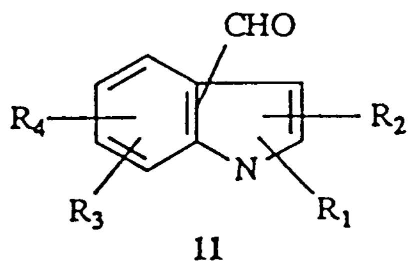

11

ou un composé de formule 14 :

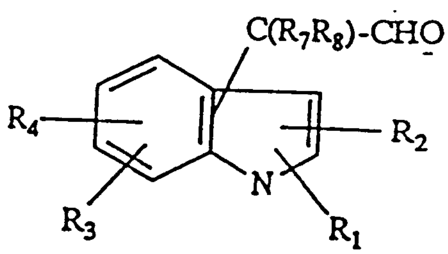

14

dans lesquelles $R_1$, $R_2$, $R_3$ et $R_4$ sont tels que définis dans la revendication 1, avec un phosphonate ou un cyanoalkylénetriphénylphosphorane.

**8.** Procédé de préparation des composés de formule générale 6

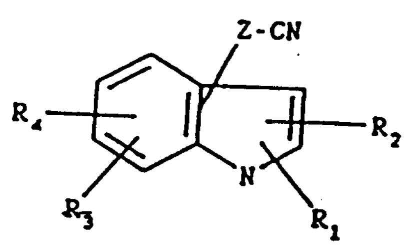

6

pour lesquels $R_1$, $R_2$, $R_3$ et $R_4$ ont la signification donnée à la revendication 1 et Z représente un biradical de formule $-(CH_2)_n-C(R_7R_8)-(CH_2)_p-$ dans laquelle $R_7$ et $R_8$ sont tels que définis dans la revendication 1, et soit n = 0 et p = 1, soit p = 0 et n = 1, ledit procédé étant caractérisé en ce qu'on réduit par le borohydrure un composé de formule 11 ou de formule 14, tels que définis dans la revendication 7, on transforme le produit ainsi obtenu en son chlorure correspondant par action du chlorure de thionyle, puis on fait réagir ledit chlorure avec un nitrile en présence d'une base.

**9.** Procédé de préparation d'une composition pharmaceutique caractérisé en ce que l'on mélange une quantité efficace d'au moins un composé de formule 1 tel que défini à la revendication 1 avec un excipient pharmaceutiquement acceptable.

**Claims**

**Claims for the following Contracting States : AT, BE, CH, LI, DE, DK, FR, GB, GR, IT, LU, NL, PT, SE**

**1.** New indole derivatives of the following general formula 1

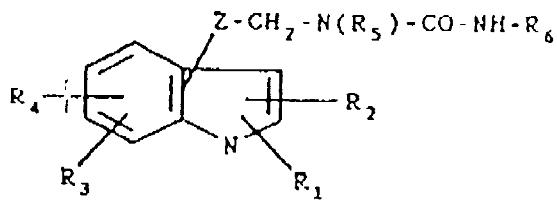

1

in which:

$R_1$ and $R_2$, which may be located at position 1-, 2- or 3- of the indole ring, independently represent a hydrogen atom, a linear alkyl radical having 1 to 12 carbon atoms or branched alkyl radical having 3 to 5 carbon atoms, or $C_3$-$C_6$ alkenyl, $C_3$-$C_8$ cycloaklkyl, N-($C_1$-$C_5$ alkyl)amino($C_1$-$C_5$ alkyl) or N,N-di($C_1$-$C_5$ alkyl)amino($C_1$-$C_5$ alkyl) radicals, or one of the substituents $R_1$ or $R_2$ represents a 2-pyridyl(or 3- or 4-pyridyl)methyl radical and the other a hydrogen atom, on the understanding that when the nitrogen atom of the indole ring is not substituted with any of the groups $R_1$, $R_2$ or $-Z-CH_2-N(R_5)CONHR_6$, it is substituted with a hydrogen atom,

$R_3$ and $R_4$, which may he located at position 4-, 5-, 6- or 7- of the indole ring, independently represent hydrogen or halogen atoms or $C_1$-$C_5$ alkyl, $C_1$-$C_5$ alkoxy or $C_1$-$C_5$ alhylthio radicals, or one of the substituents $R_3$ or $R_4$ denotes a hydrogen atom and the other substituent trifluoromethyl, nitro, N-($C_1$-$C_5$ alkyl)amino or N,N-di($C_1$-$C_5$ alkyl)amino radicals,

or three of the substituents, $R_1$, $R_2$, $R_3$ or $R_4$ have the meanings which have just been defined and the fourth represents a radical of the following formula 2:

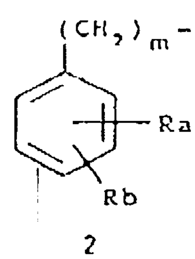

2

in which m can take the values 0, 1 or 2 and the substituents Ra and Rb independently denote hydrogen or halogen atoms or $C_1$-$C_5$ alkyl, $C_1$-$C_5$ alkoxy or $C_1$-$C_5$ alkylthio radicals,

$R_5$ denotes a hydrogen atom, a linear alkyl radical having 1 to 12 carbon atoms or branched alkyl radical having 3 to 5 carbon atoms, a $C_3$-$C_8$ cycloalkyl radical or a radical of formula 2, in which m has the value 1 and Ra and Rb having the meanings defined above,

$R_6$ denotes a $C_1$-$C_5$ alkyl radical or a radical of the following formula 3:

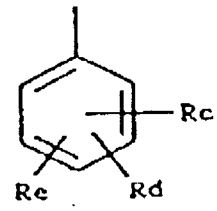

3

in which Rc, Rd and Re independently denote hydrogen or halogen atoms or $C_1$-$C_5$ alkyl, $C_1$-$C_5$ alkoxy or $C_1$-$C_5$ alkylthio radicals, or two of the substituents Rc, Rd and Re can have the meanings which have just been defined and the third represents a trifluoromethyl radical,
$R_6$ can also denote 1- or 2-naphthyl radicals or a 5- or 6-membered heterocyclic radical containing one or two heteroatoms, optionally fused with a benzene ring and, where appropriate, substituted with one to three halogen atoms, $C_1$-$C_5$alkyl radicals or $C_1$-$C_5$ alkoxy radicals, Z, which can be attached to positions 1-, 2-, 3-, 4-, 5-, 6- or 7- of the indole ring, denotes the bivalent radicals of formula $-CH=CH-C(R_7R_8)-$ or $-(CH_2)_nC(R_7R_8)-(CH_2)_p-$, in which n and p denote two integers which can take the values 0, 1 and 2, on condition that their sum (n + p) is less than or equal to 2,
$R_7$ and $R_8$ independently represent a hydrogen atom, a linear alkyl radical having 1 to 12 carbon atoms or branched alkyl radical having 3 to 5 carbon atoms, $C_3$-$C_6$ alkenyl, $C_3$-$C_8$ cycloalkyl, N-($C_1$-$C_5$ alkyl)-amino, N,N-di($C_1$-$C_5$ alkyl)amino, N-($C_1$-$C_5$ alkyl)amino($C_1$-$C_5$ alkyl) or N,N-di($C_1$-$C_5$ alkyl)amino($C_1$-$C_5$ alkyl) radicals or a group of formula 2, in which M can take the values 0 or 1 and Ra and Rb have the meanings defined above,
$R_7$ and $R_8$ can also form together a polymethylene chain $-(CH_2)_q-$in which q can take the values 3 to 8 and which is capable, where appropriate, when q is greater than or equal to 5, of containing a double bond,
$R_7$ and $R_8$ can also form together the chains:
$-CH_2-O-(CH_2)_2-$, $-(CH_2)_2-O-(CH_2)_2-$, $CH_2-S-(CH_2)_2-$, $-(CH_2)_2-S-(CH_2)_2-$, $-CH_2-N(R_9)-(CH_2)_2-$ or $-(CH_2)_2-N(R_9)-(CH_2)_2-$, $R_5$, $R_7$ and $R_8$ can also, when they represent a $C_3$-$C_8$ cycloalkyl radical, contain a double bond,
and $R_9$ represents a $C_1$-$C_5$ alkyl radical.

**2.** Compound according to claim 1, characterised in that the substituents $R_3$ and $R_4$ represent hydrogen or halogen atoms or $C_1$-$C_5$ alkyl, N-($C_1$-$C_5$ alkyl)amino, N,N-di($C_1$-$C_5$ alkyl)amino or $C_1$-$C_5$ alkoxy radicals, $R_5$ represents a hydrogen atom, a linear $C_1$-$C_{12}$ alkyl radical or a group of the following formula 2:

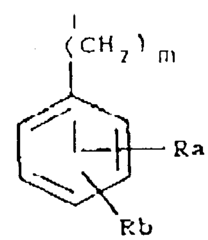

2

in which
m = 1 and the substituents Ra and Rb independently denote hydrogen or halogen atoms or $C_1$-$C_5$ alkyl, $C_1$-$C_5$ alkoxy or $C_1$-$C_5$ alkylthio radicals and $R_6$ denotes a group of formula 3:

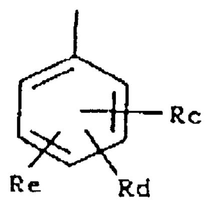

3

in which Rc, Rd and Re independently denote hydrogen or halogen atoms or $C_1$-$C_5$ alkyl, $C_1$-$C_5$ alkoxy or $C_1$-$C_5$ alkylthio radicals, or two of the substituents Rc, Rd and Re can have the meanings which have just been defined and the third represents a trifluoromethyl radical.

**3.** Compound according to Claim 1, chosen from amongst the following compounds is prepared: $N^1$-(2,6-Diisopropylphenyl)-$N^2$-[1-(methyl-3-indolyl)cyclopentylmethyl]urea, $N^1$-(1-(1-Indolyl)-cyclopentylmethyl]-$N^2$-(2,6-diisopropylphenyl)urea,$N^1$-(2,6-Diisopropyl-phenyl)-$N^2$-{3-[1-(1-methyl-3-indolyl)cyclopentyl]propyl}urea, $N^1$-(2,6-Diisopropylphenyl)-$N^2$-{2-[1-(1-methyl-3-indolyl)-cyclopentyl}ethyl)urea, $N^1$-Benzyl-$N^2$-(2,6-diisopropylphenyl)-$N^1$-[1-(1-methyl-3-indolyl)-cyclopentylmethyl]urea, $N^2$-[1-(Methyl-3-indolyl)cyclopentylmethyl]-$N^1$-phenylurea, $N^1$-[2-(3-Indolyl)-ethyl]-$N^2$-(2,6-diisopropylphenyl)urea, $N^1$-(2,4-Difluorophenyl)-$N^2$-[1-(1-methyl-3-indolyl)-cyclopentylmethyl)- urea, $N^1$-(2,6-Diisopropylphenyl)-$N^2$-[3-methyl-2-(1-methyl-3-indolyl)butyl) urea, $N^1$-tert-Butyl-$N^2$-[1-(1-methyl-3-indolyl)cyclopentylmethyl]urea, $N^1$-(2,6-Diisopropylphenyl)-$N^2$-[2-(1-methyl-3-indolyl)propyl]urea, $N^1$-[1-(1-Benzyl-3-indolyl)cyclopentylmethyl]-$N^2$-(2,6-diisopropylphenyl)urea, $N^1$-[1-(1-Ethyl-3-indolyl)cyclopentylmethyl]-$N^2$-(2,6-diisopropylphenyl)urea, $N^1$-(2,6-Diisopropylphenyl)-$N^2$-[2-(1-methyl-3-indolyl)butyl]urea, $N^1$-(2,6-Diisopropylphenyl)-$N^2$-[1-(1,5-dimethyl-3-indolyl)-cyclopentylmethyl]urea, $N^1$-(2,6-Diisopropylphenyl)-$N^2$-[1-(1-methyl-3-indolyl)cyclohexylmethyl]urea, $N^1$-[2-Ethyl-2-(1-methyl-3-indolyl)-2-butyl]-$N^2$-(2,6-diisopropylphenyl)urea, $N^1$-(2,6-Diisopropylphenyl)-$N^2$-[2-methyl-2-(1-methyl-3-indolyl)propyl]urea, $N^1$-[1-(1-Isopropyl-3-indolyl)cyclopentylmethyl]-$N^2$-(2,6-diisopropylphenyl)urea, $N^1$-[1-(1-Allyl-3-indolyl)cyclopentylmethyl]-$N^2$-(2,6-diisopropylphenyl)urea, $N^1$-(2,6-Diisopropylphenyl)-$N^2$-[2-(1-methyl-3-indolyl)-2-phenylethyl]urea, $N^1$-[2-Allyl-2-(1-methyl-3-indolyl)-4-pentenyl]-$N^2$-(2,6-diisopropylphenyl)urea, $N^1$-(2,6-Diisopropylphenyl)-$N^2$-[1-(1-phenyl-3-indolyl)-cyclopentylmethyl]-urea, $N^1$-(2,6-Diisopropylphenyl)-$N^2$-[1-(1-methyl-3-indolyl)cyclobutylmethyl]urea, $N^1$-[2-Butyl-2-(1-methyl-3-indolyl)hexyl)-$N^2$-(2,6-diisopropylphenyl)urea, $N^1$-2,6-Diisopropylphenyl)-$N^2$-[2-(1-methyl-3-indolyl)-4-pentenyl]urea, $N^1$-[1-(1-Heptyl-3-indolyl)cyclopentylmethyl]-$N^2$-(2,6-diisopropylphenyl)urea, $N^1$-[1-(1-Butyl-3-indolyl)cyclopentylmethyl)-$N^2$-(2,6-diisopropylphenyl)urea, $N^1$-(2,6-Diisopropylphenyl)-$N^2$-[2(1-methyl-3-indolyl)hexyl)urea,$N^1$-(2,6-Diisopropylphenyl)-$N^2$-[2-(1-methyl-3-indolyl)nonyl]urea, $N^1$-(2,6-Diisopropylphenyl)-$N^2$-[4,4-dimethyl-2-(1-methyl-3-indolyl)pentyl]urea, $N^1$-(2,6-Diisopropylphenyl)-$N^2$-[2-(1-methyl-3-indolyl)-3-phenylpropyl)urea, $N^1$-(2,6-Dichlorophenyl)-$N^2$-[1(1-methyl-3-indolyl)cyclopentylmethyl]urea, $N^1$-(2,6-Diisopropylphenyl)-$N^2$-[1-(1-methyl-3-indolyl)-cycloheptylmethyl]urea, $N^1$-(2,6-Diisopropylphenyl)-$N^2$-[4-(1-methyl-3-indolyl)-4-tetrahydropyranyl-methyl]urea, $N^1$-(2,6-Diethylphenyl)-$N^2$-[1-(1-methyl-3-indolyl)cyclopentylmethyl]urea, $N^1$-(2,6-Diisopropylphenyl)-$N^2$-{1-[1-(2-dimethylaminoethyl)-3-indolyl]cyclopentylmethyl}urea, $N^1$-{1-[1-(4-Fluorobenzyl)-3-indolyl]cyclopentylmethyl}-$N^2$-(2,6-diisopropylphenyl)urea, $N^1$-(2,4,6-Trimethoxyphenyl)-$N^2$-[1-(1-methyl-3-indolyl)cyclopentylmethyl]urea, $N^1$-(4,6-Dimethoxy-5-pyrimidinyl)-$N^2$-[1-(1-methyl-3-indolyl)cyclopentylmethyl]urea, $N^1$-(2,6-Diisopropylphenyl)-$N^2$-[1-(5-methoxy-1-methyl-3-indolyl)-cyclopentylmethyl]urea, $N^1$-(2,6-Diisopropylphenyl)-$N^2$-[4-dimethylamino-2-(1-methyl-3-indolyl)butyl]-urea, $N^1$-(2,6-Diisopropylphenyl)-$N^2$-[5-methyl-2-(1-methyl-3-indolyl)-4-hexenyl]urea, $N^1$-(2,6-Diisopropoxyphenyl)-$N^2$-[1-(1-methyl-3-indolyl)cyclopentylmetyl]urea, $N^1$-(2,6-Diisopropylphenyl)-$N^2$-{1-[1-(3-pyridylmethyl)-3-indolyl]cyclopentylmethyl}urea, $N^1$-Benzyl-$N^1$-[1-(1-methyl-3-indolyl)-cyclopentylmethyl]-$N^2$-(2,6-dimethylphenyl]urea, $N^1$-Benzyl-$N^2$-(2,6-dichlorophenyl)-$N^1$-[1-(1-methyl-3-indolyl)-cyclopentylmethyl]urea, $N^1$-Benzyl-$N^2$-(2,4-difluorophenyl)-$N^1$-[1(1-methyl-3-indolyl)-cyclopentylmethyl]-urea, $N^2$-(2,6-Diisopropylphenyl)-$N^1$-methyl-$N^1$-[1-(1-methyl-3-indolyl)cyclopentylmethyl]urea, $N^1$-(2,6-Diisopropylphenyl)-$N^2$-[1-(1,2-dimethyl-3-indolyl)-cyclopentylmethyl]urea, $N^1$-(2,6-Diisopropylphenyl)-$N^2$-[1-(1-methyl-2-phenyl-3-indolyl)cyclopentylmethyl]urea, $N^1$-(2,6-Diisopropylphenyl)-$N^2$-(1-(1,3-dimethyl-2-indolyl)cyclopentylmethyl]urea, (-)-$N^1$-(2,6-Diisopropylphenyl)-$N^2$-[2-(1-methyl-3-indolyl)hexyl]urea, (+)-$N^1$-(2,6-Diisopropylphenyl)-$N^2$-[2-(1-methyl-3-indolyl)hexyl]urea.

4. Process for preparing the compounds of formula 1 as defined in Claim 1, comprising the reaction of a compound of general formula 4

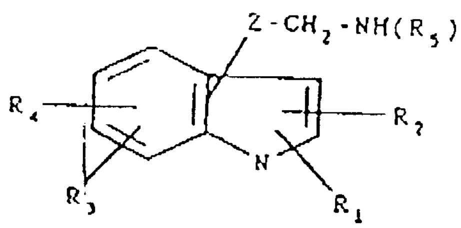

(4)

with phosgene and an amine $R_6NH_2$, or with an isocyanate $R_6NCO$, or alternatively with a trichloroacetamide $CC1_3$-CO-$NHR_6$.

5. Process according to Claim 4, comprising the reaction of a compound of general formula 5

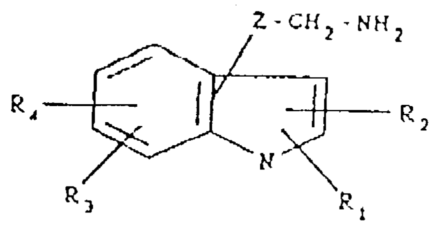

5

with a halogenated compound $R_5$-X or alternatively with an aldehyde $R_{10}$-CHO or an acid chloride $R_{10}$-COCl or an acid anhydride $(R_{10}CO)_2O$ where $R_{10}$ represents an alkyl radical optionally containing a double bond or a radical of formula 2 as defined in Claim 1 in which m is equal to 0 and $R_a$ and $R_b$ have the meanings defined in Claim 1, followed by the reduction of the imine or amide formed, to obtain a compound of formula 4.

6. Process according to Claim 5, comprising the reduction of a nitrile of formula 6

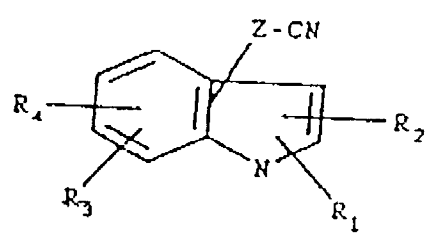

6

to obtain a compound of formula 5.

7. Intermediate compounds for the preparation of the compounds of general formula 1, chosen from amongst:
   . 1-(1-methyl-3-indolyl)cyclopentanecarbonitrile
   . 1-(1-methyl-3-indolyl)cyclopentylmethylamine
   . 1-(1-indolyl)cyclopentanecarbonitrile
   . 1-(1-indolyl)cyclopentylmethylamine
   . 3-[1-(1-methyl-3-indolyl)cyclopentyl]propylamine
   . 1-(1-methyl-3-indolyl)cyclopentaneacetonitrile
   . 2-[1-(1-methyl-3-indolyl)cyclopentyl]ethylamine
   . 3-[1-(1-methyl-3-indolyl)cyclopentyl]-2-propenenitrile

. N-benzyl-N[-1-(1-methyl-3-indolyl)cyclopentylmethyl]amine
. (-)-2-(1-methyl-3-indolyl)hexylamine
. (+)-2-(1-methyl-3-indolyl)hexylamine.

**8.** Process for preparing the compounds of general formula 6 in which Z represents a bivalent radical of formula $-(CH_2)_n-C(R_7R_8)-(CH_2)_p-$ in which n = p = 0 and $R_7$ and $R_8$ are as defined in Claim 1, the said process being characterised in that the following steps a) or b) are performed:

a) an N,N-dimethylaminomethylindole of general formula 7:

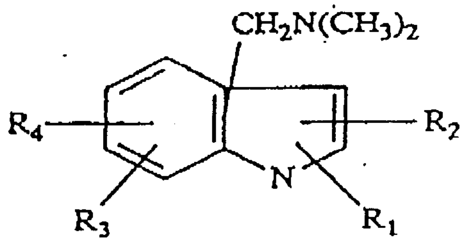

7

where $R_1$, $R_2$, $R_3$ and $R_4$ are as defined in Claim 1, is alkylated with methyl iodide and the product obtained in then treated with KCN or NaCN,

b) a compound of general formula 8:

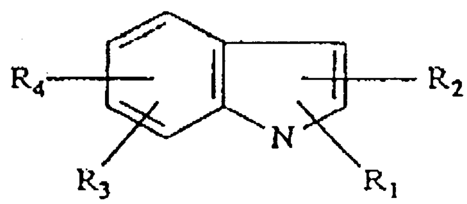

8

where $R_1$, $R_2$, $R_3$ and $R_4$ have the meanings stated above, is N-alkylated with chloroacetonitrile, and then, where appropriate, a mono- or di-C-alkylation of the compound obtained in step a) or b) is performed with a halide.

**9.** Process for preparing the compounds of general formula 6 in which Z represents a bivalent radical of formula $-CH=CH-C(R_7R_8)$ or $-C(R_7R_8)-CH=CH-$, in which $R_7$ and $R_8$ are as defined in Claim 1, the said process being characterised in that a compound of formula 11:

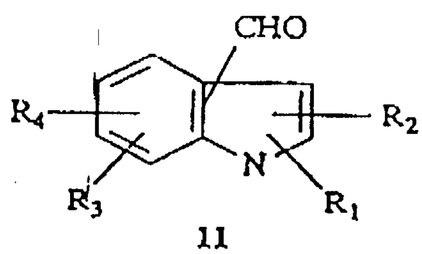

11

or a compound of formula 14:

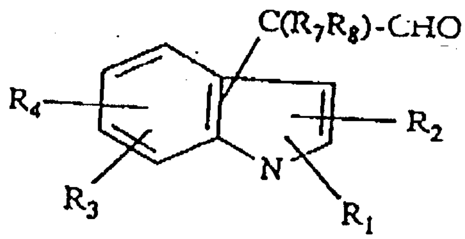

14

in which $R_1$, $R_2$, $R_3$ and $R_4$ are as defined in Claim 1, is reacted, respectively, with a phosphonate or a cyanoalkylenetriphenylphosphorane.

**10.** Process for preparing the compounds of general formula 6 in which Z represents a bivalent radical of formula $-(CH_2)_n-C(R_7R_8)-(CH_2)_p-$ in which $R_7$ and $R_8$ are as defined in Claim 1 and either N = 0 and p = 1 or p = 0 and n = 1, the said process being characterised in that a compound of formula 11 or of formula 14, as defined in Claim 9, is reduced with borohydride, the product thereby obtained is converted to its corresponding chloride by the action of thionyl chloride, and the said chloride is then reacted with a nitrile in the presence of a base.

**11.** Pharmaceutical compositions characterised in that they include an effective quantity of at least one compound of formula 1 as defined in Claim 1, mixed with a pharmaceutically acceptable excipient.

**12.** Medicaments, notably having hypolipidaemic, antiatherosclerotic and antidiabetic activities, according to Claim 11, in the form of a dosage unit in which each dosage unit contains 10 to 500 mg of active principle mixed with a pharmaceutically acceptable excipient.

**13.** Use of a compound according to Claim 1 for the preparation of a medicament intended for treating hyperglycaemia.

**14.** Use of a compound according to Claim 1 for the preparation of a medicament intended for the treatment of hyperlipidaemia.

**15.** Use of a compound according to Claim 1 for the preparation of a medicament intended for the treatment of atherosclerosis.

**Claims for the following Contracting State : ES**

**1.** Process for preparing new indole derivatives of the following general formula 1

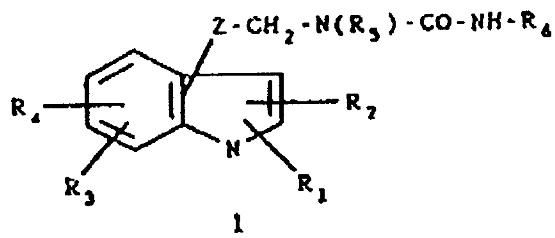

1

in which:

$R_1$ and $R_2$, which may be located at position 1-, 2- or 3- of the indole ring, independently represent a hydrogen atom, a linear alkyl radical having 1 to 12 carbon atoms or branched alkyl radical having 3 to 5 carbon atoms, or $C_3$-$C_6$ alkenyl, $C_3$-$C_8$ cycloaklkyl, N-($C_1$-$C_5$ alkyl)amino($C_1$-$C_5$ alkyl) or N,N-di($C_1$-$C_5$ alkyl)amino($C_1$-$C_5$ alkyl) radicals, or one of the substituents $R_1$ or $R_2$ represents a 2-pyridyl(or 3- or 4-pyridyl)methyl radical and the other a hydrogen atom, on the understanding that when the nitrogen

atom of the indole ring is not substituted with any of the groups $R_1$, $R_2$ or -Z-$CH_2$-N($R_5$)CONH$R_6$, it is substituted with a hydrogen atom,

$R_3$ and $R_4$, which may be located at position 4-, 5-, 6- or 7- of the indole ring, independently represent hydrogen or halogen atoms or $C_1$-$c_5$ alkyl, $C_1$-$C_5$ alkoxy or $C_1$-$C_5$ alkylthio radicals, or one of the substituents $R_3$ or $R_4$ denotes a hydrogen atom and the other substituent trifluoromethyl, nitro, N-($C_1$-$C_5$ alkyl)amino or N,N-di($C_1$-$C_5$ alkyl)amino radicals,

or three of the substituents, $R_1$, $R_2$, $R_3$ or $R_4$ have the meanings which have just been defined and the fourth represents a radical of the following formula 2:

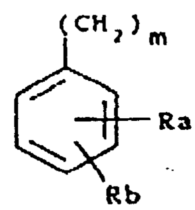

2

in which m can take the values 0, 1 or 2 and the substituents Ra and Rb independently denote hydrogen or halogen atoms or $C_1$-$C_5$ alkyl, $C_1$-$C_5$ alkoxy or $C_1$-$C_5$ alkylthio radicals,

$R_5$ denotes a hydrogen atom, a linear alkyl radical having 1 to 12 carbon atoms or branched alkyl radical having 3 to 5 carbon atoms, a $C_3$-$C_8$ cycloalkyl radical or a radical of formula 2, in which m has the value 1 and Ra and Rb having the meanings defined above,

$R_6$ denotes a $C_1$-$C_5$ alkyl radical or a radical of the following formula 3:

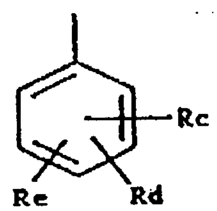

3

in which Rc, Rd and Re independently denote hydrogen or halogen atoms or $C_1$-$C_5$ alkyl, $C_1$-$C_5$ alkoxy or $C_1$-$C_5$ alkylthio radicals, or two of the substituents Rc, Rd and Re can have the meanings which have just been defined and the third represents a trifluoromethyl radical,

$R_6$ can also denote 1- or 2-naphthyl radicals or a 5- or 6-membered heterocyclic radical containing one or two heteroatoms, optionally fused with a benzene ring and, where appropriate, substituted with one to three halogen atoms, $C_1$-$C_5$ alkyl radicals or $C_1$-$C_5$ alkoxy radicals, Z, which can be attached to positions 1-, 2-, 3-, 4-, 5-, 6- or 7- of the indole ring, denotes the bivalent radicals of formula -CH=CH-C($R_7R_8$)- or -$(CH_2)_n$C($R_7R_8$)-$(CH_2)_p$-, in which n and p denote two integers which can take the values 0, 1 and 2, on condition that their sum (n+p) is less than or equal to 2,

$R_7$ and $R_8$ independently represent a hydrogen atom, a linear alkyl radical having 1 to 12 carbon atoms or branched alkyl radical having 3 to 5 carbon atoms, $C_3$-$C_6$ alkenyl, $C_3$-$C_8$ cycloalkyl, N-($C_1$-$C_5$ alkyl)-amino, N,N-di($C_1$-$C_5$ alkyl)amino, N-($C_1$-$C_5$ alkyl)amino($C_1$-$C_5$ alkyl) or N,N-di($C_1$-$C_5$ alkyl)amino($C_1$-$C_5$ alkyl) radicals or a group of formula 2, in which M can take the values 0 or 1 and Ra and Rb have the meanings defined above,

$R_7$ and $R_8$ can also form together a polymethylene chain -$(CH_2)_q$-in which q can take the values 3 to 8 and which is capable, where appropriate, when q is greater than or equal to 5, of containing a double bond,

$R_7$ and $R_8$ can also form together the chains:

-$CH_2$-O-$(CH_2)_2$-, -$(CH_2)_2$-O-$(CH_2)_2$-, -$CH_2$-S-$(CH_2)_2$-, -$(CH_2)_2$-S-$(CH_2)_2$-, -$CH_2$-N($R_9$)-$(CH_2)_2$- or -$(CH_2)_2$-N($R_9$-$(CH_2)_2$-, $R_5$, $R_7$ and $R_8$ can also, when they represent a $C_3$-$C_8$ cycloalkyl radical, contain a double bond,

and $R_9$ represents a $C_1$-$C_5$ alkyl radical,

comprising the reaction of a compound of general formula 4

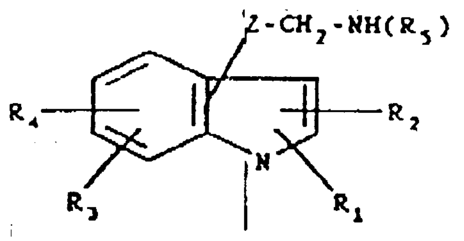

4

with phosgene and an amine $R_6NH_2$, or with an isocyanate $R_6NCO$ or alternatively with a trichloroacetamide $CCl_3$-CO-$NHR_6$.

2. Process according to Claim 1, characterised in that the substituents $R_3$ and $R_4$ represent hydrogen or halogen atoms or $C_1$-$C_5$ alkyl, N-($C_1$-$C_5$ alkyl)amino, N,N-di($C_1$-$C_5$ alkyl)amino or $C_1$-$C_5$ alkoxy radicals, $R_5$ represents a hydrogen atom, a linear $C_1$-$C_{12}$ alkyl radical or a group of the following formula 2:

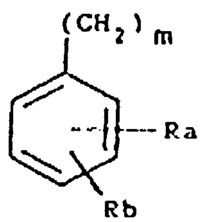

2

in which
m = 1 and the substituents Ra and Rb independently denote hydrogen or halogen atoms or $C_1$-$C_5$ alkyl, $C_1$-$C_5$ alkoxy or $C_1$-$C_5$ alkylthio radicals and $R_6$ denotes a group of formula 3:

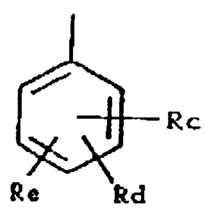

3

in which Rc, Rd and Re independently denote hydrogen or halogen atoms or $C_1$-$C_5$ alkyl, $C_1$-$C_5$ alkoxy or $C_1$-$C_5$ alkylthio radicals, or two of the substituents Rc, Rd and Re can have the meanings which have just been defined and the third represents a trifluoromethyl radical.

3. Process according to Claim 1, characterised in that a compound chosen from amongst the following compounds is prepared:
$N^1$-(2,6-Diisopropylphenyl)-$N^2$-[1-(methyl-3-indolyl)cyclopentylmethyl]urea, $N^1$-[1-(1-Indolyl)-cyclopentylmethyl]-$N^2$-(2,6-diisopropylphenyl)urea,$N^1$-(2,6-Diisopropyl-phenyl)-$N^2${3-[1-(1-methyl-3-indolyl)cyclopentyl]propyl}urea, $N^1$-(2,6-Diisopropylphenyl)-$N^2$-{2-[1-(1-methyl-3-indolyl)cyclopentyl]-ethyl}urea, $N^1$-Benzyl-$N^2$-(2,6-diisopropylphenyl)-$N^1$-[1-(1-methyl-3-indolyl)cyclopentylmethyl]urea, $N^2$-[1-(Methyl-3-indolyl)cyclopentylmethyl}-$N^1$-phenylurea, $N^1$-{2-(3-Indolyl)ethyl]-$N^2$-(2,6-diisopropyl-phenyl)urea, $N^1$-(2,4-Difluorophenyl)-$N^2$-[1-(1-methyl-3-indolyl)cyclopentylmethyl]- urea, $N^1$-(2,6-Diisopropylphenyl)-$N^2$-[3-methyl-2-(1-methyl-3-indolyl)butyl]urea, $N^1$-tert-Butyl-$N^2$-[1-(1-methyl-3-indolyl)cyclopentylmethyl]urea,$N^1$-(2,6-Diisopropylphenyl)-$N^2$-[2-(1-methyl-3-indolyl)propyl]urea, $N^1$-[1-(1-

Benzyl-3-indolyl)cyclopentylmethyl]-$N^2$-(2,6-diisopropylphenyl)urea, $N^1$-[1-(1-Ethyl-3-indolyl)-cyclopentylmethyl]-$N^2$-(2,6-diisopropylphenyl)urea, $N^1$-(2,6-Diisopropylphenyl)-$N^2$-[2-(1-methyl-3-indolyl)butyl]urea, $N^1$-(2,6-Diisopropylphenyl)-$N^2$-[1-(1,5-dimethyl-3-indolyl)cyclopentylmethyl]urea, $N^1$-(2,6-Diisopropylphenyl)-$N^2$-[1-(1-methyl-3-indolyl)cyclohexylmethyl)urea, $N^1$-[2-Ethyl-2-(1-methyl-3-indolyl)-2-butyl]-$N^2$-(2,6-diisopropylphenyl)urea, $N^1$-(2,6-Diisopropylphenyl)-$N^2$-[2-methyl-2-(1-methyl-3-indolyl)propyl]urea, $N^1$-[1-(1-Isopropyl-3-indolyl)cyclopentylmethyl]-$N^2$-(2,6-diisopropylphenyl)urea, $N^1$-[1-(1-Allyl-3-indolyl)cyclopentylmethyl]-$N^2$-(2,6-diisopropylphenyl)urea, $N^1$-(2,6-Diisopropylphenyl)-$N^2$-[2-(1-methyl-3-indolyl)-2-phenylethyl]urea, $N^1$-[2-Allyl-2-(1-methyl-3-indolyl)-4-pentenyl]-$N^2$-(2,6-diisopropylphenyl)urea, $N^1$-(2,6-Diisopropylphenyl)-$N^2$-[1-(1-phenyl-3-indolyl)cyclopentylmethyl]-urea, $N^1$-(2,6-Diisopropylphenyl)-$N^2$-[1-(1-methyl-3-indolyl)cyclobutylmethyl]urea, $N^1$-[2-Butyl-2-(1-methyl-3-indolyl)-hexyl]-$N^2$-(2,6-diisopropylphenyl)urea, $N^1$-2,6-Diisopropylphenyl)-$N^2$-[2-(1-methyl-3-indolyl)-4-pentenyl]-urea, $N^1$-[1-(1-Heptyl-3-indolyl)cyclopentylmethyl]-$N^2$-(2,6-diisopropylphenyl)urea, $N^1$-[1-(1-Butyl-3-indolyl)cyclopentylmethyl)-$N^2$-(2,6-diisopropylphenyl) urea, $N^1$-(2,6-Diisopropylphenyl)-$N^2$-[2(1-methyl-3-indolyl)hexyl]urea, $N^1$-(2,6-Diisopropylphenyl)-$N^2$-[2-(1-methyl-3-indolyl)nonyl]urea, $N^1$-(2,6-Diisopropylphenyl)-$N^2$-[4,4-dimethyl-2-(1-methyl-3-indolyl)pentyl]urea, $N^1$-(2,6-Diisopropylphenyl)-$N^2$-[2-(1-methyl-3-indolyl)-3-phenylpropyl]urea, $N^1$-(2,6-Dichiorophenyl)-$N^2$-[1(1-methyl-3-indolyl)cyclopentylmethyl]urea, $N^1$-(2,6-Diisopropylphenyl)-$N^2$-[1-(1-methyl-3-indolyl)cycloheptylmethyl]urea, $N^1$-(2,6-Diisopropylphenyl)-$N^2$-[4-(1-methyl-3-indolyl)-4-tetrahydropyranylmethyl]urea, $N^1$-(2,6-Diethylphenyl)-$N^2$-[1-(1-methyl-3-indolyl)cyclopentylmethyl]urea, $N^1$-(2,6-Diisopropylphenyl)-$N^2$-{1-[1-(2-dimethylaminoethyl)-3-indolyl]-cyclopentylmethyl}urea, $N^1$-{1-[1-(4-Fluorobenzyl)-3-indolyl]cyclopentylmethyl}-$N^2$-(2,6-diisopropylphenyl)urea, $N^1$-(2,4,6-Trimethoxyphenyl)-$N^2$-[1-(1-methyl-3-indolyl)cyclopentylmethyl]urea, $N^1$-(4,6-Dimethoxy-5-pyrimidinyl)-$N^2$-[1-(1-methyl-3-indolyl)cyclopentylmethyl]urea, $N^1$-(2,6-Diisopropylphenyl)-$N^2$-[1-(5-methoxy-1-methyl-3-indolyl)cyclopentylmethyl)urea, $N^1$-(2,6-Diisopropylphenyl)-$N^2$-[4-dimethylamino-2-(1-methyl-3-indolyl)butyl]urea, $N^1$-(2,6-Diisopropylphenyl)-$N^2$-[5-methyl-2-(1-methyl-3-indolyl)-4-hexenyl]urea, $N^1$-(2,6-Diisopropoxyphenyl)-$N^2$-[1-(1-methyl-3-indolyl)cyclopentylmetyl]urea, $N^1$-(2,6-Diisopropylphenyl)-$N^2$-{1-[1-(3-pyridylmethyl)-3-indolyl]cyclopentylmethyl}urea, $N^1$-Benzyl-$N^1$-[1-(1-methyl-3-indolyl)-cyclopentylmethyl]-$N^2$-(2,6-dimethylphenyl]urea, $N^1$-Benzyl-$N^2$-(2,6-dichlorophenyl)-$N^1$-[1-(1-methyl-3-indolyl)cyclopentylmethyl]urea, $N^1$-Benzyl-$N^2$-(2,4-difluorophenyl)-$N^1$-[1(1-methyl-3-indolyl)-cyclopentylmethyl]urea, $N^2$-(2,6-Diisopropylphenyl)-$N^1$-methyl-$N^1$-[1-(1-methyl-3-indolyl)cyclopentylmethyl]urea, $N^1$-(2,6-Diisopropylphenyl)-$N^2$-[1-(1,2-dimethyl-3-indolyl)-cyclopentylmethyl]urea, $N^1$-(2,6-Diisopropylphenyl)-$N^2$-[1-(1-methyl-2-phenyl-3-indolyl)cyclopentylmethyl]urea, $N^1$-(2,6-Diisopropylphenyl)-$N^2$-[1-(1,3-dimethyl-2-indolyl)cyclopentylmethyl]urea, (-)-$N^1$-(2,6-Diisopropylphenyl)-$N^2$-[2-(1-methyl-3-indolyl)hexyl]urea, (+)-$N^1$-(2,6-Diisopropylphenyl)-$N^2$-[2-(1-methyl-3-indolyl)-hexyl] urea.

**4.** Process according to Claim 1, comprising the reaction of a compound of general formula 5

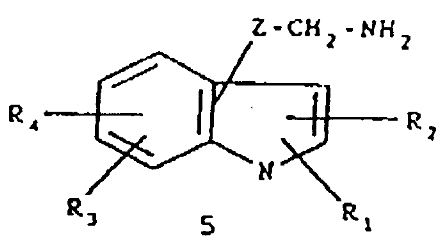

5

with a halogenated compound $R_5$-X or else with an aldehyde $R_{10}$-CHO or on acid chloride $R_{10}$-COCl or an acid anhydride $(R_{10}CO)_2O$ where $R_{10}$ represents an alkyl radical possibly containing a double bond or a radical of formula 2 as defined in claim 1 in which m is equal to O and $R_a$ and $R_b$ have the meanings defined in Claim 1, followed by reduction of the imine or amide formed, in order to obtain a compound of formula 4.

**5.** Process according to Claim 4, comprising the reduction of a nitrile of formula 6

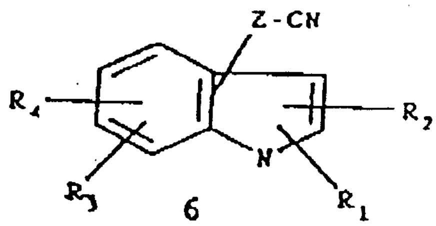

6

in order to obtain a compound of formula 5.

**6.** Process for preparing compounds of general formula 6

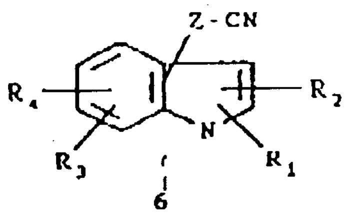

6

for which $R_1$, $R_2$, $R_3$ and $R_4$ have the meaning given in Claim 1 and Z represents a bivalent radical of formula $-(CH_2)_n-C(R_7R_8)-(CH_2)_p-$ in which n = p = O and $R_7$ and $R_8$ are as defined in Claim 1, the said process being characterised in that the following steps a) or b) are carried out:

a) an N,N-dimethyl-aminomethyl-indolyl of general formula 7

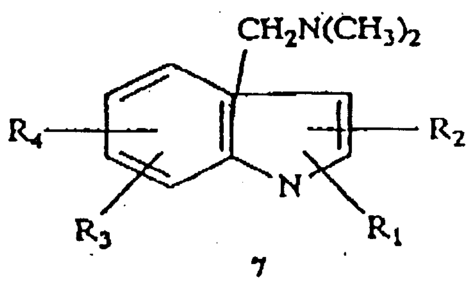

7

where $R_1$, $R_2$, $R_3$ and $R_4$ are as defined in Claim 1, are alkylated using methyl iodide, and the product obtained is then treated by means of KCN or NaCn,

b) a compound of general formula 8

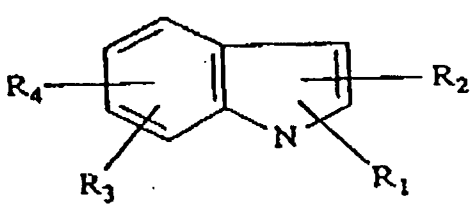

8

where $R_1$, $R_2$, $R_3$ and $R_4$ have the meanings given above, is N-alkylated using chloroacetonitrile, and then, where necessary, a mono or di C-alkylation of the compounds obtained in steps a) or b) is carried out with a halide.

**7.** Process for preparing compounds of general formula 6

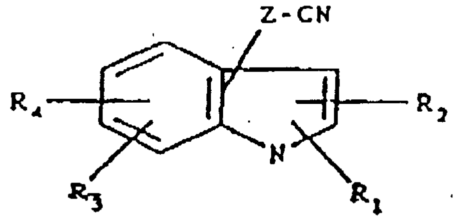

6

for which $R_1$, $R_2$, $R_3$ and $R_4$ have the meaning given in Claim 1 and Z represents a bivalent radical of formula -CH=CH-C($R_7R_8$) or -C($R_7R_8$)-CH=CH-, in which $R_7$ and $R_8$ are as defined in Claim 1, the said process being characterised in that a compound of formula 11

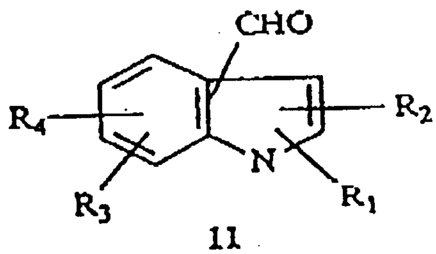

11

or a compound of formula 14

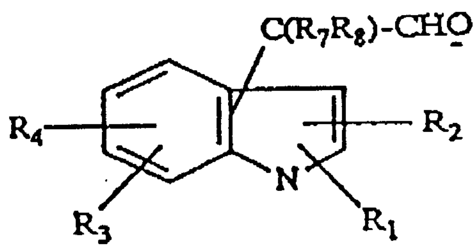

14

in which $R_1$, $R_2$, $R_3$ and $R_4$ are as defined in Claim 1, are reacted respectively with a phosphonate or a cyanoalkylenetriphenylphosphorane.

**8.** Process for preparing compounds of general formula 6

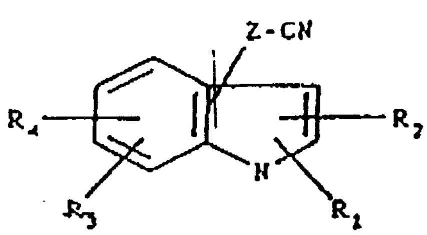

6

for which $R_1$, $R_2$, $R_3$ and $R_4$ have the meaning given in Claim 1 and Z represents a bivalent radical of formula $-(CH_2)_n-C(R_7R_8)-(CH_2)_p-$ in which $R_7$ and $R_8$ are as defined in Claim 1, and either n = 0 and p = 1, or p = 0 and n = 1, the said process being characterised in that a compound of formula 11 or formula 14, as defined in Claim 7, are reduced using borohydryde, the product thus obtained is converted into its corresponding chloride by the action or thionyl chloride, and then the said chloride is reacted with a nitrile in the presence of a base.

**9.** Process for preparing a pharmaceutical composition, characterised in that on effective quantity of at least one compound of formula 1 as defined in Claim 1 is mixed with a pharmaceutically acceptable excipient.

**Patentansprüche**

**Patentansprüche für folgende Vertragsstaaten : AT, BE, CH, LI, DE, DK, FR, GB, GR, IT, LU, NL, PT, SE**

**1.** Neue Indolderivate der folgenden allgemeinen Formel 1

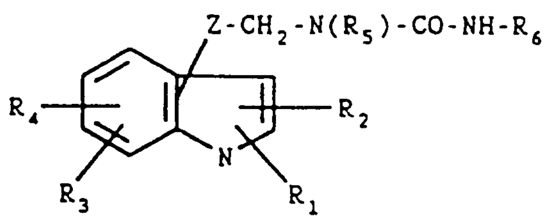

1

worin $R_1$ und $R_2$, die in 1-, 2- oder 3-Position des Indolrings angeordnet sein können, unabhängig voneinander ein Wasserstoffatom, einen geradkettigen Alkylrest mit 1 bis 12 Kohlenstoffatomen oder einen verzweigten Alkylrest mit 3 bis 5 Kohlenstoffatomen, $C_3$-$C_6$-Alkenylreste, $C_3$-$C_8$-Cycloalkylreste, N-($C_1$-$C_5$-Alkyl) amino($C_1$-$C_5$-alkyl)reste oder N,N-($C_1$-$C_5$-Dialkyl)amino($C_1$-$C_5$-alkyl)reste bedeuten oder einer der Substituenten $R_1$ oder $R_2$ einen 2-(3- oder 4-)Pyridylmethylrest und der andere ein Wasserstoffatom bedeutet,

wobei in dem Falle, in dem das Stickstoffatom des Indolrings nicht durch eine der Gruppen $R_1$, $R_2$ oder $-Z-CH_2-N(R_5)CONHR_6$ substituiert ist, es durch ein Wasserstoffatom substituiert ist,

$R_3$ und $R_4$, die in 4-, 5-, 6- oder 7-Position des Indolrings angeordnet sein können, bedeuten unabhängig voneinander Wasserstoff- oder Halogenatome oder $C_1$-$C_5$-Alkylreste, $C_1$-$C_5$-Alkoxyreste oder $C_1$-$C_5$-Alkylthioreste oder einer der Substituenten $R_3$ oder $R_4$ bezeichnet ein Wasserstoffatom und der andere einen Trifluormethylrest, Nitrorest, N-($C_1$-$C_5$-Alkyl)aminorest oder N,N-($C_1$-$C_5$-Dialkyl)-aminorest, oder

drei der Substituenten $R_1$, $R_2$, $R_3$ oder $R_4$ die zuvor definierten Bedeutungen haben und der vierte einen Rest der folgenden Formel 2 bedeutet

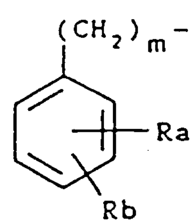

2

in der m die Werte 0, 1 oder 2 haben kann und die Substituenten Ra und Rb unabhängig voneinander Wasserstoffatome oder Halogenatome oder $C_1$-$C_5$-Alkylreste, $C_1$-$C_5$-Alkoxyreste oder $C_1$-$C_5$-Alkylthioreste bedeuten,

$R_5$ ein Wasserstoffatom, einen geradkettigen Alkylrest mit 1 bis 12 Kohlenstoffatomen oder verzweigten Alkylrest mit 3 bis 5 Kohlenstoffatomen, einen $C_3$-$C_8$-Cycloalkylrest oder einen Rest der Formel 2 bedeutet, in der m den Wert 1 hat und Ra und Rb die zuvor definierten Bedeutungen haben, $R_6$ einen $C_1$-$C_5$-Alkylrest oder einen Rest der folgenden Formel 3

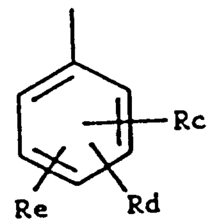

3

bedeutet, worin Rc, Rd und Re unabhängig voneinander Wasserstoffatome oder Halogenatome oder $C_1$-$C_5$-Alkylreste, $C_1$-$C_5$-Alkoxyreste oder $C_1$-$C_5$-Alkylthioreste bedeuten oder worin 2 der Substituenten Rc, Rd und Re die zuvor angegebenen Bedeutungen haben können und der dritte ein Trifluormethylrest bedeutet,

$R_6$ auch 1- oder 2-Naphtylreste oder einen 5- oder 6-gliedrigen Heterozyklus bezeichnen und ein oder zwei Heteroatome gegebenenfalls einen Benzolring bilden und gegebenenfalls mit einem bis drei Halogenatomen oder $C_1$-$C_5$-Alkylresten oder $C_1$-$C_5$-Alkoxyresten substituiert sind,

Z, das mit den 1-, 2-, 3-, 4-, 5-, 6- oder 7-Positionen des Indolrings verbunden sein kann, zweiwertige Reste der Formel $-CH=CH-C(R_7R_8)-$ oder $-(CH_2)_nC(R_7R_8)-(CH_2)_p-$ bezeichnet, worin n und p ganze Zahlen sind und die Werte 0, 1 und 2 unter der Bedingung haben können, daß ihre Summe $(n+p)$ kleiner oder gleich 2 ist,

$R_7$ und $R_8$ unabhängig voneinander ein Wasserstoffatom, einen geradkettigen Alkylrest mit 1 bis 12 Kohlenstoffatomen oder verzweigten Alkylrest mit 3 bis 5 Kohlenstoffatomen, $C_3$-$C_6$-Alkenylreste, $C_3$-$C_8$-Cycloalkylreste, N-($C_1$-$C_5$-Alkyl)amino, N,N-($C_1$-$C_5$-Dialkyl)amino, N-($C_1$-$C_5$Alkyl)amino($C_1$-$C_5$-alkyl) oder N,N-($C_1$-$C_5$-Dialkyl)amino($C_1$-$C_5$-alkyl) oder eine Gruppe der Formel 2 bedeuten, in der m die Werte 0 oder 1 haben kann und Ra und Rb die zuvor angegebenen Bedeutungen haben,

$R_7$ und $R_8$ gemeinsam eine Polymethylenkette $-(CH_2)_q-$ bilden können, worin q die Werte 3 bis 8 haben kann und gegebenenfalls, wenn q größer oder gleich 5 ist, eine Doppelbindung enthalten kann,

$R_7$ und $R_8$ auch gemeinsame Ketten $-CH_2-O-(CH_2)_2-$, $-(CH_2)_2-O-(CH_2)_2-$, $-CH_2-S-(CH_2)_2-$, $-(CH_2)_2-S-(CH_2)_2-$, $-CH_2-N(R_9)-(CH_2)_2-$ oder $-(CH_2)_2-N(R_9)-(CH_2)_2-$, wobei $R_5$, $R_7$ und $R_8$, wenn sie einen $C_3$-$C_8$-Cycloalkylrest bedeuten, eine Doppelbindung aufweisen können und

$R_9$ bedeutet einen $C_1$-$C_5$-Alkylrest.

**2.** Verbindungen nach Anspruch 1, dadurch gekennzeichnet, daß die Substituenten $R_3$ und $R_4$ Wasserstoffatome oder Halogenatome oder $C_1$-$C_5$-Alkylreste, N-($C_1$-$C_5$-Alkyl)aminoreste, N,N-($C_1$-$C_5$-Dialkyl)-aminoreste oder $C_1$-$C_5$-Alkoxyreste bedeuten, $R_5$ ein Wasserstoffatom, einen geradkettigen $C_1$-$C_{12}$-Alkylrest oder eine Gruppe der folgenden Formel

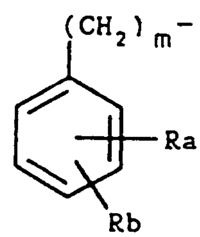

2

bedeutet, worin m gleich 1 ist und die Substituenten Ra und Rb unabhängig voneinander Wasserstoffatome oder Halogenatome oder $C_1$-$C_5$-Alkylreste, $C_1$-$C_5$-Alkoxyreste oder $C_1$-$C_5$-Alkylthioreste bedeuten und $R_6$ eine Gruppe der Formel 3

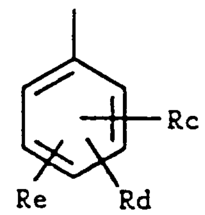

3

bedeutet, worin Rc, Rd und Re unabhängig voneinander Wasserstoffatome, Halogenatome oder $C_1$-$C_5$-Alkylreste, $C_1$-$C_5$-Alkoxyreste oder $C_1$-$C_5$-Alkylthioreste bedeuten oder zwei der Substituenten Rc, Rd und Re die zuvor angegebene Bedeutung haben können und der dritte einen Trifluormethylrest bedeutet.

**3.** Verbindung nach Anspruch 1, ausgewählt aus den folgenden Verbindungen: 1N-(2,6-Diisopropylphenyl)-2N-[1-(1-methyl-3-indolyl)cyclopentylmethyl]harnstoff, 1N-[1-(1-Indolyl)cyclopentylmethyl]-2N-(2,6-diisopropylphenyl)harnstoff, 1N-(2,6-Diisopropylphenyl)-2N-[3-[1-(1-methyl-3-indolyl)cyclopentyl]propyl]-harnstoff, 1N-(2,6-Diisopropylphenyl)-2N-[2-[1-(1-methyl-3-indolyl)cyclopentyl]ethyl]harnstoff, 1N-Benzyl-2N-(2,6-diisopropylphenyl)-1N-[1-(1-methyl-3-indolyl)cyclopentyl-methyl]harnstoff, 2N-[1-(1-Methyl-3-indolyl)cyclopentylmethyl]-1N-phenylharnstoff, 1N-[2-(3-Indolyl)ethyl]-2N-(2,6-diisopropyl-phenyl)-harnstoff, 1N-(2,4-Difluorphenyl)-2N-[1-(1-methyl-3-indolyl-cyclopentylmethyl]harnstoff, 1N-(2,6-Diisopropylphenyl)-2N-[3-methyl-2-(1-methyl-3-indolyl)butyl]harnstoff, 1N-tert.Butyl-2N-[1-(1-methyl-3-indolyl)-cyclopentylmethyl]harnstoff, 1N-(2,6-Diisopropylphenyl)-2N-[2-(1-methyl-3-indolyl)propyl]harnstoff, 1N-[1-(1-Benzyl-3-indolyl)-cyclopentylmethyl]-2N-(2,6-diisopropylphenyl)harnstoff, 1N[1-(1-Ethyl-3-indolyl)-cyclopentyl]-2N-(2,6-diisopropylphenyl)harnstoff, 1N-(2,6.-Diisopropylphenyl)-2N-[2-(1-methyl-3-indolyl)-butyl]harnstoff, 1N-(2,6-Diisopropylphenyl)-2N-[1-(1,5-dimethyl-3-indolyl)cyclopentylmethyl]harn-stoff, 1N-(2,6-Diisopropylphenyl)-2N-[1-(1-methyl-3-indolyl)cyclohexylmethyl]harnstoff, 1N-[2-Ethyl-2-(1-methyl-3-indolyl)butyl]-2N(2,6-diisopropylphenyl)harnstoff, 1N-(2,6-Diisopropylphenyl)-2N-[2-methyl-2-(1-methyl-3-indolyl)propyl]harnstoff, 1N-[1-(1-Isopropyl-1-indolyl-3)-1-cyclopentylmethyl]-2N-(2,6diisopropylphenyl)harnstoff, 1N-[1-(1-Allyl-3-indolyl)cyclopentylmethyl]-2N-(2,6-diisopropylphenyl)harnstoff, 1N-(2,6-Diisopropylphenyl)-2N-[2-(1-methyl-3-indolyl)2-phenylethyl]harnstoff, 1N-[2-Allyl-2-(1-methyl-3-indolyl)-4-pentenyl]-2N-(2,6-diisopropyl-phenyl)harnstoff, 1N-(2,6-Diisopropylphenyl)-2N-[1-(1-phenyl-3-indolyl)cyclopenthylmethyl]harnstoff, 1N-(2,6-Diisopropylphenyl)-2N-[1-(1-methyl-3-indolyl)-cyclobutylmethyl]harnstoff, 1N-[2-Butyl-2-(1-methyl-3-indolyl)hexyl]-2N-(2,6diisopropylphenyl)harnstoff, 1N-(2,6-Diisopropylphenyl)-2N-[2-(1-methyl-3-indolyl)-4-pentenyl]harnstoff, 1N-[1-(1-Heptyl-3-indolyl)-cyclopentylmethyl]-2N-(2,6-diisopropylphenyl)-harnstoff, 1N[1-(1-Butyl-3-indolyl)cyclopentylmethyl]-2N-(2,6-diisopropylphenyl)harnstoff, 1N-(2,6-Diisopropylphenyl)-2N-[2-(1-methyl-3-indolyl)hexyl]harnstoff, 1N-(2,6-Diisopropylphenyl)-2N-[2-(1-methyl-3-indolyl)nonyl]harnstoff, 1N-(2,6-Diisopropylphenyl)-2N-[4,4-dimethyl-2-(1-methyl-3-indolyl)pentyl]harnstoff, 1N-(2,6-Diisopropylphenyl)-2N-[2-(1-methyl-3-indolyl)-3-phenylpropyl]harnstoff, 1N-(2,6-Dichlorphenyl)-2N-[1-(1-methyl-3-indolyl(cyclopentylmethyl]-harnstoff, 1N-(2,6-Diisopropyl-phenyl)-2N-[1-(1-methyl-3-)cycloheptylmethyl]harnstoff, 1N-(2,6-Diisopropylphenyl)-2N-[4-(1-methyl-3-indolyl)-4-tetrahydropyranylmethyl] harnstoff, 1N-(2,6-Diethylphenyl)-2N-[1-(1-methyl-3-indolyl)cyclopentyl-methyl]harnstoff, 1N-2,6-Diisopropylphenyl)-2N-[1-[1-(2-dimethylamino-ethyl)-3-indolyl]cyclopentylmethyl]harnstoff, 1N-[1-[1-(4-Fluorbenzyl)-3-indolyl]cyclopentylmethyl]-2N-(2,6-diisopropylphenyl)harnstoff, 1N-(2,4,6-Trimethoxyphenyl)-2N-[1-(1-methyl-3-indolyl)-cyclopenthylmethyl]harnstoff, 1N-(4,6-Dimethoxy-5-pyrimidinyl)-2N-[1-(1-methyl-3-indolyl)cyclo-penthylmethyl]harnstoff, 1N-(2,6-Diisopropylphenyl)-2N-[1-(5-methoxy-1-methyl-3-indolyl)cyclopenthylbutyl]-harnstoff,
1N-(2,6-Diisopropylphenyl)-2N-[4-dimethylamino-2-(1-methyl-3-indolyl)butyl]harnstoff, 1N-(2,6-Diisopropylphenyl)-2N-[5-methyl-2-(1-methyl-3-indolyl)hexen-4-yl]harnstoff, 1N-(2,6-Diisopropoxyphenyl)-2N-[1-(1-methyl-3-indolyl)cyclopentylmethyl]harnstoff, 1N-(2,6-Diisopropylphenyl)-2N-[1-[1-(3-pyridylmethyl)-3-indolyl]cyclo-pentylmethyl]harnstoff, 1N-Benzyl-1N-[1-(1-methyl-3-indolyl)cyclo-pentylmethyl]-2N-(2,6-dimethylphenyl)harnstoff, 1N-Benzyl-2N-(2,6-dichlorphenyl)-1N-[1-(1-methyl-3-indolyl)-cyclopentylmethyl]harnstoff, 1N-Benzyl-2N-(2,4-difluorphenyl)-1N-]1-(1-methyl-3-indolyl)cyclopentyl-methyl]harnstoff, 2N-(2,6-Diisopropylphenyl)-1N-methyl-1N-[1-(1-methyl-3-indolyl)cyclopentylmethyl]-harnstoff, 1N-(2,6-Diisopropylphenyl)-2N-[1-(1,2dimethyl-3-indolyl)cyclopentylmethyl]harnstoff, 1N-(2,6-Diisopropyl-phenyl)-2N-[1-(1-methyl-2-phenyl-3-indolyl)cyclopentylmethyl]harnstoff, 1N-(2,6-Diisopropylphenyl)-2N-[1-(1,3-dimethyl-2-indolyl)cyclopentyl-methyl]harnstoff, (-)-1N-(2,6-Diisopropylphenyl)-2N-[2-(1-methyl-3-indolyl)hexyl]harnstoff, (+)-1N-(2,6-Diisopropylphenyl)-2N-[2-(1-methyl-3-indolyl)hexyl]-harnstoff.

4. Verfahren zur Herstellung der Verbindungen der Formel 1, wie sie in Anspruch 1 definiert sind, umfassend die Umsetzung einer Verbindung der allgemeinen Formel 4

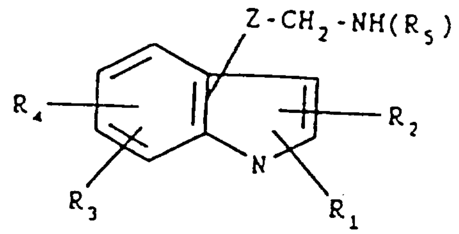

4

mit Phosgen und einem Amin $R_6NH_2$ oder mit einem Isocyanat $R_6NCO$ oder auch mit einem Trichloracetamid $CCl_3$-CO-$NHR_6$.

5. Verfahren nach Anspruch 4, umfassend die Umsetzung einer Verbindung der allgemeinen Formel 5

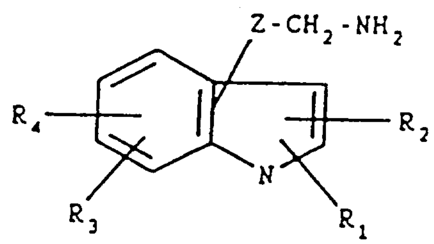

5

mit einer Halogenverbindung $R_5$-X oder auch mit einem Aldehyd $R_{10}$-CHO oder einem Säurechlorid $R_{10}$-COCl oder einem Säureanhydrid $(R_{10}CO)_2O$, worin $R_{10}$ einen Alkylrest bedeutet, der gegebenenfalls eine Doppelbindung aufweist, oder einen Rest der Formel 2, wie er in Anspruch 1 definiert ist, in dem m gleich 0 ist und $R_a$ und $R_b$, die in Anspruch 1 angegebenen Bedeutungen haben, gefolgt von der Reduktion des Imins oder des gebildeten Amids, um eine Verbindung der Formel 4 zu erhalten.

6. Verfahren nach Anspruch 5, umfassend die Reduktion eines Nitrils der Formel 6

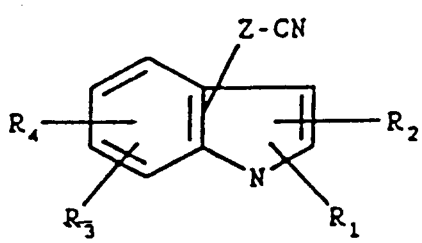

6

um eine Verbindung der Formel 5 zu erhalten.

7. Zwischenverbindungen zur Herstellung der Verbindungen der allgemeinen Formel I, ausgewählt aus
   - 1-(1-Methyl-3-indolyl)cyclopentancarbonitril,
   - 1-(1-Methyl-3-indolyl)cyclopentylmethylamin,
   - 1-(1-Indolyl)cyclopentancarbonitril,
   - 1-(1-Indolyl)cyclopentylmethylamin,
   - 3-[1-(1-Methyl-3-indolyl)cyclopentyl]propylamin,

- 1-(1-(1-Methyl-3-indolyl)cyclopentanacetonitril,
- 2-[1-)1-Methyl-3-indolyl)cyclopentyl]ethylamin,
- 3-[1-(1-Methyl-3-indolyl)cyclopentyl]-2-propennitril,
- N-Benzyl-N[1-(1-methyl-3-indolyl) cyclopentylmethyl]amin
- (-)-2-(1-Methyl-3-indolyl)hexylamin,
- (+)-2-(1-Methyl-3-indolyl)hexylamin.

**8.** Verfahren zur Herstellung von Verbindungen der allgemeinen Formel 6 in der Z einen zweiwertigen Rest der Formel $-(CH_2)_n-C(R_7R_8)-(CH_2)_p-$bedeutet, worin n = P = O ist und $R_7$ und $R_8$ die in Anspruch 1 angegebene Bedeutung haben, wobei das Verfahren dadurch gekennzeichnet ist, daß man die folgenden Stufen a) oder b) durchführt.

a) Man alkyliert mit Methyljodid ein N,N-Dimethylaminomethylindol der allgemeinen Formel 7:

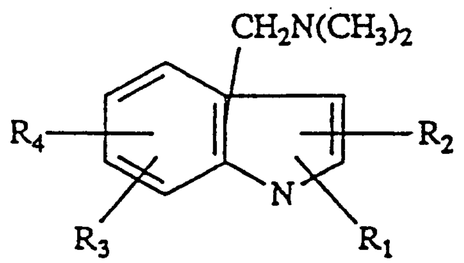

7

worin $R_1$, $R_2$, $R_3$ und $R_4$ die in Anspruch 1 angegebene Bedeutung haben, und behandelt das erhaltene Produkt dann mit KCN oder NaCN

b) man alkyliert am Stickstoff mit Chloracetonitril eine Verbindung der allgemeinen Formel 8:

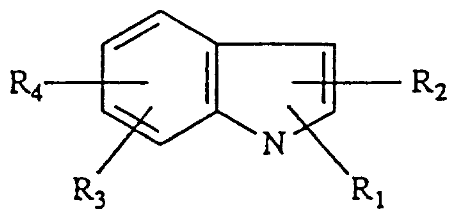

8

worin $R_1$, $R_2$, $R_3$ und $R_4$ die oben angegebenen Bedeutungen haben, führt dann gegebenenfalls eine Mono- oder Di-Kohlenstoffalkylierung der in den Stufen a) oder b) erhaltenen Verbindungen mit einem Halogenid durch.

**9.** Verfahren zur Herstellung der Verbindungen der allgemeinen Formel 6, in der Z einen zweiwertigen Rest der Formel $-CH=CH-C(R_7R_8)$ oder $-C(R_7R_8)-CH=CH-$ bedeutet, worin $R_7$ und $R_8$ die in Anspruch 1 angegebene Bedeutung haben und das Verfahren dadurch gekennzeichnet ist, daß man jeweils eine Verbindung der Formel 11

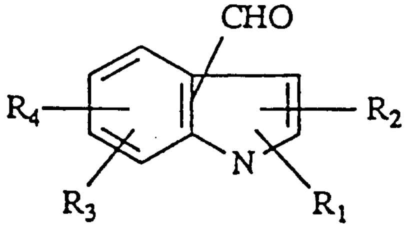

11

oder eine Verbindung der Formel 14

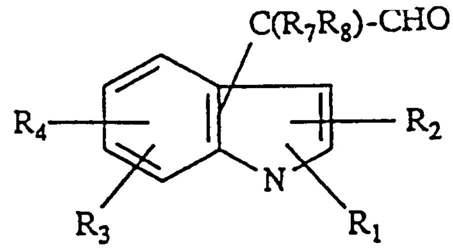

14

worin $R_1$, $R_2$, $R_3$ und $R_4$ wie in Anspruch 1 definiert sind, mit einem Phosphonat oder einem Cyanoalkylentriphenylphosphoran reagieren läßt.

**10.** Verfahren zur Herstellung der Verbindungen der allgemeinen Formel 6, worin Z einen zweiwertigen Rest der Formel $-(CH_2)_n-C(R_7R_8)-(CH_2)_p-$ bedeutet, in dem $R_7$ und $R_8$ die in Anspruch 1 angegebene Bedeutung haben und n = 0 und p = 1 oder p = 0 und n = 1 ist, wobei dieses Verfahren dadurch gekennzeichnet ist, daß man eine Verbindung der Formel 11 oder der Formel 14, wie sie im Anspruch definiert sind, mit Borhydrid reduziert, das so erhaltene Produkt in sein entsprechendes Chlorderivat mit Thionylchlorid überführt, dann das Chlorid mit einem Nitril in Gegenwart einer Base reagieren läßt.

**11.** Pharmazeutische Zubereitungen, dadurch gekennzeichnet, daß sie eine wirksame Menge wenigstens einer Verbindung der Formel 1, wie sie in Anspruch 1 definiert ist, im Gemisch mit pharmazeutisch verträglichen Hilfsstoffen enthält.

**12.** Arzneimittel, insbesondere mit serumlipidsenkender, antiarteriosklerotischer und antidiabetischer Wirkung nach Anspruch 11 in Einzeldosisform, in der jede Einzeldosis 10 bis 500 mg des wirksamen Stoffs im Gemisch mit einem pharmazeutisch verträglichen Bindemittel enthält.

**13.** Verwendung einer Verbindung nach Anspruch 1 zur Herstellung eines Arzneimittels zur Behandlung von Hyperglykämie.

**14.** Verwendung einer Verbindung nach Anspruch 1 zur Herstellung eines Arzneimittels zur Behandlung von Hyperlipidämie.

**15.** Verwendung einer Verbindung nach Anspruch 1 zur Herstellung eines Arnzeimittels zur Behandlung von Atherosklerose.

**Patentansprüche für folgenden Vertragsstaat : ES**

**1.** Verfahren zur Herstellung neuer Indolderivate der folgenden allgemeinen Formel 1

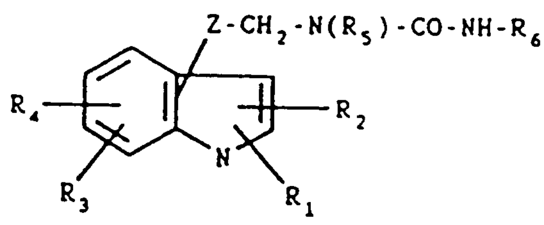

1

worin $R_1$ und $R_2$, die in 1-, 2- oder 3-Position des Indolrings angeordnet sein können, unabhängig voneinander ein Wasserstoffatom, einen geradkettigen Alkylrest mit 1 bis 12 Kohlenstoffatomen oder einen verzweigten Alkylrest mit 3 bis 5 Kohlenstoffatomen, $C_3$-$C_6$-Alkenylreste, $C_3$-$C_8$-Cycloalkylreste, N-($C_1$-$C_5$-Alkyl) amino($C_1$-$C_5$-alkyl)reste oder N,N-($C_1$-$C_5$-Dialkyl)amino($C_1$-$C_5$-alkyl)reste bedeuten oder einer der Substituenten $R_1$ oder $R_2$ einen 2-(3- oder 4-)Pyridylmethylrest und der andere ein Wasserstoffatom bedeutet, wobei in dem Falle, in dem das Stickstoffatom des Indolrings nicht durch eine der Gruppen $R_1$, $R_2$ oder

$-Z-CH_2-N(R_5)CONHR_6$ substituiert ist, es durch ein Wasserstoffatom substituiert ist,

$R_3$ und $R_4$, die in 4-, 5-, 6- oder 7-Position des Indolrings angeordnet sein können, bedeuten unabhängig voneinander Wasserstoff- oder Halogenatome oder $C_1$-$C_5$-Alkylreste, $C_1$-$C_5$-Alkoxyreste oder $C_1$-$C_5$-Alkylthioreste oder einer der Substituenten $R_3$ oder $R_4$ bezeichnet ein Wasserstoffatom und der andere einen Trifluormethylrest, Nitrorest, N-($C_1$-$C_5$-Alkyl)aminorest oder N,N-($C_1$-$C_5$-Dialkyl)-aminorest, oder

drei der Substituenten $R_1$, $R_2$, $R_3$ oder $R_4$ die zuvor definierten Bedeutungen haben und der vierte einen Rest der folgenden Formel 2 bedeutet

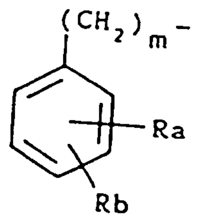

2

in der m die Werte 0, 1 oder 2 haben kann und die Substituenten Ra und Rb unabhängig voneinander Wasserstoffatome oder Halogenatome oder $C_1$-$C_5$-Alkylreste, $C_1$-$C_5$-Alkoxyreste oder $C_1$-$C_5$-Alkylthioreste bedeuten, $R_5$ ein Wasserstoffatom, einen geradkettigen Alkylrest mit 1 bis 12 Kohlenstoffatomen oder verzweigten Alkylrest mit 3 bis 5 Kohlenstoffatomen, einen $C_3$-$C_8$-Cycloalkylrest oder einen Rest der Formel 2 bedeutet, in der m den Wert 1 hat und Ra und Rb die zuvor definierten Bedeutungen haben,

$R_6$ einen $C_1$-$C_5$-Alkylrest oder einen Rest der folgenden Formel 3

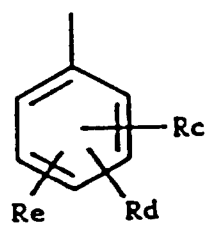

3

bedeutet, worin Rc, Rd und Re unabhängig voneinander Wasserstoffatome oder Halogenatome oder $C_1$-$C_5$-Alkylreste, $C_1$-$C_5$-Alkoxyreste oder $C_1$-$C_5$-Alkylthioreste bedeuten oder worin 2 der Substituenten Rc, Rd und Re die zuvor angegebenen Bedeutungen haben können und der dritte ein Trifluormethylrest bedeutet,

$R_6$ auch 1- oder 2-Naphtylreste oder einen 5- oder 6-gliedrigen Heterozyklus bezeichnen und ein oder zwei Heteroatome gegebenenfalls einen Benzolring bilden und gegebenenfalls mit einem bis drei Halogenatomen oder $C_1$-$C_5$-Alkylresten oder $C_1$-$C_5$-Alkoxyresten substituiert sind,

Z, das mit den 1-, 2-, 3-, 4-, 5-, 6- oder 7-Positionen des Indolrings verbunden sein kann, zweiwertige Reste der Formel $-CH=CH-C(R_7R_8)-$ oder $-(CH_2)_nC(R_7R_8)-(CH_2)_p-$ bezeichnet, worin n und p ganze Zahlen sind und die Werte 0, 1 und 2 unter der Bedingung haben können, daß ihre Summe $(n+p)$ kleiner oder gleich 2 ist,

$R_7$ und $R_8$ unabhängig voneinander ein Wasserstoffatom, einen geradkettigen Alkylrest mit 1 bis 12 Kohlenstoffatomen oder verzweigten Alkylrest mit 3 bis 5 Kohlenstoffatomen, $C_3$-$C_6$-Alkenylreste, $C_3$-$C_8$-Cycloalkylreste, N-($C_1$-$C_5$-Alkyl)amino, N,N-($C_1$-$C_5$-Dialkyl)amino, N-($C_1$-$C_5$Alkyl)amino($C_1$-$C_5$-alkyl) oder N,N-($C_1$-$C_5$-Dialkyl)amino($C_1$-$C_5$-alkyl) oder eine Gruppe der Formel 2 bedeuten, in der m die Werte 0 oder 1 haben kann und Ra und Rb die zuvor angegebenen Bedeutungen haben,

$R_7$ und $R_8$ gemeinsam eine Polymethylenkette $-(CH_2)_q-$ bilden können, worin q die Werte 3 bis 8 haben kann und gegebenenfalls, wenn q größer oder gleich 5 ist, eine Doppelbindung enthalten kann,

$R_7$ und $R_8$ auch gemeinsame Ketten $-CH_2-O-(CH_2)_2-$, $-(CH_2)_2-O-(CH_2)_2-$, $-CH_2-S-(CH_2)_2-$, $-(CH_2)_2-S-(CH_2)_2-$, $-CH_2-N(R_9)-(CH_2)_2-$ oder $-(CH_2)_2-N(R_9)-(CH_2)_2-$, wobei $R_5$, $R_7$ und $R_8$, wenn sie einen $C_3$-$C_8$-Cycloalkylrest bedeuten,

eine Doppelbindung aufweisen können und

$R_9$ einen $C_1$-$C_5$-Alkylrest bedeutet, umfassend die Reaktion einer Verbindung der allgemeinen Formel 4

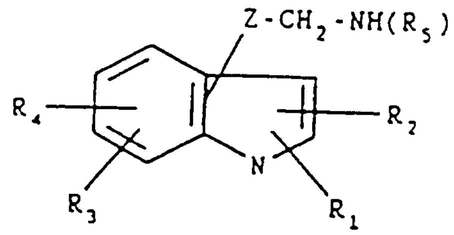

4

mit Phosgen und einem Amin $R_6NH_2$ oder mit einem Isocyanat $R_6NCO$ oder auch mit einem Trichloracetamid $CCl_3$-CO-$NHR_6$.

2. Verfahren nach Anspruch 1, dadurch gekennzeichnet, daß die Substituenten $R_3$ und $R_4$ Wasserstoffatome oder Halogenatome oder $C_1$-$C_5$-Alkylreste, N-($C_1$-$C_5$-Alkyl)aminoreste, N,N-($C_1$-$C_5$-Dialkyl)-aminoreste oder $C_1$-$C_5$-Alkoxyreste bedeuten, $R_5$ ein Wasserstoffatom, einen geradkettigen $C_1$-$C_{12}$-Alkylrest oder eine Gruppe der folgenden Formel

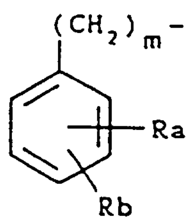

2

bedeutet, worin m gleich 1 ist und die Substituenten Ra und Rb unabhängig voneinander Wasserstoffatome oder Halogenatome oder $C_1$-$C_5$-Alkylreste, $C_1$-$C_5$-Alkoxyreste oder $C_1$-$C_5$-Alkylthioreste bedeuten und $R_6$ eine Gruppe der Formel 3

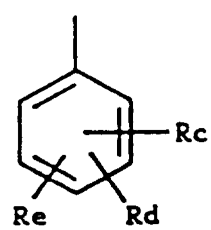

3

bedeutet, worin Rc, Rd und Re unabhängig voneinander Wasserstoffatome, Halogenatome oder $C_1$-$C_5$-Alkylreste, $C_1$-$C_5$-Alkoxyreste oder $C_1$-$C_5$-Alkylthioreste bedeuten oder zwei der Substituenten Rc, Rd und Re die zuvor angegebene Bedeutung haben können und der dritte einen Trifluormethylrest bedeutet.

3. Verfahren nach Anspruch 1, dadurch gekennzeichnet, daß man eine Verbindung herstellt, ausgewählt aus den folgenden Verbindungen:
1N-(2,6-Diisopropylphenyl)-2N-[1-(1-methyl-3-indolyl)cyclopentylmethyl]harnstoff, 1N-[1-(1-Indolyl)-cyclopentylmethyl]-2N-(2,6-diisopropylphenyl)harnstoff, 1N-(2,6-Diisopropylphenyl)-2N-[3-[1-(1-methyl-3-indolyl)cyclopentyl]propyl]harnstoff, 1N-(2,6-Diisopropylphenyl)-2N-[2-[1-(1-methyl-3-indolyl)-cyclopentyl]ethyl]harnstoff, 1N-Benzyl-2N-(2,6-diisopropylphenyl)-1N-[1-(1-methyl-3-indolyl)cyclopentyl-methyl]harnstoff, 2N-[1-(1-Methyl-3-indolyl)cyclopentylmethyl]-1N-phenylharnstoff, 1N-[2-(3-Indolyl)-ethyl]-2N-(2,6-diisopropyl-phenyl)harnstoff, 1N-(2,4-Difluorphenyl)-2N-[1-(1-methyl-3-indolyl-cyclopentyl-methyl]harnstoff, 1N-(2,6-Diisopropylphenyl)-2N-[3-methyl-2-(1-methyl-3-indolyl)butyl]harnstoff, 1N-tert.Butyl-2N-[1-(1-methyl-3-indolyl)cyclopentylmethyl]harnstoff, 1N-(2,6-Diisopropylphenyl)-2N-[2-(1-methyl-3-indolyl)propyl]harnstoff, 1N-[1-(1-Benzyl-3-indolyl)-cyclopentylmethyl]-2N-(2,6-diisopropylphenyl)harnstoff, 1N[1-(1-Ethyl-3-indolyl)cyclopentyl]-2N-(2,6-diisopropylphenyl)harnstoff,

1N-(2,6-Diisopropylphenyl)-2N-[2-(1-methyl-3-indolyl)butyl]harnstoff, 1N-(2,6-Diisopropylphenyl)-2N-[1-(1,5-dimethyl-3-indolyl)cyclopentylmethyl]harn-stoff, 1N-(2,6-Diisopropylphenyl)-2N-[1-(1-methyl-3-indolyl)cyclohexylmethyl]harnstoff, 1N-[2-Ethyl-2-(1-methyl-3-indolyl)butyl]-2N(2,6-diisopropylphenyl)-harnstoff, 1N-(2,6-Diisopropylphenyl)-2N-[2-methyl-2-(1-methyl-3-indolyl)propyl]harnstoff, 1N-[1-(1-Isopropyl-1-indolyl-3)-1-cyclopentylmethyl]-2N-(2,6diisopropylphenyl)harnstoff, 1N-[1-(1-Allyl-3-indolyl)-cyclopentylmethyl]-2N-(2,6-diisopropylphenyl)harnstoff, 1N-(2,6-Diisopropylphenyl)-2N-[2-(1-methyl-3-indolyl)2-phenylethyl]harnstoff, 1N-[2-Allyl-2-(1-methyl-3-indolyl)-4-pentenyl]-2N-(2,6-diisopropyl-phenyl)harnstoff, 1N-(2,6-Diisopropylphenyl)-2N-[1-(1-phenyl-3-indolyl)cyclopenthylmethyl]harnstoff, 1N-(2,6-Diisopropylphenyl)-2N-[1-(1-methyl-3-indolyl)cyclobutylmethyl]harnstoff, 1N-[2-Butyl-2-(1-methyl-3-indolyl)hexyl]-2N-(2,6diisopropylphenyl)harnstoff, 1N-(2,6-Diisopropylphenyl)-2N-[2-(1-methyl-3-indolyl)-4-pentenyl]harnstoff, 1N-[1-(1-Heptyl-3-indolyl)cyclopentylmethyl]-2N-(2,6-diisopropylphenyl)-harnstoff, 1N[1-(1-Butyl-3-indolyl)cyclopentylmethyl]-2N-(2,6-diisopropylphenyl)harnstoff, 1N-(2,6-Diisopropylphenyl)-2N-[2-(1-methyl-3-indolyl)hexyl]harnstoff, 1N-(2,6-Diisopropylphenyl)-2N-[2-(1-methyl-3-indolyl)-nonyl]harnstoff, 1N-(2,6-Diisopropylphenyl)-2N-[4,4-dimethyl-2-(1-methyl-3-indolyl)pentyl]harnstoff, 1N-(2,6-Diisopropylphenyl)-2N-[2-(1-methyl-3-indolyl)-3-phenylpropyl]harnstoff, 1N-(2,6-Dichlorphenyl)-2N-[1-(1-methyl-3-indolyl(cyclopentylmethyl]harnstoff, 1N-(2,6-Diisopropyl-phenyl)-2N-[1-(1-methyl-3-)cycloheptylmethyl]harnstoff, 1N-(2,6-Diisopropylphenyl)-2N-[4-(1-methyl-3-indolyl)-4-tetrahydropyranylmethyl] harnstoff, 1N-(2,6-Diethylphenyl)-2N-[1-(1-methyl-3-indolyl)cyclopentyl-methyl]harnstoff, 1N-2,6-Diisopropylphenyl)-2N-[1-[1-(2-dimethylamino-ethyl)-3-indolyl]cyclopentylmethyl]harnstoff, 1N-[1-[1-(4-Flurorbenzyl)-3-indolyl]cyclopentylmethyl]-2N-(2,6-diisopropylphenyl)harnstoff, 1N-(2,4,6-Trimethoxyphenyl)-2N-[1-(1-methyl-3-indolyl)cyclopenthylmethyl]harnstoff, 1N-(4,6-Dimethoxy-5-pyrimidinyl)-2N-[1-(1-methyl-3-indolyl)cyclopenthylmethyl]harnstoff, 1N-(2,6-Diisopropylphenyl)-2N-[1-(5-methoxy-1-methyl-3-indolyl)cyclopenthylbutyl]harnstoff,

1N-(2,6-Diisopropylphenyl)-2N-[4-dimethylamino-2-(1-methyl-3-indolyl)butyl]harnstoff, 1N-(2,6-Diisopropylphenyl)-2N-[5-methyl-2-(1-methyl-3-indolyl)hexen-4-yl]harnstoff, 1N-(2,6-Diisopropoxyphenyl)-2N-[1-(1-methyl-3-indolyl)cyclopentylmethyl[harnstoff, 1N-(2,6-Diisopropylphenyl)-2N-[1-[1-(3-pyridylmethyl)-3-indolyl]cyclo-pentylmethyl]harnstoff, 1N-Benzyl-1N-[1-(1-methyl-3-indolyl)cyclo-pentylmethyl]-2N-(2,6-dimethylphenyl)harnstoff, 1N-Benzyl-2N-(2,6-dichlorphenyl)-1N-[1-(1-methyl-3-indolyl)-cyclopentylmethyl]harnstoff, 1N-Benzyl-2N-(2,4-difluorphenyl)-1N-]1-(1-methyl-3-indolyl)cyclopentyl-methyl]harnstoff, 2N-(2,6-Diisopropylphenyl)-1N-methyl-1N-[1-(1-methyl-3-indolyl)cyclopentylmethyl]-harnstoff, 1N-(2,6-Diisopropylphenyl)-2N-[1-(1,2dimethyl-3-indolyl)cyclopentylmethyl]harnstoff, 1N-(2,6-Diisopropyl-phenyl)-2N-[1-(1-methyl-2-phenyl-3-indolyl)cyclopentylmethyl]harnstoff, 1N-(2,6-Diisopropylphenyl)-2N-[1-(1,3-dimethyl-2-indolyl)cyclopentyl-methyl]harnstoff, (-)-1N-(2,6-Diisopropylphenyl)-2N-[2-(1-methyl-3-indolyl)hexyl]harnstoff, (+)-1N-(2,6-Diisopropylphenyl)-2N-[2-(1-methyl-3-indolyl)hexyl]-harnstoff.

**4.** Verfahren nach Anspruch 4, umfassend die Umsetzung einer Verbindung der allgemeinen Formel 5

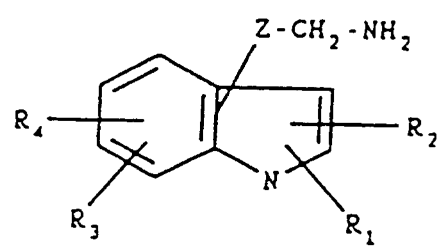

5

mit einer Halogenverbindung $R_5$-X oder auch mit einem Aldehyd $R_{10}$-CHO oder einem Säurechlorid $R_{10}$-COCl oder einem Säureanhydrid $(R_{10}CO)_2O$, worin $R_{10}$ einen Alkylrest bedeutet, der gegebenenfalls eine Doppelbindung aufweist, oder einen Rest der Formel 2, wie er in Anspruch 1 definiert ist, in dem m gleich 0 ist und $R_a$ und $R_b$, die in Anspruch 1 angegebenen Bedeutungen haben, gefolgt von der Reduktion des Imins oder des gebildeten Amids, um eine Verbindung der Formel 4 zu erhalten.

**5.** Verfahren nach Anspruch 5, umfassend die Reduktion eines Nitrils der Formel 6

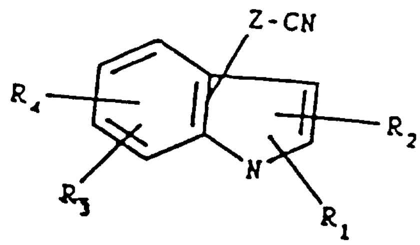

6

um eine Verbindung der Formel 5 zu erhalten.

**6.** Verfahren zur Herstellung von Verbindungen der allgemeinen Formel 6

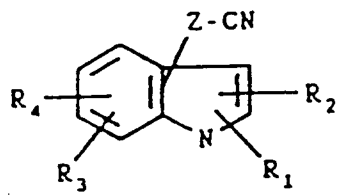

6

in der Z einen zweiwertigen Rest der Formel $-(CH_2)_n-C(R_7R_8)-(CH_2)_p-$bedeutet, worin n = P = O und $R_7$ und $R_8$ die in Anspruch 1 angegebene Bedeutung haben, wobei das Verfahren dadurch gekennzeichnet ist, daß man die folgenden Stufen a) oder b) durchführt.

a) man alkyliert mit Methyljodid ein N,N-Dimethylaminomethylindol der allgemeinen Formel 7:

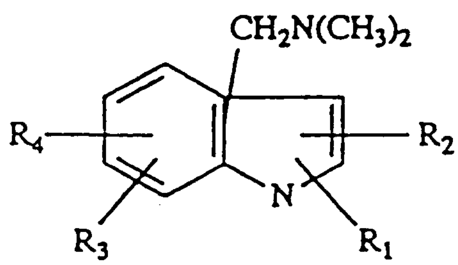

7

worin $R_1$, $R_2$, $R_3$ und $R_4$ die in Anspruch 1 angegebene Bedeutung haben, und behandelt das erhaltene Produkt dann mit KCN oder NaCN

b) man alkyliert am Stickstoff mit Chloracetonitril eine Verbindung der allgemeinen Formel 8:

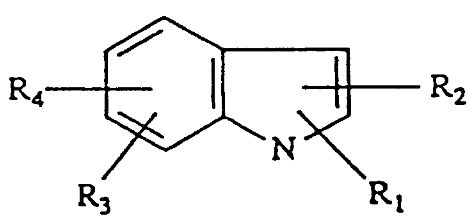

8

worin $R_1$, $R_2$, $R_3$ und $R_4$ die oben angegebenen Bedeutungen haben, führt dann gegebenenfalls eine Mono- oder Di-Kohlenstoffalkylierung der in den Stufen a) oder b) erhaltenen Verbindungen mit einem Halogenid durch.

**7.** Verfahren zur Herstellung der Verbindungen der allgemeinen Formel 6

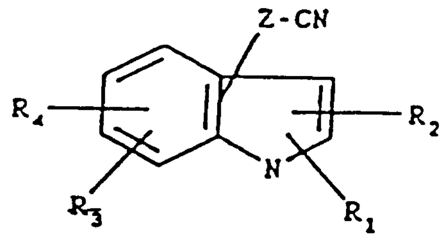

6

worin Z einen zweiwertigen Rest der Formel -CH=CH-C($R_7R_8$)- oder -C($R_7R_8$)-CH=CH- bedeutet, worin $R_7$ und $R_8$ die in Anspruch 1 angegebene Bedeutung haben und das Verfahren dadurch gekennzeichnet ist, daß man jeweils eine Verbindung der Formel 11

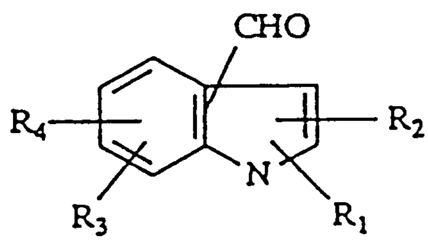

11

oder eine Verbindung der Formel 14

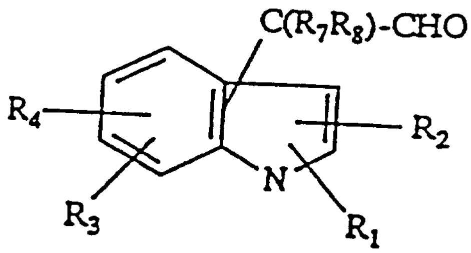

14

worin $R_1$, $R_2$, $R_3$ und $R_4$ wie in Anspruch 1 definiert sind, mit einem Phosphonat oder einem Cyanoalkylentriphenylphosphoran reagieren läßt.

**8.** Verfahren zur Herstellung der Verbindungen der allgemeinen Formel 6,

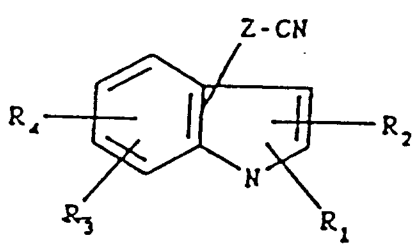

6

worin $R_1$, $R_2$, $R_3$ und $R_4$ die in Anspruch 1 angegebene Bedeutung haben und Z einen zweiwertigen Rest der Formel $-(CH_2)_n-C(R_7R_8)-(CH_2)_p-$ bedeutet, worin $R_7$ und $R_8$ die in Anspruch 1 angegebene Bedeutung haben und n = 0 und p = 1 oder p = 0 und n = 1 ist, wobei das Verfahren dadurch gekennzeichnet ist, daß man eine Verbindung der Formel 11 oder der Formel 14, wie sie in Anspruch 7 definiert sind, mit Borhydrid reduziert, das so erhaltene Produkt in sein entsprechendes Chlorid mit Thionylchlorid überführt, anschließend das Chlorid mit einem Nitril in Gegenwart einer Base reagieren

läßt.

9. Verfahren zur Herstellung einer pharmazeutischen Zubereitung, dadurch gekennzeichnet, daß man eine wirksame Menge wenigstens einer Verbindung der Formel 1, wie sie in Anspruch 1 definiert ist, mit einem pharmazeutisch verträglichen Bindemittel vermischt.